# EUROPEAN PATENT APPLICATION

(11) **EP 2 407 178 A2**
(43) Date of publication of application: **18.01.2012**
(21) Application number: 11184556.6
(22) Date of filing: 19.04.2006
(51) Int. Cl.: A61K 39/385, A61K 39/00, A61P 37/02, A61K 47/48

(54) **Monovalent and polyvalent synthetic polysaccharide antigens for immunological intervention in disease**

(30) Priority: 19.04.2005 US 672807 P
(62) Divisional of application: 06758412.8
(71) Applicant: Eli Lilly and Company, Indianapolis, IN 46202 (US)
(72) Inventor: The designation of the inventor has not yet been filed
(74) Representative: Carter, Caroline Mercedes

(57) **Abstract**

The present invention provides a pro-inflammatory synthetic polysaccharide antigen (SPA), or a pharmaceutically acceptable salt thereof, comprising a TLR2 -targeting synthetic peptidoglycan (PGN) moiety onto which a first epitope and a second epitope are each covalently attached. The first epitope comprises one or more than one generic T helper peptide sequence, and the second epitope comprises one or more than one target epitope. The first and second epitopes are present in one or more copies each within the SPA. Each target epitope is a peptide sequence or a carbohydrate moiety, and is an immunogen to CD8+ T cells or B cells. The present invention also provides a suppressive synthetic polysaccharide antigen (SPA), or a pharmaceutically acceptable salt thereof, comprising a TLR2-targeting synthetic peptidoglycan (PGN) moiety onto which one or more than one target epitope is covalently attached. Each target epitope is a peptide sequence or carbohydrate moiety and is present in one or more copies within the SPA.

## Description

### FIELD OF INVENTION

The present invention relates to monovalent and polyvalent synthetic polysaccharide antigens for immunological intervention in disease. More specifically, the present invention relates to antigen-specific stimulation and suppression of the immune response by monovalent and polyvalent synthetic polysaccharide antigens.

### BACKGROUND OF THE INVENTION

Dendritic cells (DCs) reside in almost all peripheral tissues in an immature state (iDC), which allows them to phagocytose antigens, generate peptide epitopes from the antigens, and load the epitopes into recognition clefts of molecules that are encoded by the major histocompatibility complex (MHC). The uptake and processing of antigen leads to maturation of the DC, which results in loss of its ability to take up and process antigen, display the processed antigen on its surfaces, and is characterized by an increased expression of surface MHC II molecules and co-stimulatory molecules such as CD80 and CD86 (Chakraborty et al. (2000) Clin. Immunol. 94:88-98). Initialtion of DC maturation can be caused by the stimulation of toll-like receptors (TLRs), which are present on dendritic cells. TLRs recognize antigens with highly conserved structural motifs, for example pathogen-associated molecular patterns (PAMPs; Medzhitov (2001) Nat. Rev. Immunol. 135-145), including lipopolysaccharides (LPS), peptidoglycan and lipopeptides, as well as flagellin, bacterial DNA, and viral double-stranded RNA.

Mature dendritic cells are potent stimulators of T cells and, with their multitentacled (dendritic) shape, proceed to make cell-cell contact with large numbers of T cells (Banchereau et al. (2000) Annu. Rev. Immunol.18:767-811) through the epitope-laden MHC molecules. Activated CD4+ T helper (Th) cells are then able to deliver chemokine and cytokine signals to other DCs, enabling them to activate naive CD8+ T cells, transforming these cells into antigen-specific cytotoxic T lymphocytes (CTL). Activated Th cells interact with B cells as well, providing them with molecular signals that control differentiation, clonal expansion, and definition of the antibody isotype that they will secrete in mounting the humoral response of adaptive immunity.

The capacity of DCs to activate T cells is linked to their constitutive expression of both MHC and costimulatory markers such as CD80 and CD86) (Banchereau et al. (2000) Annu. Rev. Immunol. 18:767-811). If these molecules are decreased or absent from the DC cell surface, the DCs are unable to participate in stimulatory cognate interactions with T cells. Immature DCs contribute to peripheral tolerance by inducing the differentiation of human T regulatory (Treg) cells (Jonuleit et al. (2000) J. Exp. Med. 192:1213-1222), which display regulatory functions *in vitro* and *in vivo.* Activated Treg cells have also been shown to elicit the production of IL-10, an anti-inflammatory cytokine, through autocrine expression or induction in effector T cells (Dieckmann et al. (2002) J. Exp. Med. 196:247-253).

IL10, a type II cytokine, has potent anti-inflammatory activity, down-modulating inflammatory responses of T effector cells (Morel et al. (2002) Immunol. 106:229-236), dendritic cells (Martin et al. (2003) Immunity 18:155-167), and other antigen presenting cells (Williams et al. (2002) J. Leuko. Biol.72:800-809). IL10 acts to down regulate unchecked inflammatory responses that could otherwise be deleterious to the host (Moore et al. (2001) Annu. Rev. Immunol. 19:683-765).

Vaccination and immunotherapy strategies are directed to exogenous manipulation of this intricately choreographed series of cellular interactions.

Current Vaccine Technologies

The generation of a strong CD8+ T cell response against a given CTL epitope and antibody response against a given antigenic epitope both require the generation of a strong Th response. It is therefore desirable to administer at least one T helper cell epitope with the antigenic epitope (Vitello et al. J. Clin. Invest (1995) 95:341; Livingston et al. (1997) J. Immunol. 159:1383). To avoid large genetic variation in the immune responses of individuals to a particular antigen, the antigen is often administered in conjuction with a large protein having a range of Th epitopes, for example keyhole limpet hemocyanin (KLH).

Alternatively, promiscuous or permissive Th epitope-containing peptides are administered with the antigen. Promiscuous or permissive Th epitope-containing peptides are presented in the context of a majority of MHC class II haplotypes, therefore inducing a strong CD4+ Th response in the majority of the human population. Examples of promiscuous or permissive T helper epitopes include tetanus toxoid peptide, *Plasmodium falciparum* pfg27, lactate dehydrogenase peptide, and gp120 of HIV.

Immunotherapy and vaccination are attractive approaches for prophylaxis or therapy of a range of disorders such as certain infectious diseases or cancers. However, the success of such treatments is often limited by several shortcomings inherent to immunotherapeutic protocols. Most common is poor immunogenicity of the chosen CTL epitope. Synthetic peptides representing T cell immunogens elicit only a weak immune response when delivered in isolation. As a consequence, they are not effective as vaccine or immunotherapy preparations. Full-length proteins that contain CTL epitopes do not efficiently enter the MHC class I processing pathway. Additionally, CTL epitopes are also HLA-restricted and so the large degree of MHC class I polymorphism in the human population means that CTL epitope-based vaccines may not provide broad based protection to all genotypes within a population. In addition, multiple antigens may be, for reasons of pathogen/tumor heterogeneity, required for effective elimination of a target microbe or tumor cell.

The standard method to increase the immune response is to use an adjuvant, such as complete Freund's adjuvant (CFA), that is separate from the immunogen. However, many of the effective adjuvants, including CFA, are too toxic for use in humans. Some adjuvants require prior formulation with the immunogen immediately before administration because of very poor solubility. Alum, among the very few adjuvants approved for use in humans, is an example of such an adjuvant. Recently, certain microbial natural products have been shown to be useful in immunomodulation, in particular as adjuvants, and have set the stage for development of new vaccine technologies (Kensil, Methods Mol. Med. (2000) 42:259).

Pro-Inflammatory Responses

Microbial antigens such as lipopolysaccharide (LPS) from Gram negative bacteria, and bacterial cell wall glycopeptides, also known as murein or peptidoglycan (PGN), from both Gram negative and Gram positive bacteria are powerful immunomodulators. For example, high molecular weight bacterial PGN from natural sources is well known as a potent inflammatory agent and has long been used to induce arthritis in experimental animals (Wahl et al. (1986) J. Exp. Med. 165:884).

Many microbial antigens, including PGN, are thought to exert their pro-inflammatory effects by activating one or more of the mammalian TLR. Binding to and activation of a TLR triggers an intracellular signaling cascade that leads to induction of the transcription factor NF-κB, which in turn stimulates expression of genes encoding pro-inflammatory mediators such as chemokines and certain cytokine.

A second natural product ligand of TLR2 is the lipid component of macrophage-activating lipopeptide 2 (MALP-2) from mycoplasma (Mulradt et al. (2002) J. Exp. Med. 185:1951). Pam3Cys, a synthetic version of MALP-2, has been shown to be capable of promoting virus-specific CTL responses against influenza virus infected cells (Deres et al. (1989) Nature 342:561) and to stimulate the production of protective antibodies to foot-and-mouth disease (Weismuller et al. (1989) Vaccine 7:29; U.S. Patent No. 6,024,964) when conjugated to appropriate epitopes. Another synthetic version of MALP-2, Pam2Cys, has been covalently attached to various antigenic peptide epitopes and these monovalent epitope-based vaccine/therapeutics have demonstrated cellular (cytotoxic T cell) or humoral (antibody mediated) immune responses in animal models (Jackson et al. (2004) PNAS 101:16440; WO 2003/014956; and WO 2003/014957).

Monovalent, epitope-based vaccine/therapeutic strategies have attracted considerable attention, especially in the area of cancer chemotherapy. For example, heat shock protein-peptide conjugates (WO 2004/071457; WO 2004/091493), carbohydrate-carrier protein conjugates (Slovin et al. (1999) PNAS 96:5710), and Pam2Cys-peptide conjugates (WO 2004/014956; WO 2004/14957) have all been used to elicit cellular and/or humoral immune responses to specific antigens. However, considering the antigenic heterogeneity of tumors and the heterogeneity of the human immune response against any one given antigenic epitope, polyvalent vaccines are required to produce consistent clinical success. For example, Pneumovax® 23 (Merck and Co.) comprises epitopes from twenty-three different serotypes of *Streptococcus pneumoniae,* and is able to confer broad, generalized immunity to strep infection.

Suppressive Responses

Not all ligands of TLR2 initiate the pro-inflammatory intracellular signaling cascade. For example, a mouse anti-human blocking antibody specific for TLR2 has been shown to be internalized by TLR2 and incorporated into the MHC class II processing pathway, however no maturing of the DCs, upregulating of the co-stimulatory molecules CD80 and CD86, upregulating of MHC class II molecules, or inducing of pro-inflammatory mediators was observed (Schejetne et al. (2003) J. Immunol. 171:32).

WO 2003/070761 discloses a specific fragment, designated p277, of heat shock protein 60 that binds to TLR2 and results in anti-inflammatory responses. Conjugation of p277 to an antigenic epitope that is specific for a cell mediated autoimmune disease results in bifunctional molecules (TLR2 ligand-epitope) that are antigen-specifically anti-inflammatory. Each bifunctional molecule, however, is limited to a single epitope from the inflammatory or autoimmune disease state of interest.

WO 2003/075593 describes a version of totally synthetic bacterial PGN that does not induce NF-κB through TLR2 when compared with natural bacterial PGNs, which did induce the production of NF-κB. The structure of the synthetic PGN resembles that of natural PGNs, but like p277 and the blocking antibody discussed above, the synthetic PGN binds to TLR2 without stimulation through TLR2. Peritoneal abscess formation, post-sugical adhesion formation, and the candin DTH response are all suppressed in vivo by the synthetic PGN. Furthermore, these authors showed by array analysis that the anti-inflammatory mediators IL-10 and IL-19, and not stimulatory cytokines and chemokines, are upregulated upon treatment with the synthetic PGN. Basd on these results, the synthetic PGN of WO 2003/075593 is a generalized suppressor of pro-inflammatory effector T cells.

There are numerous animal models of inflammation in which IL10 has been shown to be efficacious, e.g., inflammatory bowel disease (IBD), Crohn's disease, rheumatoid arthritis, autoimmune diabetes, and allergic disease (Madsen (2002) Gastroenterol. 123:2140-2144; Barnes (2001) Curr. Opin, Allergy Clin. Immunol.1:555-560; Bremeanu et al (2001) Int. Rev. Immunol. 20:301-331; St. Clair (2000) Curr. Dir. Autoimmun. 2:126-149). Clinical trials using recombinant IL10 for the treatment of inflammatory bowel disease have, however, met with mixed results. Requirements for repeated high dose regimens, as well as some resulting toxicity, have hampered the success of these efforts.

There remains a need for therapeutic molecules that modulate the immune response, in both pro-inflammatory and suppressive contexts, in a safe and effective manner. Such additional molecules could facilitate the development of more effective immunotherapeutic strategies for disease prevention and treatment.

### SUMMARY OF THE INVENTION

The present invention relates to monovalent and polyvalent synthetic polysaccharide antigens (SPAs) for immunological intervention in disease. More specifically, the present invention relates to antigen-specific stimulation and suppression of the immune response by monovalent and polyvalent synthetic polysaccharide antigens.

The present invention provides a pro-inflammatory synthetic polysaccharide antigen (SPA), comprising:
a TLR2-targeting synthetic peptidoglycan (PGN) moiety onto which a first epitope and a second epitope are each covalently attached;
the first epitope comprising one or more than one generic T helper epitope, the second epitope comprising one or more than one target epitope; the first and
second epitopes are present in one or more copies each within the SPA,
wherein each target epitope is a peptide sequence or a carbohydrate moiety, and wherein each target epitope is an immunogen to CD8+ T cells or B cells, or a pharmaceutically acceptable salt thereof.

The pro-inflammatory SPA, or a pharmaceutically acceptable salt thereof, may comprise a single target epitope in one or more copies each within the SPA.

The present invention also provides a pro-inflammatory SPA as just described, which is selected from 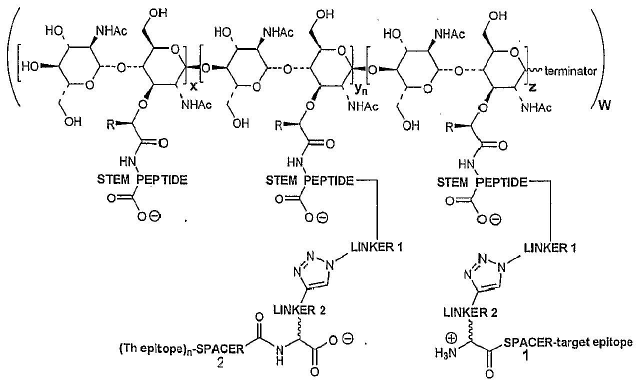 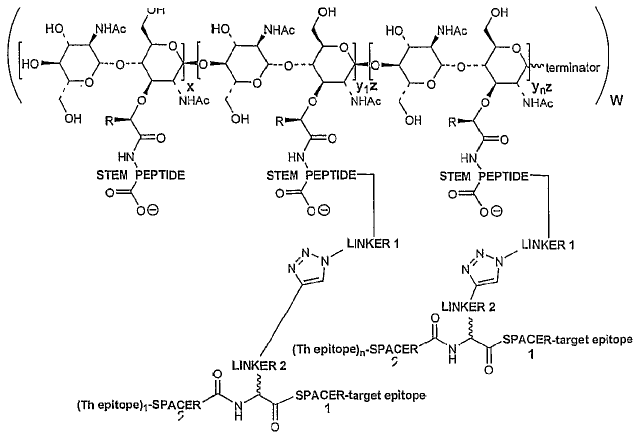 and pharmaceutically acceptable salts thereof, wherein
W is the total number of monomeric units in the SPA and is an integer in the range of about 10 to about 375;
R are independantly selected from H or lower alkyl;
x is the mole fraction of unsubstituted repeat units (UR) in the SPA;
yₙ is mole fraction of the nth species of Th epitope repeat units (ThR) in the SPA;
z is the mole fraction of target epitope repeat unit (TR) in the SPA;
yₙz is mole fraction of the nth species of combined Th epitope / target epitope repeat units (Th/TR) in the SPA;
STEM PEPTIDE are independantly selected, and comprise about 2 to about 5 amino acids, wherein the amino acids are independantly joined at the α or γ carboxyl groups, and at the α or ε amino groups, or any combination thereof, provided that a pendant carboxylate or carboxamide group is present;
LINKER 1 and LINKER2 are independantly selected, and comprise about 1 to about 6 segments, each segment selected from -CH₂-, -CHR-, =CH-, and ≡CH-, -O-, NH-, NR-, -S-, -SO-, and -SO₂-, provided that there are no contiguous heteroatom segments and that the heteroatom segments are not in segments 1 and 2, where R is a lower alkyl;
SPACER1 is a peptide of about 1 to about 10 amino acids in length;
SPACER2 is 0 to about 10 amino acids in length ;
target epitope is a peptide sequence or carbohydrate moiety that is an immunogen to CD8+ T cells or to B cells; and
(Th epitope)ₙ is a number n of different Th epitopes, each Th epitope is independantly selected and comprises a generic T helper epitope.

The pro-inflammatory SPA of the present invention, or pharmaceutically acceptable salt thereof, may alternatively comprise more than one target epitope, in one or more copies each within the SPA.

The present invention further provides a pro-inflammatory SPA as just described, which is selected from 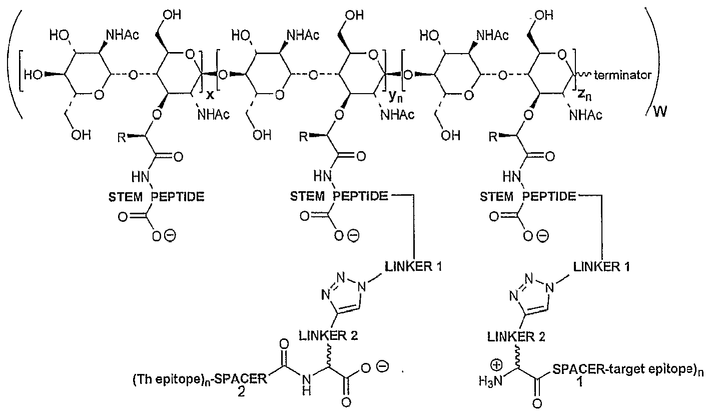 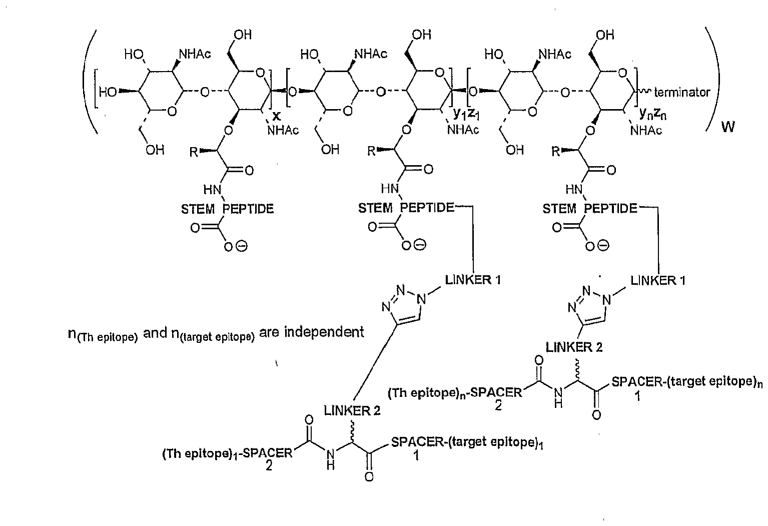 and pharmaceutically acceptable salts thereof, wherein
W is the total number of monomeric units in the SPA and is an integer in the range of about 10 to about 375;
R are independantly selected from H or lower alkyl;
x is the mole fraction of unsubstituted repeat units (UR) in the SPA;
yₙ is mole fraction of the nth species of Th epitope repeat units (ThR) in the SPA;
zₙ is the mole fraction of the nth species of target epitope repeat unit (TR) in the SPA;
yₙzₙ is mole fraction of the nth/nth species of combined Th epitope / target epitope repeat units (Th/TR) in the SPA;
STEM PEPTIDE are independantly selected, and comprise about 2 to about 5 amino acids, wherein the amino acids are independantly joined at the α or γ carboxyl groups, and at the α or ε amino groups, or any combination thereof, provided that a pendant carboxylate or carboxamide group is present;
LINKER 1 and LINKER2 are independantly selected, and comprise about 1 to about 6 segments, each segment selected from -CH₂-, -CHR-, =CH-, and ≡CH-, -O-, NH-, NR-, -S-, -SO-, and -SO₂-, provided that there are no contiguous heteroatom segments and that the heteroatom segments are not in segments 1 and 2, where R is a lower alkyl;
SPACER1 is a peptide of about 1 to about 10 amino acids in length;
SPACER2 is 0 to about 10 amino acids in length ;
(target epitope)ₙ is a is a number n of different target epitopes, each target epitope is independantly selected and is peptide sequence or carbohydrate moiety that is an immunogen to CD8+ T cells or to B cells; and
(Th epitope)ₙ is a number n of different Th epitopes, each Th epitope is independantly selected and comprises a generic T helper epitope.

The pro-inflammatory SPA as described above may comprise about 2 to about 180 target epitopes and about 1 to about 180 Th helper epitopes, in one or more copies each.

The present invention provides a suppressive synthetic polysaccharide antigen (SPA), comprising:
a TLR2-targeting synthetic peptidoglycan (PGN)moiety onto which one or more than one target epitope is covalently attached, in one or more copies each, within the SPA, wherein each species of target epitope is a peptide sequence or carbohydrate moiety,
or a pharmaceutically acceptable salt thereof.

The suppressive SPA, or a pharmaceutically acceptable salt thereof, may comprise a single target epitope in one or more copies each within the SPA.

The present invention also provides a suppressive SPA as just described, which is the SPA of 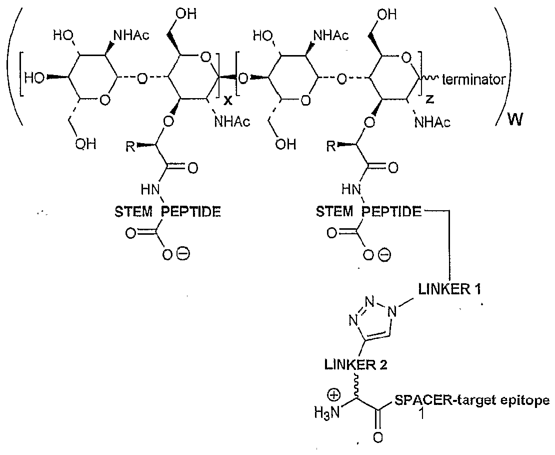 or a pharmaceutically acceptable salt thereof, wherein
W is the total number of monomeric units in the SPA and is an integer in the range of about 10 to about 375;
R are independantly selected from H or lower alkyl;
x is the mole fraction of unsubstituted repeat units (UR) in the SPA;
z is the mole fraction of target epitope repeat unit (TR) in the SPA;
STEM PEPTIDE are independantly selected, and comprise about 2 to about 5 amino acids, wherein the amino acids are independantly joined at the α or γ carboxyl groups, and at the α or ε amino groups, or any combination thereof, provided that there is no pendant carboxylate or carboxamide group; LINKER 1 and LINKER2 are independantly selected, and comprise about 1 to about 6 segments, each segment selected from -CH2-, -CHR-, =CH-, and ≡CH-, -O-, NH-, NR-, -S-, -SO-, and -SO₂-, provided that there are no contiguous heteroatom segments and that the heteroatom segments are not in segments 1 and 2, where R is a lower alkyl;
SPACER1 is a peptide of about 1 to about 10 amino acids in length; and target epitope is a peptide sequence or carbohydrate moiety.

The suppressive SPA of the present invention, or pharmaceutically acceptable salt thereof, may alternatively comprise more than one target epitope, in one or more copies each within the SPA.

The present invention also provides a suppressive SPA as just described, which is the SPA of 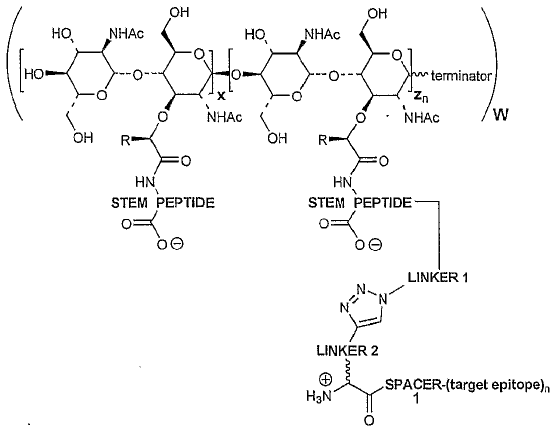 or a pharmaceutically acceptable salt thereof, wherein
W is the total number of monomeric units in the SPA and is an integer in the range of about 10 to about 375;
R are independantly selected from H or lower alkyl;
x is the mole fraction of unsubstituted repeat units (UR) in the SPA;
zₙ is the mole fraction of the nth species of target epitope repeat unit (TR) in the SPA;
STEM PEPTIDE are independantly selected, and comprise about 2 to about 5 amino acids, wherein the amino acids are independantly joined at the α or γ carboxyl groups, and at the α or ε amino groups, or any combination thereof, provided that there is no pendant carboxylate or carboxamide group;
LINKER1 and LINKER2 are independantly selected, and comprise about 1 to about 6 segments, each segment selected from -CH₂-, -CHR-, =CH-, and ≡CH-, -O-, NH-, NR-, -S-, -SO-, and -SO₂-, provided that there are no contiguous heteroatom segments and that the heteroatom segments are not in segments 1 and 2, where R is a lower alkyl;
SPACER1 is a peptide of about 1 to about 10 amino acids in length; and (target epitope)ₙ is a is a number n of different target epitopes, each target epitope is independantly selected and is peptide sequence or carbohydrate moiety.

The suppressive SPA as described above may comprise about 2 to about 180 target epitopes, in one or more copies each.

The present invention further provides a synthetic polysaccharide antigen, wherein the SPA is a polymer comprising the sequence:

X¹-[-MO-]_{w}-X²

wherein
X¹ and X² are independently H or a terminator;
W represents the number of monomeric units (MO) in the polymer, and may be an integer in the range of from about 2 to about 375;
each MO is a monomeric unit selected from the group comprising unsubstituted repeat units (UR), one or more more than one species of Th epitope repeat units (ThR), one or more more than one species of target epitope repeat units (TR), one or more than one species of combined Th/target epitope repeat unit (Th/TR), and a combination thereof,
or a pharmaceutically acceptable salt thereof.

The SPA as just described may be a random copolymer, a block copolymer, or an alternating copolymer.

The present invention also provides a synthetic polysaccharide antigen, or pharmaceutically acceptable salt thereof, as described above, wherein the SPA is a pro-inflammatory synthetic polysaccharide antigen comprising a TLR2-targeting synthetic peptidoglycan (PGN) moiety onto which a first epitope and a second epitope are covalently attached; the first epitope comprising one or more than one generic T helper epitope; the second epitope comprising one or more than one target epitope, and the first and second eptiope present in one or more copies each, within the SPA; wherein each target epitope is a peptide sequence or a carbohydrate moiety, and wherein each target epitope is an immunogen to CD8+ T cells or B cells, or a pharmaceutically acceptable salt thereof.

The present invention also provides a synthetic polysaccharide antigen, or pharmaceutically acceptable salt thereof as described above, wherein the SPA is a suppresive synthetic polysaccharide antigen comprising, a TLR2-targeting synthetic peptidoglycan (PGN) moiety onto which one or more than one target epitope is covalently attached, in one or more copies each within the SPA, wherein each target epitope is a peptide sequence or carbohydrate moiety.

The present invention further provides a pro-inflammatory synthetic polysaccharide antigen (SPA) comprising from about 10 to about 375 monomeric units, the monomeric units independantly selected from 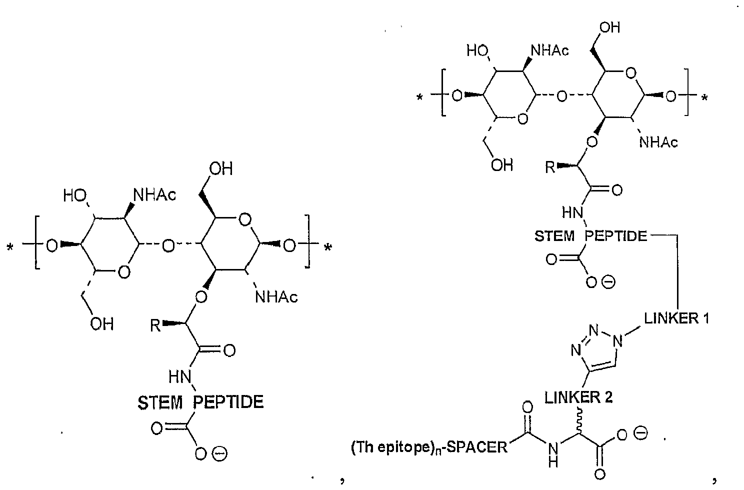 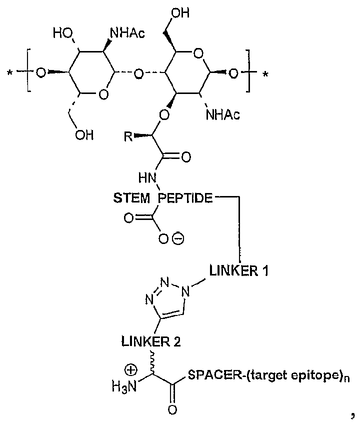 and pharmaceutically acceptable salts thereof,
wherein
R are independantly selected from H or lower alkyl;
STEM PEPTIDE are independantly selected, and comprise about 2 to about 5 amino acids, wherein the amino acids are independantly joined at the α or γ carboxyl groups, and at the α or ε amino groups, or any combination thereof, provided that a pendant carboxylate or carboxamide group is present;
LINKER 1 and LINKER2 are independantly selected, and comprise about 1 to about 6 segments, each segment selected from -CH₂-, -CHR-, =CH-, and ≡CH-, -O-, NH-, NR-, -S-, -SO-, and -SO₂-, provided that there are no contiguous heteroatom segments and that the heteroatom segments are not in segments 1 and 2, where R is a lower alkyl;
SPACER1 is a peptide of about 1 to about 10 amino acids in length;
SPACER2 is 0 to about 10 amino acids in length ;
(target epitope)ₙ is a is a number n of different target epitopes, each target epitope is independantly selected and is peptide sequence or carbohydrate moiety that is an immunogen to CD8+ T cells or to B cells; and
(Th epitope)ₙ is a number n of different Th epitopes, each Th epitope is independantly selected and comprises a generic T helper epitope.

The present invention also provides a pro-inflammatory synthetic polysaccharide antigen (SPA) comprising from about 10 to about 375 monomeric units, the monomeric units independantly selected from 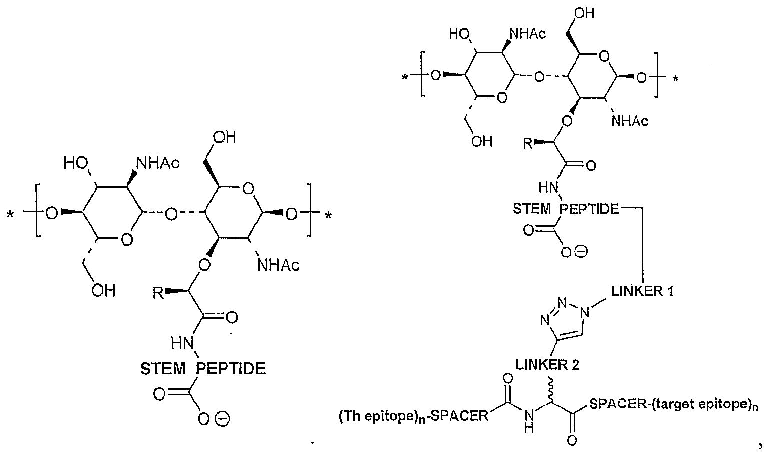 and pharmaceutically acceptable salts thereof,
wherein
R are independantly selected from H or lower alkyl;
STEM PEPTIDE are independantly selected, and comprise about 2 to about 5 amino acids, wherein the amino acids are independantly joined at the α or γ carboxyl groups, and at the α or ε amino groups, or any combination thereof, provided that a pendant carboxylate or carboxamide group is present;
LINKER 1 and LINKER2 are independantly selected, and comprise about 1 to about 6 segments, each segment selected from -CH₂-, -CHR-, =CH-, and ≡CH-, -O-, NH-, NR-, -S-, -SO-, and -SO₂-, provided that there are no contiguous heteroatom segments and that the heteroatom segments are not in segments 1 and 2, where R is a lower alkyl;
SPACER1 is a peptide of about 1 to about 10 amino acids in length;
SPACER2 is 0 to about 10 amino acids in length ;
(target epitope)ₙ is a is a number n of different target epitopes, each target epitope is independantly selected and is peptide sequence or carbohydrate moiety that is an immunogen to CD8+ T cells or to B cells; and
(Th epitope)ₙ is a number n of different Th epitopes, each Th epitope is independantly selected and comprises a generic T helper epitope.

The present invention further provides a suppressive synthetic polysaccharide antigen (SPA) comprising from about 10 to about 375 monomeric units, the monomeric units independantly selected from 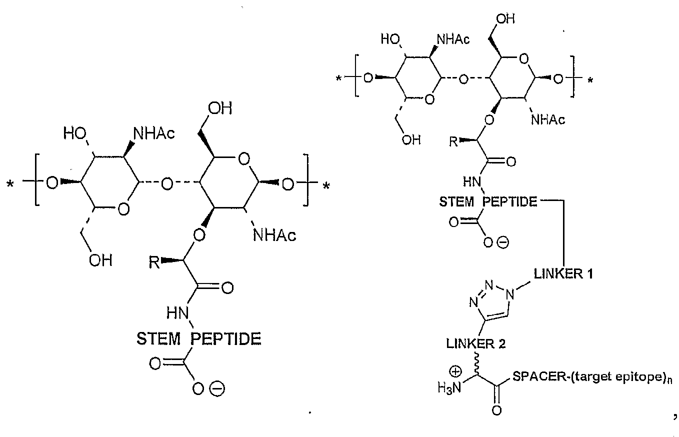 and
pharmaceutically acceptable salts thereof,
wherein
R are independantly selected from H or lower alkyl;
STEM PEPTIDE are independantly selected, and comprise about 2 to about 5 amino acids, wherein the amino acids are independantly joined at the α or γ carboxyl groups, and at the α or ε amino groups, or any combination thereof, provided there is no pendant carboxylate or carboxamide group;
LINKER 1 and LINKER2 are independantly selected, and comprise about 1 to about 6 segments, each segment selected from -CH₂-, -CHR-, =CH-, and ≡CH-, -O-, NH-, -NR-, -S-, -SO-, and -SO₂-, provided that there are no contiguous heteroatom segments and that the heteroatom segments are not in segments 1 and 2, where R is a lower alkyl;
SPACER1 is a peptide of about 1 to about 10 amino acids in length; and (target epitope)ₙ is a is a number n of different target epitopes, each target epitope is independantly selected and is peptide sequence or carbohydrate moiety.

The present invention further provides pharmaceutical compositions comprising any of the synthetic polysaccharide of described in the present invention, or a pharmaceutically acceptable salt thereof, and a pharmaceutically acceptable diluent, excipient or carrier.

The present invention also provides a use of any of the synthetic polysaccharide antigen of the present invention, or pharmaceutically acceptable salt thereof, as a medicament.

The present invention further provides the use of any of the the compound of any one of synthetic polysaccharide described in the present invention, or pharmaceutically acceptable salt thereof, for the preparation of a medicament for the prevention or treatment of a disease or disorder susceptible to treatment with an immunomodulator.

The present invention further provides a method of treating or preventing a disease or disorder susceptible to treatment with an immunomodulator, comprising administering to a patient in need thereof an effective amount of any of the synthetic polysaccharide of the present invention, or a pharmaceutically acceptable salt thereof.

The present invention also provides a method of inducing an immune response in a mammal, comprising administering to the mammal an effective amount of any of the synthetic polysaccharide described in the present invention, or a pharmaceutically acceptable salt thereof.

The monovalent and polyvalent synthetic polysaccharide antigens of the present invention are capable of delivering, within a single molecular entity, multiple copies of a single epitope or multiple epitopes, each in multiple copies. These new molecules can be designed to provide either pro-inflammatory or anti-inflammatory therapies in a rationally directed, antigen-specific manner.

The inflammatory *mono*SPAs and *poly*SPAs of the present inventnion can be used in humans and other mammals to induce an antigen-specific inflammatory response to treat disease states or conditions in which an inflammatory response is therapeutically beneficial, for example in antimicrobial, antiviral, or anticancer therapy. The suppressive *mono*SPAs and *poly*SPAs of the present invention can be used in humans and other mammals to treat disease states where suppression of a pro-inflammatory immune response is therapeutically beneficial, for example in treatment of autoimmune diseases such as insulin dependent diabetes mellitus, lupus erythematosis, multiple sclerosis, and graft rejection.

Harnessing an individual's immune system to selectively produce endogenous cytokines and chemokines may provide a better route to immunotherapy. Expression of endogenous cytokines and chemokines, modulated by the host within the entirety of the immune system, may provide the appropriate context to achieve efficacy without the requirement for repeated dosing or the problems of cytokine/chemokine toxicity. Furthermore, the selective enhancement of a cell population may prove to be the ideal delivery system for such a potent cytokine/chemokine. Inherent in the immune cell repertoire is the ability to traffic within the body to sites of inflammation. This therapeutic approach avoids the problems associated with systemic administration of potent cytokines/chemokines, and better mimic the naturally localized action of this immune mediator.

This summary of the invention does not necessarily describe all features of the invention.

### BRIEF DESCRIPTION OF THE DRAWINGS

These and other features of the invention will become more apparent from the following description in which reference is made to the appended drawings wherein:

FIGURE 1 is a schematic showing the T regulatory cell hypothesis.

FIGURE 2 is a schematic showing the events that may occur when interactions between an inflammatory compound, dendritic cells, and T cells lead to inflammation or adaptive immunity.

FIGURE 3 shows a pro-inflammatory *mono*SPA of the present invention.

FIGURE 4 shows a pro-inflammatory *mono*SPA of the present invention.

FIGURE 5 shows a pro-inflammatory *poly*SPA of the present invention.

FIGURE 6 shows a pro-inflammatory *poly*SP*A* of the present invention.

FIGURE 7 shows a suppressive *mono*SPA of the present invention.

FIGURE 8 shows, a suppressive *poly*SPA of the present invention.

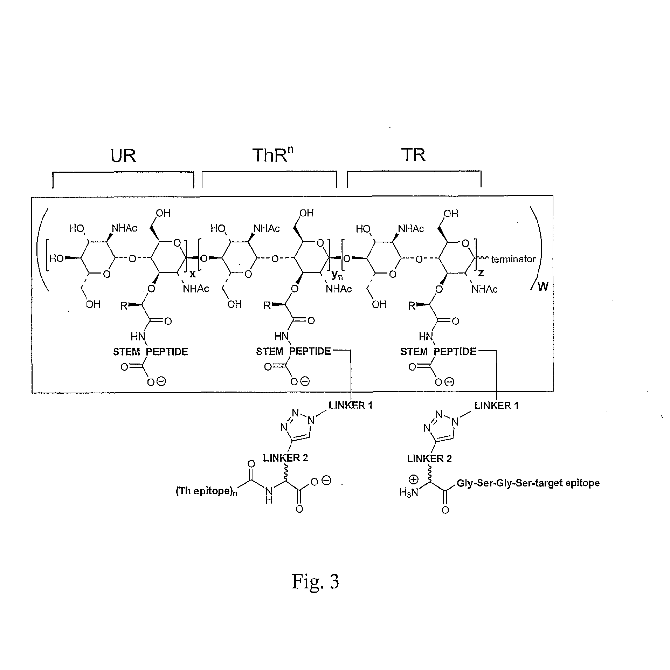

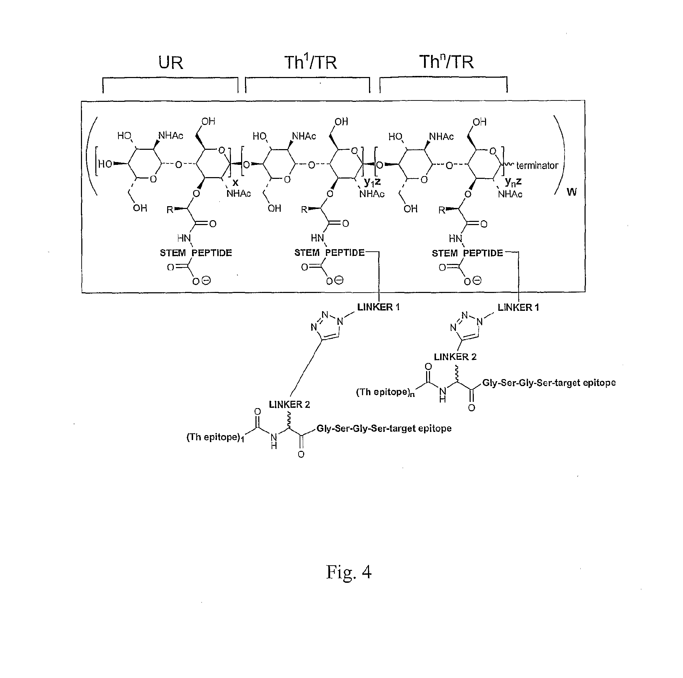

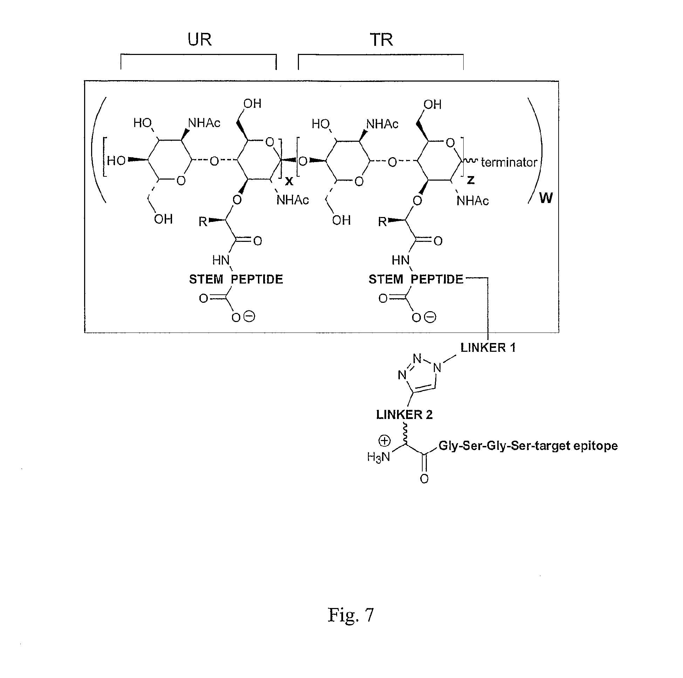

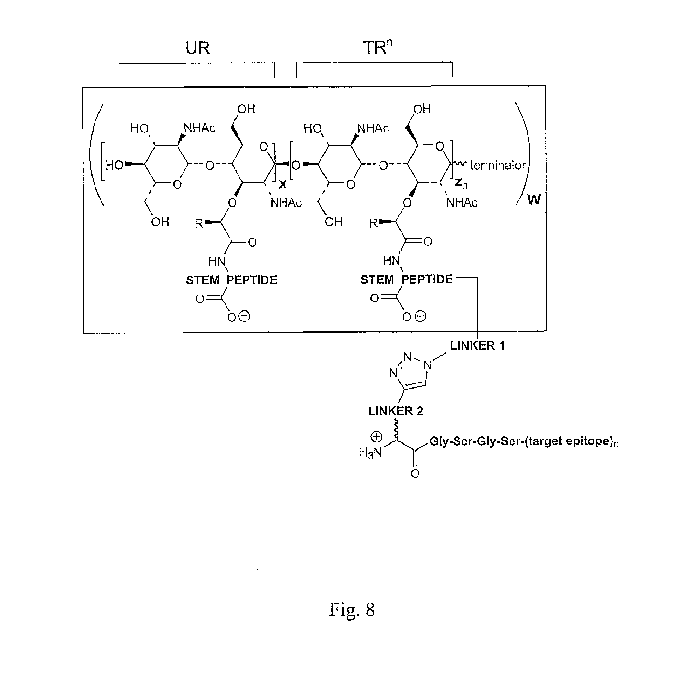

In FIGURES 3 to 8, the box denotes the TLR2 binding domain of the SPAs. Also, the mole fraction of each type of monomeric unit is designated as a subscript (x, yₙ, zₙ, or yₙzₙ). A mole fraction of 0.6 indicates that the given monomeric unit exists as 60% of the repeat units in the SPA. The designation of the mole fraction of unsubstituted repeat units (UR) is x (Figures 7 and 8); for example, if x = 0.4, the UR exists as 40% of the monomeric units in the SPA. The designation of the mole fraction of Th epitope repeat units (ThR) species is yₙ; for example, if yₙ = 0.15, the n^{th} different species ofThR exists as 15% of the monomeric units in the SPA. The designation of the mole fraction of target epitope repeat units (TR) species is zₙ; for example, if zₙ = 0.20, the n^{th} different species of TR exists as 20% of the monomeric units in the SPA. The designation of the mole fraction of combined Th/target epitope repeat units (Th/TR) species is yₙzₙ; for example, if yₙzₙ = 0.17, the n^{th} different species of Th/TR exists as 17% of the monomeric units in the SPA. In the case of a *monoSPA,* the designation of the mole fraction of a TR may be z₁, or z (see Figures 3 and 7); similarly, the mole fraction of a Th/TR species in a *monoSPA* may be yₙz₁, or yₙz (see Figure 4).

A person of skill in the art will recognize that the sum of the mole fractions must be equal to 1.00, i.e., the sum of x+y+y₁ +y₂ +... + yₙ + z + z₁ + z₂ + ... + zₙ, (as the case may be) = 1.00.. Since the rate of enzymatic polymerization of the various repeat units varies little, if at all, with substitution, UR, ThR, TR and/or Th/TR are evenly distributed along the carbohydrate axis of the polymer according to their respective mole fractions in the composition. Note that UR, ThR, TR and/or Th/TR can exist in any order within the polysaccharide as a consequence of the random nature of formation of the copolymer.

W is the total number of monomeric units in the SPA polymer. The number W may be between 10 and 375, and may be more generally described as a centre of distribution lying between about 130 and about 180.

### DETAILED DESCRIPTION

Definitions

As used herein, unless indicated otherwise, the following terms have the following meanings:

"Ac" means CH₃C(O)-.

"Alkyl" means an aliphatic hydrocarbon group that may be straight or branched having about 1 to about 20 carbon atoms in the chain, or any amount therebetween; for example, the hydrocarbon group may have about1, 2, 3, 4, 5, 6, 7, 8, 9, 10, 11, 12, 13, 14, 15, 16, 17, 18, 19, or 20 carbon atoms, or any amount of carbon atoms in a range defined by any two amounts defined herein. In a non-limiting example, the alkyl group may have about 1 to about 12 carbon atoms in the chain, or may be a lower alkyl. Branched means that one or more lower alkyl groups such as methyl, ethyl or propyl are attached to a linear alkyl chain. "Lower alkyl" indicates a hydrocarbon group having about 1 to about 5 carbon atoms, or any amount therebetween, in a straight or branched chain; for example, that lower alkyl may have about 1, 2, 3, 4, or 5 carbon atoms.

"Amino acid" refers to an amino acid selected from the group consisting of natural and unnatural amino acids. Amino acid is also meant to include -amino acids having L or D stereochemistry at the α-carbon; in a specific, non-limiting exaple, the amino acids are those possessing an α-amino group. Natural amino acids can be divided into the following four groups: (1) acidic (negatively charged) amino acids such as aspartic acid and glutamic acid; (2) basic (positively charged) amino acids such as arginine, histidine, and lysine; (3) neutral polar amino acids such as glycine, serine, threonine, cysteine/cystine, tyrosine, asparagine, and glutamine; and (4) neutral nonpolar amino acids such as alanine, leucine, isoleucine, valine, proline, phenylalanine, tryptophan, and methionine. "Unnatural amino acid" means an amino acid for which there is no nucleic acid codon; these amino acids may also be neutral, or may have a positive or negative charge. Non-limiting examples of unnatural amino acids include the D-isomers of the natural α-amino acids as indicated above; Aib (aminobutyric acid), βAib (3-amino-isobutyric acid), Nva (norvaline), β-Ala, Aad (2-aminoadipic acid), βAad (3-aminoadipic acid), Abu (2-aminobutyric acid), Gaba (γ-aminobutyric acid), Acp (6-aminocaproic acid), Dbu (2,4-diaminobutryic acid), α-aminopimelic acid, TMSA (trimethylsilyl-Ala), aIle (allo-isoleucine), Nle (norleucine), *tert*-Leu, Cit (citrulline), Orn, Dpm (2,2'-diaminopimelic acid), Dpr (2,3-diaminopropionic acid), α- or β-Nal, Cha (cyclohexyl-Ala), hydroxyproline, Sar (sarcosine), and the like; cyclic amino acids; N^{a}-alkylated amino acids such as MeGly (N^{a}-methylglycine), EtGly (N^{a}-ethylglycine) and EtAsn (N^{a}-ethylasparagine); and amino acids in which the α-carbon bears two side-chain substituents. The names of natural and unnatural amino acids and residues thereof used herein follow the naming conventions suggested by the IUPAC Commission on the Nomenclature of Organic Chemistry and the IUPAC-IUB Commission on Biochemical Nomenclature as set out in "Nomenclature of a-Amino Acids (Recommendations, 1974) *"* Biochemistry, 14(2), (1975).

"Amino acid residue" means the individual amino acid units incorporated into a peptide, or peptide portion of a molecule, through an amide linkage.

"Peptide" means a polymer comprising amino acid residues joined together through amide bonds.

"Net charge" means the arithmetic sum of the charges in an ionic species. A person of skill in the art would be familiar with the determination of net charge. "Zwitterion" refers to a unimolecular dipolar ion or polypolar ion within the polysaccharide monomeric unit including, for example, molecules with net negative, positive or neutral charges.

"Conservative amino acid substitution" refers to an amino acid substitution within a protein or peptide to produce a resultant peptide that retains peptide structure and biological functionality. Various factors can be considered in making such changes, including the hydropathic index and hydrophilicity of amino acids. Another factor that may be used in considering conservative amino acid mutations is the relative similarity of the amino acid side-chain substituents, which takes into account the hydrophobicity, hydrophilicity, charge, size, etc. Conservative amino acid substitutions resulting in silent changes within peptides may be selected from other members of the class to which the naturally occurring amino acid belongs, as described above.

The relative hydropathic character of amino acids contributes to the secondary structure of the resultant peptides and polypeptides, which in turn affects the interaction of the peptides or polpeptides with molecules such as enzymes and cellular receptors, etc. Based on its hydrophobicity and charge characteristics, each amino acid has been assigned a hydropathic index as follows: isoleucine (+4.5); valine (+4.2); leucine (+3, 8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate/glutamine/aspartate/asparagine (-3.5); lysine (-3.9); and arginine (-4.5). Similarly, like amino acids can also be substituted on the basis of hydrophilicity. The following hydrophilicity values have been assigned to amino acids: arginine/lysine (+3.0); aspartate/glutamate (+3.0±1); serine (+0.3); asparagine/glutamine (+0.2); glycine (0); threonine (-0.4); proline (-0.5±1); alanine/histidine (-0.5); cysteine (-1.0); methionine (-1.3); valine (-1.5); leucine/isoleucine (-1.8); tyrosine (-2.3); phenylalanine (-2.5); and tryptophan (-3.4). As would be recognized by a person of skill in the art, an amino acid in a peptide, polypeptide can be substituted by another amino acid having a similar hydropathic index or hydrophilicity score and still produce a resultant peptide having similar biological activity. In making such changes, amino acids having hydropathic index or hydropathic indices within ±2 are generally substituted for one another; for example, an amino acid may be stubstituted by another amino acid having a hydropathic index or hydrophilicity score within ±1, or ±0.5.

"Microbe" means any free-living unicellular organism. Non-restrictive examples include protozoa, parasites, bacteriae including mycobacteria, archeae, mycoplasmas and chlamydiae.

"Non-immune cell" means a cell that is not normally involved in immune responses but that may have the capacity to be modulated by products of the immune system.

"Immune cell" means any cell capable of responding or mounting a response within the entirety of the host immune system. Generally these cells are referred to as "white blood cells" but are not necessarily limited to this category. Examples of immune cells include, but are not limited to, T and B cells, monocytes, macrophages, natural ' killer cells, dendritic cells, antigen presenting cells, and polymorphonuclear leukocytes.

"T regulatory cells" or "T_{regs}" refers to a unique lineage of immunoregulatory T cells that potently suppress inflammatory effector T cells *in vitro* and *in vivo*. T_{regs} are characterized by expression of certain cell surface markers including, for example, CD4 and CD25 (CD4+/CD25+),

"Immune response" means either a pro-inflammatory or anti-inflammatory response of the immune system.

The terms "inflammation," "inflammatory response," "pro-inflammatory response," or the like, refer to the complex bodily process initiated by tissue damage, either endogenous or exogenous. Inflammatory response to such damage involves the induction of soluble factors such as cytokines including, but not limited to, interleukin-(IL-) 1, IL-6, and tumor necrosis factor (TNF)-α, as well as chemokines including, but not limited to, IL-8, interferon-y, and macrophage induction protein (MIP)-1β. Several immune cell populations also participate in the inflammatory response, including, but not limited to neutrophiles, macrophages, and lymphocytes. Although inflammation may be induced as, a protective function, numerous examples of inflammatory pathologies may be encountered (for example, but not limited to, inflammatory bowel disease, formation of excess post-surgical adhesions, and abscess formation).

The terms "anti-inflammation," "anti-inflammatory response," "suppressive response," or the like refer to any process by which an inflammatory response is attenuated or reversed. Such processes include, but are not limited to, induction of soluble mediators such as IL-10, or induction of cell populations such as regulatory T (T_{reg}) cells.

"IL10" is an endogenous mediator that is often involved in the downmodulation of inflammatory responses. Directed, endogenous generation of IL10 may maximize efficacy and minimize toxic effects.

The terms "modulate" or "modulation" or the like mean either an increase or a decrease in a selected parameter.

"Synthetic polysaccharide antigen" or "SPA" is synthetically produced, substantially pure, linear, uncrosslinked, polymer of *N*-acylglucosaminyl-β-[1,4]-*N-*acylmuramyl-peptide. The peptide may comprise one or more amino acids, natural or unnatural structures, D or L configuration. Substantially pure synthetic polysaccharide antigen as disclosed herein is essentially devoid of naturally occurring bacterial cell wall contaminants. Such antigens are not available from natural sources. SPAs include, but are not limited to, native, uncrosslinked, bacterial peptide sequences, or can be produced by total synthesis. Examples of SPAs include, but are not limited to **Compounds 1, 2,** and **3,** or *mono*SPA and *poly*SPAs disclosed herein, which are synthetic peptidoglycans (PGNs).

The SPAs encompassed by the present invention include, but are not limited to the SPAs as disclosed herein, and may also comprise additional substituents. Such substituents, however, should not materially affect the basic and novel characteristic of the SPAs in modulating immune responses as disclosed herein, nor their quantitative effect compared to those of the corresponding SPAs disclosed herein.

"Carbohydrate Core" refers to the SPA carbohydrate polymer comprised of β-[1,4]-linked repeat units of *N*-acetylglucosaminyl-β-[1,4]-*N*-acetylmuramyl.

"Phytanyl" refers to the lipid component of the SPA immediate synthetic precursor. It is the fully saturated hydrocarbon comprising four prenyl units (C₂₀) arranged in the usual "head-to-tail" (unbranched-to-branched) orientation, with the connection point at the unbranched terminus.

"Terminal group" or "terminator": The synthetic polymers of the present invention terminate at a muramic acid residue with a free reducing anomeric alcohol. It will be recognized by those skilled in the art that the N-acetylmuramyl tennini, being glucopyranosyl in structure, may be treated with an aryl amine to form C-1 *N*-aryl derivatives and with aryl hydrazines to form C-1 hydrazones. Furthermore, limited enzymatic digestion of the synthetic polymers with a lytic transglycosylase (e.g., Dijkstra et al. (1994) Curr. Opin. Struct. Biol. 4:810) will produce termini with muramyl-[1,6]-anhydro linkages which can be used for chemical modifications of the resulting anomeric carbons.

"Stem Peptide" refers to the peptide that extends N to C from the lactyl carbonyl (muramyl) function of the carbohydrate core. The stem peptides are muramyl substituents of the SPA carbohydrate core.

"Epitope" means the portion of an antigen that defines specificity, *i.e.,* the antigenic determinant. An epitope may be, for example, a peptide or a carbohydrate.

"T-helper (Th) Epitope" means an antigenic determinant that, in the context of MHC class II, induces an activation of CD4+ T cells which then induce clonal expansion of CD8+ cytotoxic T lymphocytes (CTL) and/or antibody production from B cells.

"Generic Th Epitope" refers to certain T helper (Th) epitope-containing peptides that are promiscuous or permissive (generic), and which can be presented in the context of a majority of MHC class II haplotypes such that they induce strong CD4+ Th responses and/or CD8+ CTL responses and/or antibody production in the majority of the outbred human or other mammalian populations.

"Target Epitope" means an antigenic determinant that drives expansion and activation of specific CD8+ CTL clones ("CTL epitope") or antibody production from B cells ("B cell epitope"). CTL epitopes and B cell epitopes are particular types of target epitopes.

"Valency" refers to the number of target epitopes contained in a synthetic polysaccharide antigen (SPA), excluding the number of Th epitopes contained in the SPA.

"Monovalent" means display of one or more copies of a single antigenic determinant or epitope along the polymeric backbone of an SPA.

"Polyvalent" means display of one or more copies each of more than one different antigenic determinants or epitopes along the polymeric backbone of an SPA.

"Monovalent synthetic polysaccharide antigen" or "*mono*SPA" is defined herein as an SPA displaying one or more disaccharide repeat unit species that have been modified to contain a single species of target epitope. The antigenic determinant, or epitope, may be present in multiple copies within a single SPA molecule.

"Polyvalent synthetic polysaccharide antigen" or "*poly*SPA" is defined herein as an SPA displaying two or more different disaccharide repeat unit species that have been modified to contain one distinct target epitope each. Each target epitope may be present in one or more copies within a single SPA molecule. The *poly*SPA comprises two or more different target epitopes.

"Pharmaceutically acceptable salts" refers to the relatively non-toxic, inorganic and organic acid addition salts, and base addition salts, of compounds of the present invention. These salts can be prepared *in situ* during the final isolation and purification of the compounds. In particular, acid addition salts can be prepared by separately reacting the purified compound in its free base form with a suitable organic or inorganic acid and isolating the salt thus formed. Exemples of acid addition salts include, but are not limited to the hydrobromide, hydrochloride, sulfate, bisulfate, phosphate, nitrate, acetate, oxalate, valerate, oleate, palmitate, stearate, laurate, borate, benzoate, lactate, phosphate, tosylate, citrate, maleate, fumarate, succinate, tartrate, naphthylate, mesylate, glucoheptonate, lactiobionate, sulphamates, malonates, salicylates, propionates, methylene-bis-β hydroxynaphthoates, gentisates, isethionates, di-p-toluoyltartrates, methanesulphonates, ethanesulphonates, benzenesulphonates, p-toluenesulphonates, cyclohexylsulphamates and quinateslaurylsulphonate salts, and the like. See, for example S.M. Berge, et al., "Pharmaceutical Salts," J. Pharm. Sci., 66, 1-19 (1977), which is incorporated herein by reference. Base addition salts can also be prepared by separately reacting the purified compound in its acid form with a suitable organic or inorganic base and isolating the salt thus formed. Base addition salts include, but are not limited to, pharmaceutically acceptable metal and amine salts. Suitable metal salts include the sodium, potassium, calcium, barium, zinc, magnesium, and aluminum salts. In a particular example, the metal salts are sodium and potassium salts. Suitable inorganic base addition salts are prepared from metal bases including, but not limited to sodium hydride, sodium hydroxide, potassium hydroxide, calcium hydroxide, aluminum hydroxide, lithium hydroxide, magnesium hydroxide, zinc hydroxide. Suitable amine base addition salts are prepared from amines which have sufficient basicity to form a stable salt, and include those amines that are frequently used in medicinal chemistry because of their low toxicity and acceptability for medical use, for example, but not limited to, ammonia, ethylenediamine, N-methyl-glucamine, lysine, arginine, ornithine, choline, N,N'-dibenzylethylenediamine, chloroprocaine, diethanolamine, procaine, N-benzylphenethylamine, diethylamine, piperazine, tris(hydroxymethyl)-aminomethane, tetramethylammonium hydroxide, triethylamine, dibenzylamine, ephenamine, dehydroabietylamine, N-ethylpiperidine, benzylamine, tetramethylammonium, tetraethylammonium, methylamine, dimethylamine, trimethylamine, ethylamine, basic amino acids, e.g., lysine and arginine, and dicyclohexylamine, and the like.

"Substantially pure" refers to a purity in the range of from about 90% to about 100%, or any percentage therebetween; for example, "substantially pure" may be a purity of about 90%, 91%, 92%, 93%, 94%, 95%, 96%, 97%, 98%, 99%, and 100%, or any purity in a range defined by any two percentages herein. For example, "substantially pure" may be from about 95% to about 100% or from about 97% to about 100% pure. Compounds of the present invention can be obtained in substantially pure or isolated form, free from the bulk of biological contaminants, including other molecules having immunomodulatory activity, that are customarily present in preparations of peptidoglycans isolated from natural bacterial sources.

"Adjuvant" is a substance that, when combined with an immunogen, enhances the immune response against the immunogen.

The term "biomarker" means a marker of a specific activity that correlates with the administration of a drug. Non-limiting examples of biomarkers include a cell surface receptor, a soluble mediator, an mRNA message, or an *in vivo* response that is modulated and that can be measured.

"Effective amount" refers to an amount of a compound or composition of the present invention effective to produce the desired or indicated immunologic or therapeutic effect.

The terms "patient" or "subject" refers to mammals and other animals including humans and other primates; companion, zoo, and farm animals, including, but not limited to, cats, dogs, rodents, horses, cows, sheep, pigs, goats; poultry; etc.

**Antigen Non-Specific SPAs**

Antigen non-specific SPAs have been described in WO 2005/03588 and WO 2003/075953 (which are both incorporated herein in their entirety). They are linear, non-crosslinked polymers, and include homopolymers and copolymers of various types. These polymers can be accessed through chemo-enzymatic total synthesis, for example from N-acetyl-glucosamine. Furthermore, depending on their structure, compounds of Formula I can either be inflammatory or anti-inflammatory.

The linear, non-crosslinked polymers of Formula I 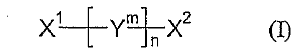 comprise n independent monomeric units of Y^{m}. X¹ and X² are independently H or a terminator. The subscript n, representing the number of momomeric units of Y^{m} in the polymer, is a single integer in the range from about 2 to about 375, or any amount therebetween; for example, the number of monomers Y^{m} may be about 2, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 300, 305, 310, 315, 320, 325, 330, 335, 340, 345, 350, 355, 360, 365, 370 or 375, or any amount therebetween, or any amount in a range defined by any two amounts defined herein. The superscript m, representing the position of a particular monomeric unit Y^{m} in the polymer sequentially from non-reducing terminus to reducing terminus, is a series of integers from 1 to n. In a non-limiting example, when n=2, there are two monomeric units: Y¹ and Y²; when n=3, there are three monomeric units: Y¹, Y² and Y³; or when n=375, there are 375 monomeric units: Y¹, Y², Y³,..., Y³⁷⁴ and Y³⁷⁵. Y¹ is directly attached to X¹ while Yⁿ is directly attached to X².

Each monomeric unit Y^{m} (i.e., each of Y¹, Y² ... Yⁿ⁻¹ and Yⁿ) is independently selected, such that they can all be the same, all be different, or any combination thereof. Thus, the invention includes homopolymers (*i.e.,* all monomers are the same) and copolymers (*i.e.*, two or more different monomers). The copolymers can be random copolymers, block copolymers or alternating copolymers, as defmed in WO 2005/035588, incorporated herein by reference.

In the polymers of Formula I, each monomeric unit of Formula Y^{m} is independently:

### (a) a group of Formula IIa, when Y^{m} is not Yⁿ

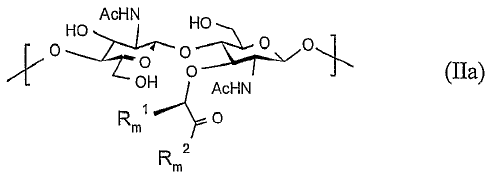

wherein the reducing end of the monomer is in the β configuration; or

### (b) a group of Formula IIb, when Y^{m} is Yⁿ

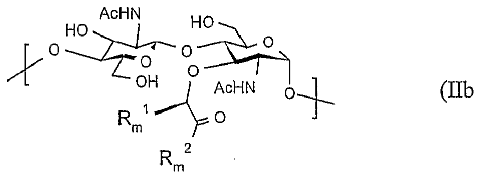

wherein the reducing end of the monomer may be in the α or β configuration (the α configuration is shown above).

Each monomeric unit of Formula Y^{m} comprises two independent sets of variables: Rₘ¹ and Rₘ², as follows:
Set 1: R₁¹, R₂¹, R₃¹,...,Rₙ₋₁¹ and Rₙ¹
Set 2: R₁², R₂², R₃²,...,Rₙ₋₁² and Rₙ²

Within each set, the variables are independently selected to be all the same, all different, or any combination thereof. That is, each of R₁¹, R₂¹, R₃¹,...,Rₙ₋₁¹ and Rₙ¹ is independently selected. Likewise, each of R₁², R₂², R₃²,...,Rₙ₋₁² and Rₙ² is independently selected.

Each variable Rₘ¹ (i.e., R₁¹, R₂¹, R₃¹,...,Rₙ₋₁¹ and Rₙ¹) may be H or lower alkyl; each variable Rₘ² (R₁², R₂², R₃²,...,Rₙ₋₁² and Rₙ²) may be-OH or -NH₂, an amino acid residue, or a peptide comprising 2 to 10 amino acid residues, wherein:
(i) each amino acid residue is independently in the D or L configuration;
(ii) each amino acid residue is unsubstituted or substituted with one or more groups selected from halo, alkyl, hydroxy, alkoxy, phenoxy, CF₃, amino, alkylamino, dialkylamino, -C(O)Oalkyl and -NO₂; and
(iii) the amino acid residues are independently joined at the α or γ carboxyl groups, and at the α or ε amino groups, or any combination thereof,
or pharmaceutically acceptable salts thereof.

Inflammatory compounds

Some of the compounds of Formula I induce an inflammatory response, for example, where one or more of the monomeric units of Y^{m} is:

### (a) a group of Formula IIIa, when Y^{m} is not Yⁿ

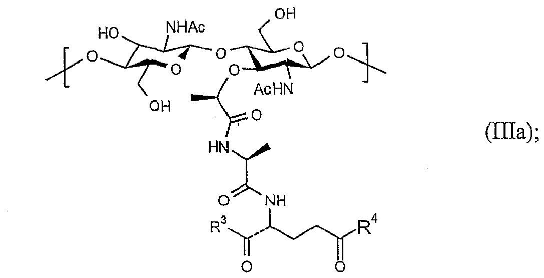

wherein the reducing end of the monomer is in the β configuration; or

### (b) a group of Formula IIIb, when Y^{m} is Yⁿ

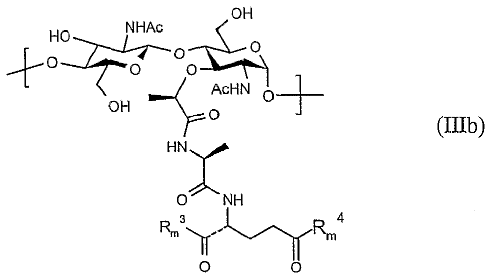

wherein the reducing end of the monomer may be in the α or β configuration (the α configuration is shown above).

wherein:
each of R₁³, R₂³,...Rₙ₋₁³ and Rₙ³ is independently -OH or -NH₂;
each of R₁⁴, R₂⁴,...Rₙ₋₁⁴ and Rₙ⁴ is independently -OH or -NH₂, an amino acid residue, or a peptide comprising 2 to 8 amino acid residues, wherein:
   (i) each amino acid residue is independently in the D or L configuration;
   (ii) each amino acid residue is unsubstituted or substituted with one or more groups selected from halo, alkyl, hydroxy, alkoxy, phenoxy, CF₃, amino, alkylamino, dialkylamino, -C(O)Oalkyl and -NO₂; and
   (iii) the amino acid residues are independently joined at the α of γ carboxyl groups, and at the α or ε amino groups, or any combination thereof.

These inflammatory compounds have a pendant carboxylate or carboxamide group and are referred to herein as compounds of Formula V. Examples include **Compounds 2** and **3,** and polymers of GMDP and GMDP-A.

**Compound 2,** which is representative of compounds of Formula V of the present invention, is an example of a pro-intlammatory immunomodulator. This molecule activates TLR2 and induces production of the pro-inflammatory cytokine TNF-α by human PBMCs. The pro-inflammatory activity of **Compound 2** is significantly less than the potent inflammatory activity of natural peptidoglycans isolated from bacterial sources. This difference is most likely due to the presence and activities of numerous biological contaminants present in the heterogeneous material isolated from bacteria.

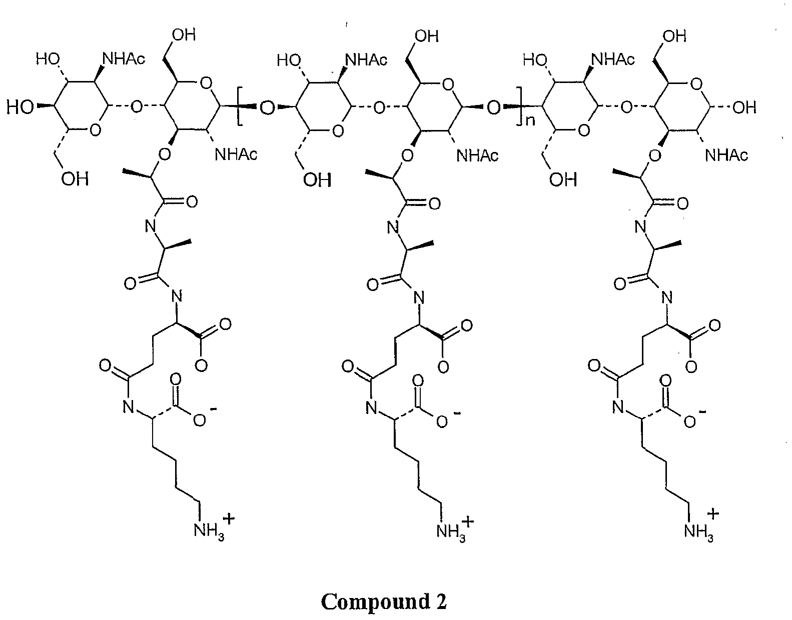

The disaccharide monomers GMDP (N-acetylglucosaminyl-N-acetylmuramyl-L-alanyl-D-isoglutamine) and GMDP-A (N-acetylglucosaminyl-N-acetylmuramyl-L-alanyl-D-glutamic acid), of the following structures: 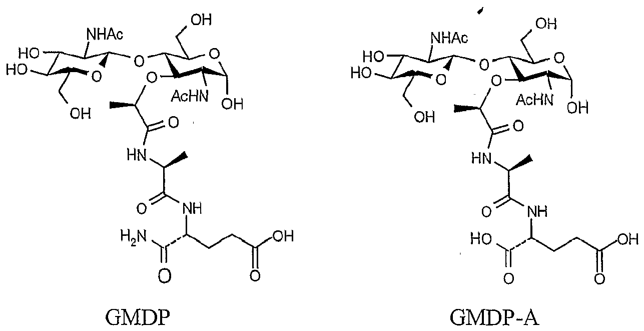 have been reported to induce an inflammatory response (see, e.g., U.S. Patent 4,395,399).

Similarly, commercially available samples of polymeric bacterial peptidoglycan (*Staphylococcus aureus,* Sigma; *Streptococcus pyogenes,* Lee Laboratories) are potently inflammatory (*Staphylococcus* > *Streptococcus*). While these materials are heterogeneous in composition, smaller disaccharide fragments (some of which have peptide crosslinks) have been purified by HPLC and characterized, and are also inflammatory. The inflammatory potency of these materials is reportedly dependent on structure (Tuomanen et al. (1993) J. Clin. Invest. 92:297). The smallest fragment of peptidoglycan that reportedly has biological activity is muramyl dipeptide, or MDP, and its biological activity is inflammatory in nature (Chedid (1983) Microbio. Immunol. 27:723). In fact, the MDP and MDP-A motifs, shown below, are a common feature of known inflammatory compounds:

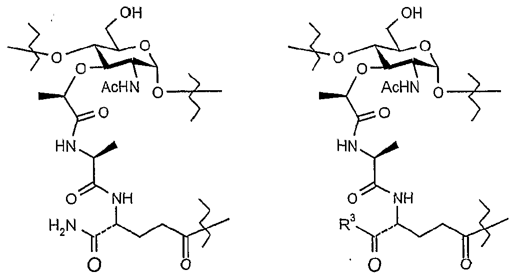

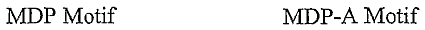

At a minimum, compounds of Formula I must include one of the following motifs to induce an inflammatory response (WO 2005/035588, incorporated herein by reference):

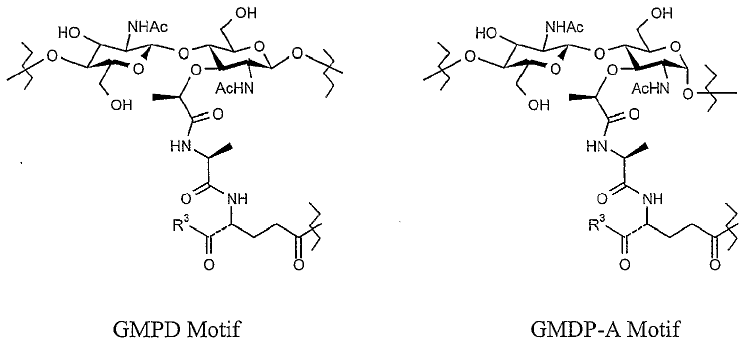

If these motifs are absent or modified, the polymer may induce an anti-inflammatory repsponse. If the second amino acid (D-iso-Glu or D-iso-Gln) is missing the pendant carboxyl, or if the pendant carboxyl is of the L configuration, inflammatory activity is abolished (Girardin et al. (2003) J. Biol Chem. 278:8869). Addition of one or more of the remaining three amino acids (Lys-D-Ala-D-Ala) results in retention of activity. It has been shown (WO 2005/035588) that **Compound 2** produces pro-inflammatory responses from human peripheral blood mononuclear cells. Its polymeric structure is -[NAG-NAM- tripeptide]ₙ, wherein n is an integer whose distribution is centered around *ca*. 135, and the tripeptide is a native bacterial sequence (Ala-D-iso-Glu-Lys).

**Compound 3** is another pro-inflammatory (stimulatory) synthetic bacterial peptidoglycan prepared from *N*-acetylglucosamine by chemo-enzymatic total synthesis using the methodololgy of WO 2003/075953. Its glycan backbone is comprised of β-[1,4]-linked *N*-acetylglucosaminyl-β-[1,4]-*N*-acetyl-muramyl repeat units wherein the lactyl substituent R may be H or lower (C₁-C₅) alkyl, preferably methyl. The methyl or lower alkyl substituent is preferably of the D configuration.

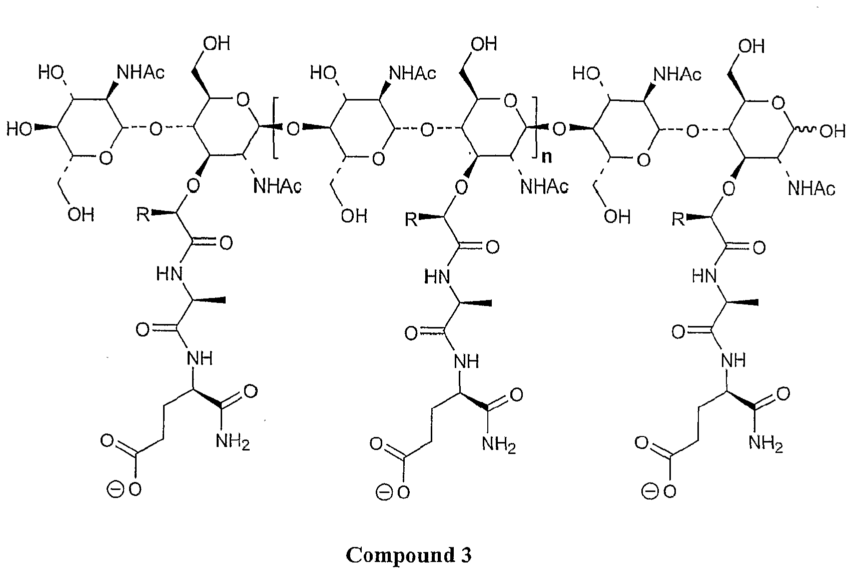

Molecules of this type exist as molecular weight distributions centered around *ca*. 130-180 repeat units (n = 130-180). The polymers are hygroscopic white powders that are soluble in water or saline. The stem peptide attached to each disaccharide repeat unit can contain from about one to about five amino acids. Position one may be occupied by alanine, a lower alkyl (C₁-C₅) homologue of alanine, or glycine; for example, but not intending to be limiting, alanine or its homologues are of the L-configuration at the α-carbon. Position 2 may be occupied by glutamic acid or glutamine; these amino acids may be of the D-configuration at the α-carbon, and the amide, which may be a primary amide, is may be in the *iso* (non-protein) position. Conservative amino acid substitution is contemplated in positions one and 2 (N to C from the lactyl carbonyl). Position 3 may be occupied by any α-amino acid, natural or unnatural; in a non-limiting example, lysine or diaminopimelic acid is at position 3. Position 4 may be occupied by any α-amino acid, natural or unnatural; in a non-limiting example, position 4 is occupied by D-alanine. Position 5 may be occupied by any α-amino acid, natural or unnatural, in a non-limiting example, position 5 is D-alanine. **Compound 3** represents the peptide-minimal example of a stimulatory (pro-inflammatory) synthetic peptidoglycan.

Anti-inflammatory compounds

In contrast, some of the compounds of Formula I induce an anti-inflammatory response, for example, where the monomeric units Y^{m} are selected from a group of Formula IIa and Formula IIb, as defined above, with the proviso that the monomeric units are not (a) a group of Formula IIIa (as defined above) when Y^{m} is not Yⁿ; or (b) a group of Formula IIIb (as defined above) when Y^{m} is Yⁿ.

These anti-inflammatory compounds do not comprise the pendant carboxylate or carboxamide group, and are referred to herein as compounds of Formula VI. **Compound 1** is an example of an anti-inflammatory compound of Formula VI. It should be noted that **Compound 1** has the same structure as **Compound 2,** with the exception that **Compound 1** does not have the pendant carboxylate or carboxamide group.

**Compound 1** is an anti-inflammatory immunomodulator. It is a homopolymer of the indicated repeat unit, existing as a distribution of molecular weights centered around 150 kilodaltons. The polymer is a hygroscopic white powder that is soluble in water or saline.

Furthermore, it has been shown (WO 2005/035588), herein incorporated by reference) that **Compound 1** produces anti-inflammatory responses in a number of biological systems. This molecule is the same as **Compound 2** except that the second amino acid is missing its pendant carboxyl.

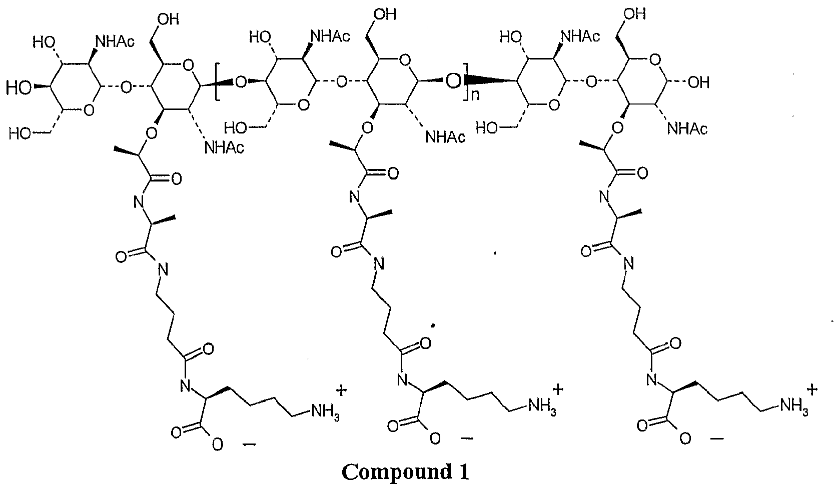

Natural peptidoglycan in the bacterial cell wall is a single covalently closed macromolecule that precisely defines the shape of a bacterial cell throughout the cell cycle. It is composed of a rigid axis of parallel polymeric peptidoglycan glycan strands wherein the repeat unit is β-[1,4]-linked *N*-acetylglucosaminyl-β-[1,4]-*N*-acetyl-muramylpentapeptide. The glycan strand is helical in shape with about four repeat units per complete turn of the helix. The more flexible pentapeptide axes extend N to C from the lactyl carboxyls of the muramic acid residues. The peptide is generally H₂N-Ala-D-*iso*-Glu (or *iso*-Gln)-Lys (or diaminopimelate, DAP)-D-Ala-D-Ala-COOH. The peptides may be crosslinked between Lys (or DAP) from a donor strand to the carbonyl of the penultimate D-Ala of an acceptor strand. The actual degree of crosslinking in a living cell varies with by genus, and is always less than 100%. In comparison, in the above compounds, there is no crosslinking in the peptides.

Effect of compounds of Formula I

Compounds of Formula I were prepared and analysed as disclosed in WO 2005/035588, which is incorporated herein by reference in its entirety. More specifically, **Compound 1** was prepared as an example of a coumpound of Formula VI, while **Compound 2** was prepared as an example of a coumpound of Formula V. The structural identity of the synthesized compounds was determined by size exclusion chromatography, ¹H NMR spectroscopy, enzymatic susceptibility, mass spectrometry, or a combination thereof.

The *in vitro* treatment of human peripheral blood mononuclear cells (PBMCs) with **Compound 1** resulted in negligible expression of inflammatory cytokines IL2, IFN-γ, TNF-α, IL6, or IL12, therefore indicating a failure to stimulate TLR2. However, the predominant response was the expression of the anti-inflammatory cytokine IL10. The expression of IL10 was observed late in the time course, detectable at day 5 and continuing to rise at day 8 to a concentration of approximately 80 pg/ml. These results indicated that compounds of Formula VI can selectively induce the expression of IL10 in PBMC cell culture, and may be efficacious in animal models of inflammation, and treating various types of inflammatory pathologies.

It was also determined, by means of an in vitro model system, whether compounds of Formula VI can activate NK-κB, a transcription factor for pro-inflammatory cytokines, *in vitro.* While varying concentrations of commercially-available natural peptidoglycans stimulated a significant induction of a luciferase NF-κB reporter in HEK293 cells, **Compound 1** showed a lack of luciferase NF-κB reporter activation at concentrations up to 500 µg/ml. These results indicated that unlike natural peptidoglycan, **Compound 1** does not induce activation of luciferase NF-κB reporter through TLR2. Further experiments showed that **Compound 1** elicited no luciferase NF-κB reporter signaling with any of the other TLR receptors.

The maturation state of dendritic cells incubated with **Compound 1** was tracked using the expression levels of of specific cluster differentiation markers. The data showed that incubation with **Compound 1** failed to change the staining profile from the immature dendritic cell state, indicating that this compound is capable of affecting the maturation of dendritic cells.

To evaluate whether the inhibition of maturation of DCs induced by **Compound 1** was due an inability to endocytose high molecular weight immunomodulatory polysaccharide antigens such as compounds of Formula VI, uptake studies were performed using a fluorescent derivative of **Compound 1** and confocal microscopy. The intracellular localization of **Compound 1** indicated that the internalized polymers are not spread throughout the cytoplasm, but are instead localized in discrete packets or vesicles, consistent with their presence in endocytic vacuoles. Furthermore, it was shown that immature DCs are capable of rapidly endocytosing fluorescently labeled **Compound 1,** and that the inability of the molecule to cause maturation of DCs is not due to recalcitrance to endocytic uptake thereof.

The capacity of a compound of Formula VI to interfere with the maturation of immature DCs was also examined. The results showed that **Compound 1** was able to interfere with LPS-induced maturation of iDCs. Specifically, surface expression of the co-stimulatory marker CD86 was decreased in the presence of **Compound 1,** while the other markers tested were essentially unchanged. Additional experiments also demonstrated that CD80, another marker of co-stimulation, was also decreased. Thus, the capability of compounds of Formula VI to influence the expression of costimulatory markers on the DC surefac suggests a mechanism of action for molecules of this type in the induction of toleragenic DCs. These anergic DCs could then induce T-cell anergy directly or through the activity of a Treg cell population.

It was also shown that human PBMC cultures treated with **Compound 1** did not respond by proliferation when compared to control cultures treated with polyclonal mitogens such as phytohaemagglutinin (PHA) or superantigens such as *Staphylococcus aureus* enterotoxin A (SEA) (see Example 3). However, incubation of human PBMCs with **Compound 2** did result in recognition and production of the pro-inflammatory cytokine TNF-α (see Example 3). Furthermore, when **Compound 1-**treated PBMC cultures were stimulated with anti-CD3 antibodies, there was a marked suppression in the proliferative capacity of the culture compared to that of untreated controls. Microarray analysis further revealed that PBMC cultures treated with **Compound 1** and anti-CD3 antibodies selectively upregulated the expression of IL10 and IL19 (an IL10 paralogue) messages in the CD3+ T cell population while downregulating several inflammatory cytokine messages such as IL17 and TNFb.

Taken together, these data indicate that compounds of Formula VI, such as **Compound 1,** inhibit the maturation of dendritic cells. An increase in the number of CD4+CD25+ cells present in PBMC cultures following treatment with compounds of Formula VI indicated that these compounds create a population of immature APCs that drive the stimulation of T regulatory cells within the culture. This was supported by the observation of suppression of proliferation of T cells in PBMC cultures stimulated with anti-CD3 antibodies following treatment with **Compound 1.** Immature dendritic cells have a unique capacity to drive the generation of Treg cells. Treg cells may then participate in the inhibition of inflammatory responses through cell-cell signaling as well as through the stimulation of IL10 expression from anergized T cells at the sites of inflammation.

The induction of Treg cells with suppressive function *in vitro,* as well as the late production of IL10 from human PBMCs led to an assessment of **Compound 1**'s ability to protect animals against the inflammatory formation of abscesses *in vivo.* Results showed that **Compound 1** produces considerable protection against the formation of abscesses at various doses. Protected animals show no deleterious effects of antigen administration, with few, if any, signs of fever and lethargy, which are common symptoms of inflammation, or of sepsis. Furthermore, post-surgical adhesion formation in rats treated with **Compound 1** was significantly limited, indicating that this polysaccharide antigen effectively protects rats from the formation of severe surgically induced adhesions, and suggests that compounds of Formula VI induce an anti-inflammatory effect *in vivo.*

Clinical evaluation of the safety and efficacy of immune modulators such as compounds of Formula VI requires a convenient biomarker. Therefore, a Guinea pig model of delayed type hypersensitivity (DTH) was developed to assess the ability of compounds of Formula VI to limit the localized inflammatory reaction in the skin. The antigen used to elicit inflammatory T cell activity is derived from Candida albicans (Candin). A reduction in the flare area in animals treated with **Compound 1** is observed compared to that of control animals.

The results obtained were in direct contrast to the body of literature characterizing the recognition of bacterial peptidoglycans by the immune system. Furthermore, the stimulation of an anti-inflammatory response by compounds of Formula VI was completely novel and unexpected in view of the current body of evidence regarding natural peptidoglycans, indicating that bacterial peptidoglycan is a potent inflammatory agent. Thus, while natural peptidoglycans are potently inflammatory, the compounds of Formula VI are anti-inflammatory. The discovery that compounds of Formula VI exhibit *in vitro* anti-inflammatory activity contrasted markedly with previously published observations on the activity of purified bacterial peptidoglycans.

In contrast to **Compound 1** which fails to stimulate TLR2, **Compound 2** and **Compound 3** bind and stimulate TLR2, thus inducing production of the pro-inflammatory cytokine TNF-α by human PBMCs. Thus, it appears that the structural differences between **Compound 1** and **Compounds 2** and **3** represent a fundamental structure/biological activity relationship in bacterial peptidoglycans. Therefore, **Compound 2** and **Compound 3,** have the characteristics necessary to function as adjuvants in human or other mammalian immunotherapy, however **Compound 1** is suppressive in its effect and is contraindicated for adjuvant applications.

Mechanism of Action of Synthetic Polysaccharide Antigens of Formula VI: The T Regulatory Cell Hypothesis

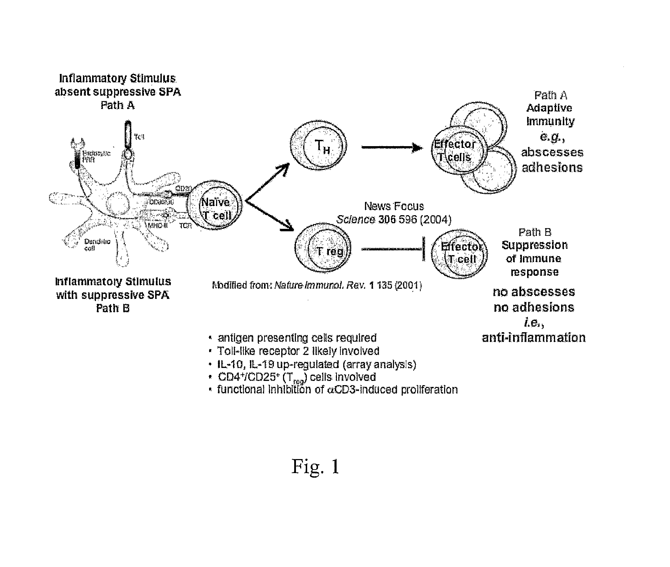

From the studies of the effect of compounds of Formula I, a mechanism of action of the synthetic polysaccharide antigens of Formula VI has emerged, and is summarized in Figure 1. Synthetic immunomodulatory polysaccharide antigens of Formula VI inhibit the maturation of dendritic cells. Immature dendritic cells (iDCs) express low CD80 and CD86 co-stimulatory molecules. In this state, iDCs have the unique ability to interact with naïve T cells and induce the generation of CD4+CD25+ Treg cells (pathway B). In the face of an inflammatory response, Treg cells interact with T effector cells through cell-cell dependent contact and inhibit the proliferative capacity of these T inflammatory effector cells. Further, contact between Treg cells and T effector cells renders the effectors anergic and stimulates these cells to express large amounts of IL10. Elicitation of IL10 expression in the former inflammatory T cell effectors serves to amplify the suppressive effects of direct Treg cell contact and broadens the protection against an ongoing inflammatory process. The inhibition of maturation of dendritic cells observed by the present investigators could also inhibit the clonal expansion of T effector cells through the lack of cognate interactions between these two cell types (pathway A). However, the data more compellingly supported the hypothesis that T regulatory cells are ultimately generated by the synthetic polysaccharide antigens of Formula VI of the present invention and afford protection against inflammatory pathologies.

Mechanism of Action of Synthetic Polysaccharide Antigens of Formula V: The Inflammatory Hypothesis

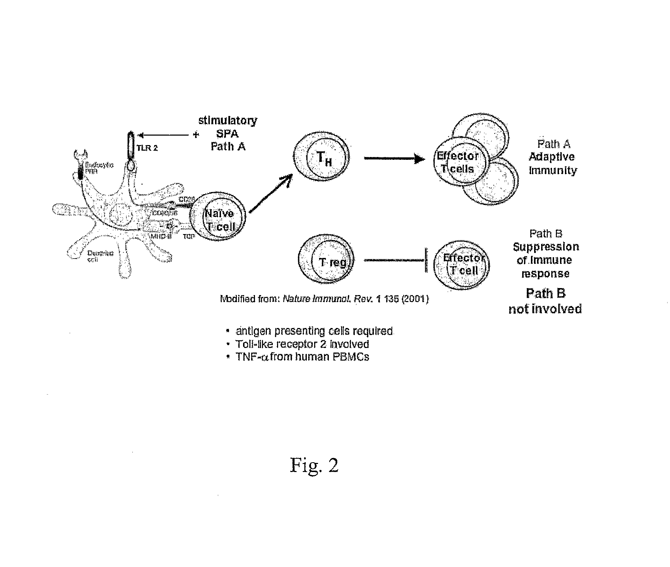

Compounds of Formula V, exemplified by **Compounds 2** and **3,** appear to stimulate an inflammatory response as evidenced by the production of TNF-α. **Compounds 2** and **3** may interact with immune cells in a fashion similar to that of either whole bacteria or bacterial cell wall antigens, most likely through the activation of TLR2. In this case, interactions between compounds of Formula V and TLR2-bearing cells stimulate characteristic markers of inflammation. This would suggest that inflammatory cells would come into play, as is the case following the detection of an invading pathogen. These concepts are summarized in Figure 2.

**Antigen-Specific SPAs**

The antigen non-specific SPAs described above serve to activate or suppress an inflammatory response in a non-specific manner. While this type of activation or suppression would affect a large number of T cells, a more targeted approach may be desired to suppress or activate a specific inflammatory response, by targeting a specific T cell population. To provide a targeted activation or suppression of inflammation, specific SPAs were developed based on the structure of the compounds of Families V and VI, described above. The suppressive SPAs comprise of a TLR2 binding domain based on the compounds of Formula VI, and a target epitope. The pro-inflammatory SPAs comprise a TLR2 binding domain based on the compounds of Formula VI, a target epitope, and a Th helper epitope that amplifies the inflammatory response.

Pro-Inflammatory *monoSPAs* and *polySPAs*

Synthetic pro-inflammatory SPAs according to the present invention comprise:
a TLR2-targeting synthetic PGN moiety (Figures 3 to 6, Box) that supplies the adjuvant function and provides glycopeptide backbone onto which a first epitope and a second epitope are each covalently attached;
the first epitope comprising one or more generic T helper epitope;
the second epitope comprising one or more than one target epitope;
the first and second epitopes are present in one or more copies each within the SPA
wherein each target epitope may be a peptide sequence or carbohydrate moiety, and wherein each target epitope is an immunogen to CD8+ T cells or B cells, or a pharmaceutically acceptable salt thereof.

Specific examples of pro-inflammatory SPAs are shown diagrammatically in Figures 3 through 6, but are not meant to be limiting in any manner.

Similar to the non-specific SPA, the SPA is a linear, non-crosslinked polymeric compound of Formula VII:

X¹-[-MO-]_{w}-X² (VII)

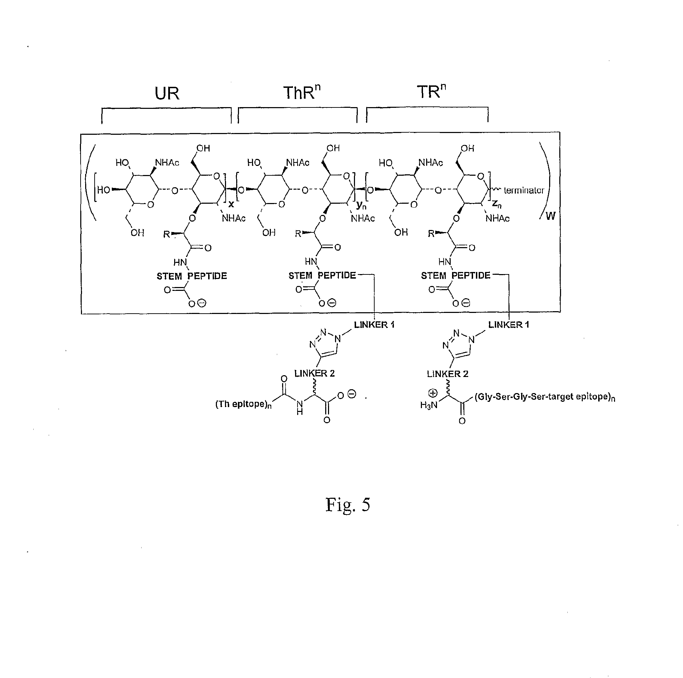

wherein
X¹ and X² are independently H or a terminator;
W represents the number of monomeric units (MO) in the polymer, and may be an integer in the range of from about 10 to about 375, or any amount therebetween; for example, n may be about 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 300, 305, 310, 315, 320, 325, 330, 335, 340, 345, 350, 355, 360, 365, 370 or 375, or any amount therebetween, or any amount in a range defined by any two amounts defined herein. In a further, non-limiting example, W may be described as a centre of distribution lying between about 130 and about 180, or any amount therebetween;
the monomeric units MO comprise:
unsubstituted repeat units (UR; see, for example Figures 3 to 6);
one or more more than one species of Th epitope repeat units (ThR; see, for example Figures 3 and 5); and
one or more more than one species of target epitope repeat units (TR; see, for example Figures 3 and 5).

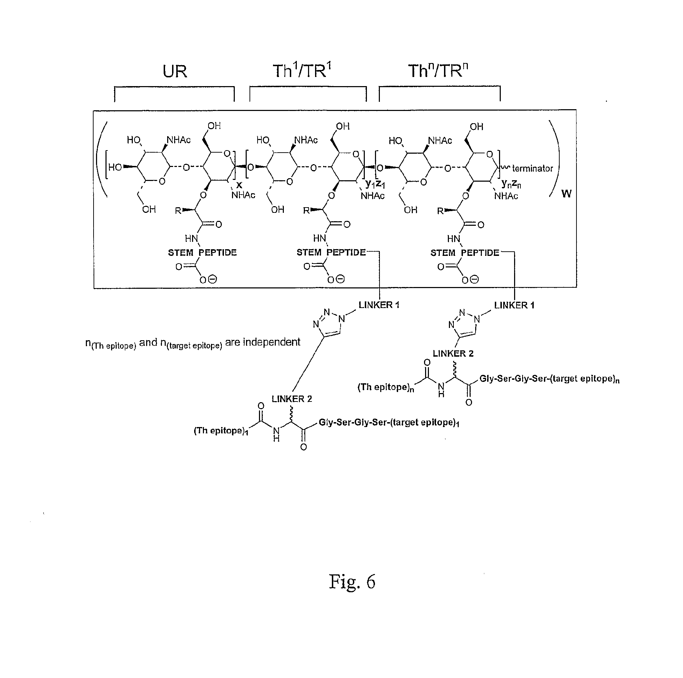

Alternatively, the one or more than one species of ThR and one or more than one species ofTR may be replaced with one or more than one species of combined Th/target epitope repeat units (Th/TR; see, for example Figures 4 and 6).

The pro-inflammatory SPAs may comprise from about 1 to about 180 different Th epitopes in the ThR or Th/TR species of the SPA molecule, or any amount therebetween. Each epitope is designated "(Th epitope)ₙ" (see Figures 3 to 6). For example, there may be about 1, 2, 3, 4, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, or 180 different Th epitopes in the ThR or Th/TR species of the SPA molecule, or any amount therebetween, or any amount in a range defined by any two amounts disclosed herein. Various types ofTh epitopes are contemplated by the present invention and non-limiting examples of suitable epitopes are described later in the present description. A person of skill in the art will recognize that the number of Th epitopes will determine the number of ThR or Th/TR species, as the case may be. For example, and without wishing to be limiting in any manner, if 4 different Th epitopes are used, the epitopes would be designated (Th epitope)₁, (Th epitope)₂, (Th epitope)₃, (Th epitope)₄, with each epitope present on its respective species ofThR, i.e. 4 different ThR species designated ThR¹ (carrying (Th epitope)₁), ThR² (carrying (Th epitope)₂), ThR³ (carrying (Th epitope)₃), and ThR⁴ (carrying (Th epitope)₄).

The number of target epitopes in the pro-inflammatory SPAs is independent from the number of Th epitopes. Thus, the SPA may comprise from about 1 to about 180 different target epitopes in the TR or Th/TR species of the SPA molecule, or any amount therebetween. Each epitope is designated "(target epitope)ₙ" (see Figures 3 to 6). For example, there may be about 1, 2, 3, 4, 5,10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, or 180 different target epitopes in the TR or ThR species of the SPA molecule, or any amount therebetween, or any amount in a range defined by any two amounts disclosed herein. Various types of target epitopes are contemplated by the present invention, including peptide or carbohydrate, CD8+ T cell or B cell epitopes, or any combination thereof. Non-limiting examples of suitable epitopes are described later in the present description. A person of skill in the art will recognize that the number of target epitopes will determine the number of TR or Th/TR species, as the case may be. For example, and without wishing to be limiting in any manner, if 3 different target epitopes are used, the epitopes would be designated (target epitope)₁, (target epitope)₂, and (target epitope)₃, with each epitope present on its respective species of TR, i.e. 3 different TR species designated TR¹ (carrying (target epitope)₁), TR² (carrying (target epitope)₂), and TR³ (carrying (target epitope)₃).

When a single species of target epitope is present, as shown in Figures 3 and 4, the SPA is a monovalent SPA, or *mono*SPA. When more than one species of target epitope is present, as shown in Figures 5 and 6, the SPA is a polyvalent SPA, or *polySPA.*

The optimum ratio of Th epitopes to target epitopes is determined empirically without undue experimentation.

Each species of monomeric unit is present in the SPAs in a given mole fraction designated as a subscript (x, yₙ, zₙ or yₙzₙ). For example, a mole fraction of 0,6 indicates that the given monomeric unit exists as 60% of the repeat units in the SPA. The designation of the mole fraction of unsubstituted repeat units (UR) is x; for example, if x = 0.4, the UR exists as 40% of the monomeric units in the SPA. The designation of the mole fraction of Th epitope repeat units (ThR) species is yₙ; for example, if yₙ = 0.15, the n^{th} different species of ThR exists as 15% of the monomeric units in the SPA. The designation of the mole fraction of target epitope repeat units (TR) species is Zₙ; for example, if zₙ = 0.20, the n^{th} different species of TR exists as 20% of the monomeric units in the SPA. The designation of the mole fraction of combined Th/target epitope repeat units (Th/TR) species is yₙzₙ; for example, if yₙzₙ = 0.17, the n^{th} different species of Th/TR exists as 17% of the monomeric units in the SPA.

A person of skill in the art will recognize that the sum of the mole fractions must be equal to 1.00, i.e., the sum of x + y + y₁ + y₂ + ... + yₙ + z + z₁ + z₂ +. .. + zₙ, (as the case may be) = 1.00. Since the rate of enzymatic polymerization of the various repeat units varies little, if at all, with substitution, UR, ThR, TR and/or Th/TR are evenly distributed along the carbohydrate axis of the polymer according to their respective mole fractions in the composition. Note that UR, ThR, TR and/or Th/TR can exist in any order within the polysaccharide as a consequence of the random nature of formation of the co-polymer.

To illustrate the relationship of the values described above, the following non-limiting example is set forth: a SPA polymer comprising a total of 50 monomeric units (i.e. W= 50). The SPA polymer has 2 Th epitopes ((Th epitope)₁ and (Th epitope)₂) and 4 target epitopes ((target epitope)₁, (target epitope)₂, (target epitope)₃, and (target epitope)₄); thus the polymer comprises 1 species of UR, 2 species of ThR, and 4 species of TR. If the the mole fraction of UR (i.e. x) is 0.4; the mole fraction of ThR carrying (Th epitope) (i.e. y₁) is 0.08; the mole fraction of ThR carrying (Th epitope)₂ (i.e. y₂) is 0.12; the mole fraction of TR carrying (target epitope)₁ (i.e. z₁) is 0.10; the mole fraction of TR carrying (target epitope)₂ (i.e. z₂) is 0.06; the mole fraction of TR carrying (target epitope)₃ (i.e. z₃) is 0.06; and the mole fraction ofTR carrying (target epitope)₄ (i.e. z₁) is 0.18; the polymer will comprise 40% UR, 8% ThR¹, 12% ThR², 10% TR¹, 6% TR², 6% TR³, and 18% TR⁴ (i.e. 20 UR monomers, 4 ThR¹ monomers, 6 ThR² monomers, 5 TR¹ monomers, 3 TR² monomers, 3 ThR³ monomers, and 9 TR⁴ monomers).

Another illustrative, non-limiting example, is of a SPA polymer comprising a total of 100 monomeric units (i.e. W= 100). The SPA polymer has 3 Th epitopes ((Th epitope)₁, (Th epitope)₂, and (Th epitope)₃) and 2 target epitopes ((target epitope)₁, and (target epitope)2); the polymer comprises 1 species ofUR, and 6 species of Th/TR. If the the mole fraction of UR (i.e. x) is 0.35; the mole fraction of Th/TR carrying (Th epitope)₁ and (target epitope)₁ (i.e. y₁z₁) is 0.13; the mole fraction of Th/TR carrying (Th epitope)₁ and (target epitope)₂ (i.e, y₁z₂) is 0.04; the mole fraction of Th/TR carrying (Th epitope)₂ and (target epitope)₁ (i.e. y₂z₁) is 0.15; the mole fraction of Th/TR carrying (Th epitope)₂ and (target epitope)₂ (i.e, y₂z₂) is 0.20; the mole fraction of Th/TR carrying (Th epitope)₃ and (target epitope)₁ (i.e. y₃z₁) is 0.08; and the mole fraction of Th/TR carrying (Th epitope)₃ and (target epitope)₂ (i.e. y₃z₂) is 0.05; the polymer will comprise 35% UR, 13% Th¹/TR¹, 4% Th¹/TR², 15% Th²/TR¹, 20% Th²/TR², 8% Th³/TR¹, and 5% Th³/TR² (i.e. 35 UR monomers, 13 Th¹/TR¹ monomers, 4 Th¹/TR² monomers, 15 Th²/TR¹ monomers, 20 Th²/TR² monomers, 8 Th³/TR¹ monomers, and 5 Th³/TR² monomers).

The antigen-specific pro-inflammatory SPAs of the present invention are co-polymers (i.e., two or more different monomers). The rate of enzymatic polymerization of the various monomeric units (UR, ThR, TR, and/or Th/TR) varies little, and thus the monomers may be evenly distributed along the length of the SPA copolymer, according to their respective mole fractions in the composition. A person of skill in the art would readily recognize that, while Figures 3 to 6 depict the monomers in a specific order within the SPA, the monomers may exist in any order within the copolymer as a result of the random nature of polymerization. Thus, the copolymers may be random copolymers, block copolymers or alternating copolymers. For example, and without wishing to be limiting, for a SPA comprising UR, one species of TR (TR¹), and one species of ThR (ThR¹), the polymer types may include:

| **Polymer Type** | **Example** |
|---|---|
| Random copolymer* | X¹-UR-ThR¹-TR¹-ThR¹-UR-TR¹-TR¹-ThR¹-UR-TR¹-X² |
| Block copolymer** | X¹-UR-UR-UR-ThR¹-ThR¹-ThR¹-TR¹-TR¹-TR¹-X² |
| Alternating copolymer* | X^{l}-UR-ThR¹-TR¹-UR-ThR¹-TR¹-UR-ThR¹-TR¹-X² |

| | |
|---|---|
| * the length of this copolymer may vary from that as shown; * * wherein each of the 'blocks' may be of varied length, and may be repeated throughout the copolymer; the length of this copolymer may also vary from that as shown | |

The pro-inflammatory *mono*SPAs and *poly*SPAs of the present invention are random linear co-polymers comprised of distinct types of β-[1,4]-linked *N-*acetylglucosaminyl-β-[1,4]-*N*-acetylmuramyl peptide repeat units. Conservative substitution is contemplated in the carbohydrate core. For example, and without wishing to be limiting, the lactyl methyl group may also be lower alkyl (C₁-C₅) or hydrogen. In a further non-limiting example, the oxygen-bearing carbon is in the D-configuration when an alkyl group is present.

In general, the various monomeric units (MO) can be described by the following structures:

UR (Formula VIII):

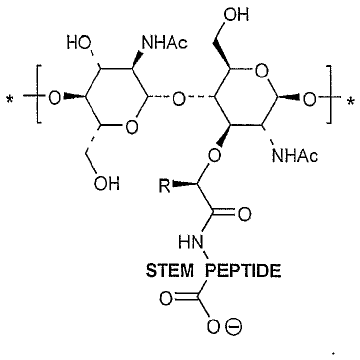

ThR (Formula IX):

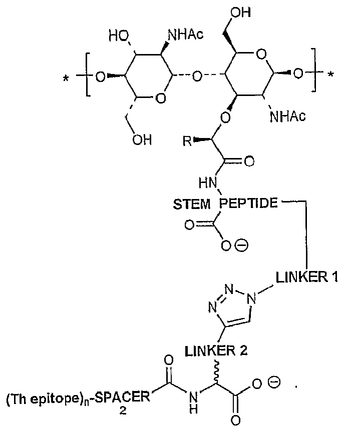

TR (Formula X):

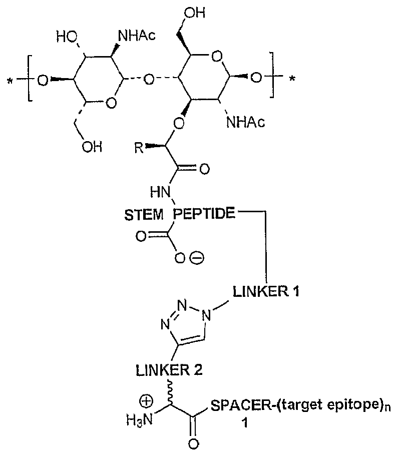

Th/TR (Formula XI):

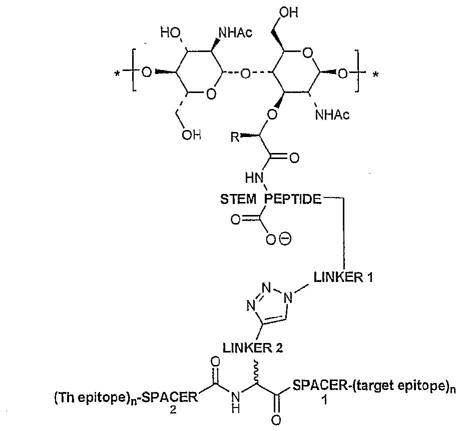

The R group of the monomeric units may be independently chosen, and may be either H or a lower alkyl (C₁-C₅).

The stem peptide of the unsubstitued repeat units (UR; see, for example Figures 3 to 6), the Th epitope repeat unit (ThR; see, for example Figures 3 and 5), the target epitope repeat units (TR; see, for example Figures 3 and 5), and the combined Th/target epitope repeat units (Th/TR; see, for example Figures 4 and 6) are independently selected and may each contain from about two to about five amino acids. The stem peptide may comprise any amino acid, natural or unnatural. For example, and without wishing to be limiting in any manner, the following amino acids may be used. Position 1 may be occupied by alanine, a lower alkyl (C₁-C₅) homologue of alanine, or glycine; in a further non-limiting example, the L-configuration is preferred at the α-carbon for alanine or its homologues. Glutamic acid, glutamine, or lower alkyl (C₁-C₅) glutamine secondary or tertiary amides may be at position 2; in a further non-limiting example, the D-configuration is preferred for the amino acids, and the pendant amide may be in the *iso* (non-protein) position. Position 3 may be occupied by any α-amino acid, natural or unnatural; in a further non-limiting example, lysine or diaminopimelic acid are at position 3. Position 4 may be occupied by any α-amino acid, natural or unnatural; in a further non-limiting example, the amino acid at position 4 is D-alanine. Position 5 may be occupied by any α-amino acid, natural or unnatural; in a further non-limiting example, D-alanine is at position 5. The amino acid residues may be independently joined at the α or γ carboxyl groups, and at the α or ε amino groups, or any combination thereof, provided that a pendant carboxylate or carboxamide group is present. In a specific, non-limiting example, the pendant carboxylate or carboxamide group is on amino acid at position 2. In addition, each amino acid residue of the stem peptide may be unsubstituted or substituted with one or more groups selected from halo, alkyl, hydroxy, alkoxy, phenoxy, CF₃, amino, alkylamino, dialkylamino, -C(O)Oalkyl and -NO₂.

LINKER1 and LINKER2 may be independently chosen, and may comprise any suitable linker known in the art. In a particular example, each linker may comprise from about 1 to about 6 segments, or any amount therebetween; for example, the linker may comprise 1, 2, 3, 4, 5, or 6 segments. Without wishing to be limiting, each segment may be chosen from -CH₂-, -CHR-, =CH-, and ≡CH-, where R is a lower alkyl. In the case where there are 3 to 6 segments, segments 1 to 4, when present, may also be chosen from -O-, NH-, -NR-, -S-, -SO-, and -SO₂-, provided that there are no contiguous heteroatom segments. In a specific, non-limiting example, LINKER1 may be the side chain of a lysine that is par of the stem peptide.

The connection between the stem peptide and LINKER 1 (see Figures 3 to 6) may each be independently made at any one of the amino acids of the stem peptide. In a non-limiting example, the connection between the stem peptide and LINKER 1 is made at position three of the stem peptide. The connector between LINKER 1 and LINKER 2 may be 1,4-[1,2,3-triazole] (Rostovtsev et al. (2002) Angew. Chem. Int. Ed. 114:2708) or any other connection chemistry known to those skilled in the art, for example, but not limited to thiolate/maleimide (Verez-Bencomo et al. (2004) Science 305:522) and amine/aldehyde reductive alklyation (Slovin et al. (1999) PNAS 96:5710). In a specific, non-limiting example, the target epitope is a carbohydrate, and the connector between LINKER1 and LINKER2 is amine/aldehyde reductive alkylation.

The SPACER1 for the target epitope may be from about one to about 10 amino acids in length, or any amount therebetween; for example, the spacer may be about 1, 2, 3, 4, 5, 6, 7, 8, 9, or 10 amino acids in length. The amino acids may be any natural or unnatural amino acid known in the art. In a specific, non-limiting example, the spacer may be Gly-Ser-Gly-Ser (see Figures 1-6), however, other amino acids within the spacer may be used if desired, and the spacer may be of a different length that as just described, for example from about 2 to about 10 amino acids, or any amount therebetween, for example from about 4 to about 8 amino acids, or any amount therebetween. In a specific, non-limiting example, the spacer is 4 amino acids in length. The spacer may be connected to the monomeric unit at its N-terminus (i.e., by its α-amino group) or by an ε amino group of a side chain of any one of the amino acids thereof, if present; for example, but not wishing to be limiting, the spacer is connected to the monomeric unit at amino acid at the α-amino group of position 1 of the spacer. The spacer is connected to the target epitope through either a peptide bond (if the eptope is a peptide) or through O-linked glycosylation (if the epitope is a carbohydrate).

The SPACER2 for the Th epitope may be from about 0 to about 10 amino acids in length, or any amount therebetween; for example, the spacer may be about 0, 1, 2, 3, 4, 5, 6, 7, 8, 9, or 10 amino acids in length. The amino acids may be any natural or unnatural amino acid known in the art. In a specific, non-limiting example, the spacer comprises 0 amino acids (see Figures 1-6), however, other amino acids within the spacer may be used if desired, and the spacer may be of a different length that as just described, for example from about 2 to about 10 amino acids, or any amount therebetween, for example from about 4 to about 8 amino acids, or any amount therebetween. The spacer may be connected to the monomeric unit at its N-terminus (i.e., by its α-amino group) or by an ε amino group of a side chain of any one of the amino acids thereof, if present; for example, but not wishing to be limiting, the spacer is connected to the monomeric unit at amino acid at the α-amino group of position 1 of the spacer. The spacer is connected to the Th epitope through either a peptide bond (if the eptope is a peptide) or through O-linked glycosylation (if the epitope is a carbohydrate).

The present invention also contemplates pro-inflammatory *mono*SPAs and *poly*SPAs that contain only Th epitopes. Theses particular SPAs can be potent general adjuvants.

Suppressive *mono*SPAs and *poly*SPAs

Synthetic suppressive SPAs of the present invention comprise:
a TLR2-targeting synthetic PGN moiety (Figures 7 and 8, Box) that supplies access to APC cellular machinery for processing and presentation and provides the glycopeptide backbone onto which one or more than one target epitope is/are covalently attached; and
one or more than one target epitope, in one or more copies each within the SPA molecule. The target epitope(s) may be a peptide sequence or carbohydrate moiety,
or a pharmaceutically acceptable salt thereof.

Specific examples of suppressive SPAs are shown diagrammatically in Figures 7 and 8, but are not meant to be limiting in any manner.

Similar to the non-specific SPA, the SPA is a linear, non-crosslinked polymeric compound of Formula VII:

X¹-[-MO-]_{w}-X ² (VII)

wherein
X¹ and X² are independently H or a terminator;
n represents the number of monomeric units (MO) in the polymer, and may be an integer in the range of from about 10 to about 375, or any amount therebetween; for example, n may be about 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, 180, 185, 190, 195, 200, 205, 210, 215, 220, 225, 230, 235, 240, 245, 250, 255, 260, 265, 270, 275, 300, 305, 310, 315, 320, 325, 330, 335, 340, 345, 350, 355, 360, 365, 370 or 375, or any amount therebetween, or any amount in a range defined by any two amounts defined herein. In a further, non-limiting example, W may be described as a centre of distribution lying between about 130 and about 180, or any amount therebetween;
the monomeric units MO comprise:
unsubstituted repeat units (UR; see, for example Figures 7 and 8); and
one or more more than one species of target epitope repeat units (TR; see, for example Figures 7 and 8),
or a pharmaceutically acceptable salt thereof.

The suppressive SPAs may comprise from about 1 to about 180 different target epitopes in the TR species of the SPA molecule, or any amount therebetween. Each epitope is designated "(target epitope)ₙ" (see Figures 3 to 6). For example, there may be about 1, 2, 3, 4, 5, 10, 15, 20, 25, 30, 35, 40, 45, 50, 55, 60, 65, 70, 75, 80, 85, 90, 95, 100, 105, 110, 115, 120, 125, 130, 135, 140, 145, 150, 155, 160, 165, 170, 175, or 180 different target epitopes in the TR species of the SPA molecule, or any amount therebetween, or any amount in a range defined by any two amounts disclosed herein. Various types oftarget epitopes are contemplated by the present invention. Non-limiting examples of suitable epitopes are described later in the present description. A person of skill in the art will recognize that the number of target epitopes will determine the number of TR species. For example, and without wishing to be limiting in any manner, if 3 different target epitopes are used, the epitopes would be designated (target epitope)₁, (target epitope)₂, and (target epitope)₃, with each epitope present on its respective species of TR, i.e. 3 different TR species designated TR¹ (carrying (target epitope)₁), TR² (carrying (target epitope)₂), and TR³ (carrying (target epitope)₃).

When a single species of target epitope is present, as shown in Figure 7, the SPA is a *mono*SPA. When more than one species of target epitope is present, as shown in Figure 8, the SPA is *a poly*SPA.

Each species of monomeric unit is present in the SPAs in a given mole fraction designated as a subscript (x or zₙ). For example, a mole fraction of 0.6 indicates that the given monomeric unit exists as 60% of the repeat units in the SPA. The designation of the mole fraction ofunsubstituted repeat units (UR) is x; for example, if x = 0.4, the UR exists as 40% of the monomeric units in the SPA. The designation of the mole fraction of target epitope repeat units (TR) species is zₙ; for example, if zₙ = 0.20, the n^{th} different species of TR exists as 20% of the monomeric units in the SPA.

A person of skill in the art will recognize that the sum of the mole fractions must be equal to 1.00, i.e., the sum of x + y + y₁ + y₂ + ... + yₙ + z + z₁ + z₂ + . .. + zₙ, (as the case may be) = 1.00. Since the rate of enzymatic polymerization of the various repeat units varies little, if at all, with substitution, UR, ThR, TR and/or Th/TR are evenly distributed along the carbohydrate axis of the polymer according to their respective mole fractions in the composition. Note that UR, ThR, TR and/or Th/TR can exist in any order within the polysaccharide as a consequence of the random nature of formation of the co-polymer.

To illustrate the relationship of the values described above, the following non-limiting example is set forth: a SPA polymer comprising a total of 50 monomeric units (i.e. W= 50). The SPA polymer has 4 target epitopes ((target epitope)₁, (target epitope)₂, (target epitope)₃, and (target epitope)₄); thus the polymer comprises 1 species of UR and 4 species of TR. If the the mole fraction of UR (i.e. x) is 0.40; the mole fraction of TR carrying (target epitope)₁ (i.e. z₁) is 0.06; the mole fraction ofTR carrying (target epitope)₂ (i.e. z₂) is 0.20; the mole fraction of TR carrying (target epitope)₃ (i.e. z₃) is 0.24; and the mole fraction of TR carrying (target epitope)₄ (i.e. z₁) is 0.10; the polymer will comprise 40% UR, 6% TR¹, 20% TR², 24% TR³, and 10% TR⁴ (i.e. 20 UR monomers, 3 TR¹ monomers, 10 TR² monomers, 12 ThR³ monomers, and 5 TR⁴ monomers).

The antigen-specific suppressive SPAs of the present invention are copolymers (i.e., two or more different monomers). The rate of enzymatic polymerization of the various monomeric units (UR and TR) varies little, and thus the monomers may be evenly distributed along the length of the SPA copolymer, according to their respective mole fractions in the composition. A person of skill in the art would readily recognize that, while Figures 7 and 8 depict the monomers in a specific order within the SPA, the monomers may exist in any order within the copolymer as a result of the random nature of polymerization. Thus, the copolymers may be random copolymers, block copolymers or alternating copolymers. For example, and without wishing to be limiting, for a SPA comprising UR and one species of TR (TR¹), the polymer types may include:

| **Polymer Type** | **Example** |
|---|---|
| Random copolymer* | X¹-UR-TR¹-TR¹-UR-UR-TR¹-TR¹-UR-TR¹-X² |
| Block copolymer** | X¹-UR-UR- TR¹-TR¹-UR-UR-TR¹-TR¹-X² |
| Alternating copolymer* | X¹-UR-TR¹-UR-TR¹-UR-TR¹-UR-TR¹-X² |

| | |
|---|---|
| * the length of this copolymer may vary from that as shown; * * wherein each of the 'blocks' may be of varied length, and may be repeated throughout the copolymer; the length of this copolymer may also vary from that as shown | |

The suppressive *mono*SPAs of the present invention are random linear co-polymers comprised of distinct types of β-[1,4]-linked *N*-acetylglucosaminyl-β-[1,4]-*N-*acetylmuramyl peptide repeat units. Conservative substitution is contemplated in the carbohydrate core. Thus, the lactyl methyl group may also be lower alkyl (C₁-C₅) or hydrogen, and the D-configuration at the oxygen-bearing carbon is preferred when any alkyl group is present.

In general, the monomeric units (MO) can be described by the following structures:

UR (Formula VII):

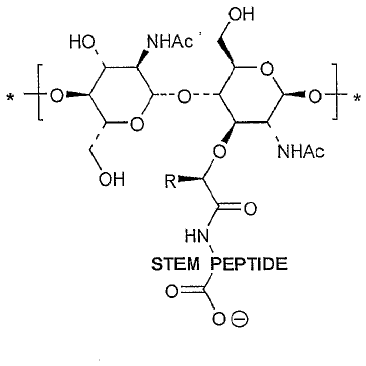

TR (Formula X):

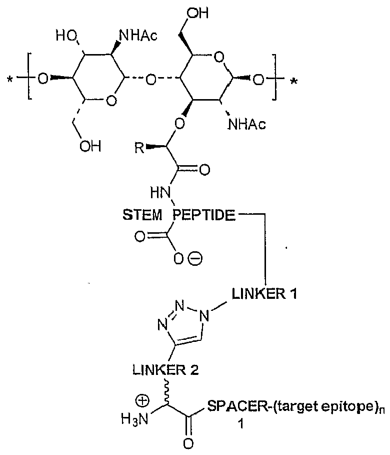

The R group of the monomeric units may be independently chosen, and may be either H or a lower alkyl (C₁-C₅).

The stem peptide of the unsubstitued stem peptide repeat units (UR; see, for example Figures 7 and 8) and the stem peptide of the target epitope repeat units (TR; see, for example Figures 7 and 8) are independantly selected, and may comprise from about one to about five amino acids. The stem peptide may comprise any amino acid, natural or unnatural. For example, and without wishing to be limiting in any manner, the following amino acids may be used. Position 1 may be occupied by alanine, a lower alkyl (C₁-C₅) homologue of alanine, or glycine; in a further non-limiting example, the L-configuration is preferred at the α-carbon for alanine or its homologues. Position 2 may be occupied by γ-aminobutyric acid (Gaba), glycine, β-aminopropionic acid, δ-aminopentanoic acid and ε-amino hexanoic acid; in a further non-limiting example, Gaba is at position 2. Position three may be occupied by any α-amino acid, natural or unnatural; in a further non-limiting example, lysine or diaminopimelic acid is at position 3. Position 4 may be occupied by any α-amino acid, natural or unnatural; in a further non-limiting example, position 4 is occupied by D-alanine. Position 5 may be occupied by any α-amino acid, natural or unnatural; in a further non-limiting example, D-alanine is at position 5. The amino acid residues may be independently joined at the α or γ carboxyl groups, and at the α or ε amino groups, or any combination thereof, provided that no pendant carboxylate or carboxamide group is present in the stem peptide. In addition, each amino acid residue of the stem peptide may be unsubstituted or substituted with one or more groups selected from halo, alkyl, hydroxy, alkoxy, phenoxy, CF₃, amino, alkylamino, dialkylamino, -C(O)Oalkyl and -NO₂.

LINKER1 and LINGER2 may be independently chosen, and may comprise any suitable linker known in the art. In a particular example, each linker may comprise from about 1 to about 6 segments, or any amount therebetween; for example, the linker may comprise 1,2,3,4,5, or 6 segments. Without wishing to be limiting, each segment may be chosen from -CH₂-, -CHR-, =CH-, and ≡CH-, where R is a lower alkyl. In the case where there are 3 to 6 segments, segments 1 to 4, when present, may also be chosen from -O-, NH-, NR-, -S-, -SO-, and -SO₂-, provided that there are no contiguous heteroatom segments.

The connection between the stem peptide and LINGER 1 (see Figures 3 to 6) may each independently be made at any one of the amino acids of the stem peptide. In a non-limiting example, the connection between the stem peptide and LINGER 1 is made at position three of the stem peptide. The connector between LINKER 1 and LINKER 2 may be 1,4-[1,2,3-triazole] (Rostovtsev et al. (2002) Angew. Chem. Int. Ed. 114:2708) or any other connection chemistry known to those skilled in the art, for example, but not limited to thiolate/maleimide (Verez-Bencomo et al. (2004) Science 305:522) and amine/aldehyde reductive alklyation (Slovin et al. (1999) PNAS 96:5710). In a specific, non-limiting example, the target epitope is a carbohydrate, and the connector between LINKER1 and LINKER2 is amine/aldehyde reductive alkylation.

The SPACER for the target epitope may be from about one to about 10 amino acids in length, or any amount therebetween; for example, the spacer may be about 1, 2, 3, 4, 5, 6, 7, 8, 9, or 10 amino acids in length. The amino acids may be any natural or unnatural amino acid known in the art. In a specific, non-limiting example, the spacer may be Gly-Ser-Gly-Ser (see Figures 7 and 8), however, other amino acids within the spacer may be used if desired, and the spacer may be of a different length that as just described, for example from about 2 to about 10 amino acids, or any amount therebetween, for example from about 4 to about 8 amino acids, or any amount therebetween. In a specific, non-limiting example, the spacer is 4 amino acids in length. The spacer may be connected to the monomeric unit at its N-terminus (i.e., by its α-amino group) or by an ε amino group of a side chain of any one of the amino acids thereof, if present; for example, but not wishing to be limiting, the spacer is connected to the monomeric unit at amino acid at the α-amino group of position 1 of the spacer. The spacer is connected to the target epitope through either a peptide bond (if the eptope is a peptide) or through O-linked glycosylation (if the epitope is a carbohydrate).

Generic Th Epitopes

The T-helper (Th) epitope may be any suitable T-helper epitope known to the skilled artisan for enhancing an immune response in a particular target subject (i.e., a human subject, or a specific non-human animal subject such as, for example, a rat, mouse, guinea pig, dog, horse, pig, or goat). The Th epitopes are present in the pro-inflammatory *mono-* and *poly*SPAs of the present invention. Preferred T-helper epitopes comprise at least about 10-24 amino acids in length, or any amoun therebetween; for example, the Th epitope may comprise about 10, 11, 12, 13, 14, 15, 16, 17, 18, 19, 20, 21, 22, 23, or 24 amino acids. In a non-limiting example, the Th epitope may be about 15 to about 20 amino acids in length. Generic (promiscuous or permissive) T-helper epitopes may be used, as these are readily synthesized chemically and obviate the need to use proteins or longer polypeptides comprising multiple T-helper epitopes.

Non-limiting examples of promiscuous or permissive T-helper epitopes suitable for use in the SPAs of the present invention may be selected from the group consisting of:
i. a rodent or human T-helper epitope of tetanus toxoid peptide (TTP), such as, for example amino acids 830-843 of TTP (Panina-Bordignon et al.(1989) Eur. J. Immun. 19:2237);
ii. a rodent or human T-helper epitope of Plasmodium falciparum pfg27;
iii. a rodent or human T-helper epitope of lactate dehydrogenase;
iv. a rodent or human T-helper epitope of the envelope protein of HIV or HIVgp120 (Berzofsky et al. (1991) J. Clin. Invest. 88:876);
v. a synthetic human T-helper epitope (PADRE) predicted from the amino acid sequence of known anchor proteins (Alexander et al. (1994) Immunity 1:751);
vi. a rodent or human T-helper epitope of measles virus fusion protein MV 5 F (Muller et al. (1995) Mol. Immunol. 32:37; Partidos et al. (1990) J. Gen. Virol. 71:2099);
vii. a T-helper epitope comprising at least about 10 amino acid residues of canine distemper virus fusion protein (CDV-F) such as, for example, from amino acid positions 148-283 of CDV-F (Ghosh et al. (2001) Immunol.104:58 and WO 2000/46390);
viii. a human T-helper epitope derived from the peptide sequence of extracellular tandem repeat domain of MUC1 mucin (WO 20018806);
ix. a rodent or human T- helper epitope of influenza virus haemagglutinin Is (IV-H) (Jackson et al. (1994) Virol. 198:613); and
x. a bovine or camel T-helper epitope of the VP3 protein of foot and mouth disease virus (FMDV-O Kaufbeuren strain) comprising residues 173 to 176 of VP3 or the corresponding amino acids of another strain of FMDV.

As will be known to those skilled in the art, a T-helper epitope may be recognized by one or more mammals of different species. Accordingly, the designation of any T-helper epitope herein is not to be considered restrictive with respect to the immune system of the species in which the epitope is recognised. For example, a rodent T-helper epitope can be recognised by the immune system of a mouse, rat, rabbit, guinea pig, or other rodent, or a human or dog.

The T-helper epitope may comprise, for example, but not wishing to be limiting, an amino acid sequence (WO 2004/014956, WO 2004/014957) selected from the group consisting of:
i. GALNNRFQIKGVELKS from IV-H;
ii. ALNNRFQIKGVELKS from IV-H;
iii. LSEIKGVIVHRLEGV from MV-F;
iv. TMQITAGIALHQSNLN from CDV-F;
v. IGTDNVHYKIMTRPSHQ from CDV-F;
vi. YKIMTRPSHQYLVIKLI from CDV-F;
vii. SHQYLVIKLIPNASLIE from CDV-F;
viii. KLIPNASLIENCTKAEL from CDV-F;
ix. LIENCTKAELGEYEKLL from CDV-F;
x. AELGEYEKLLNSVLEPI from CDV-F;
xi. KLLNSVLEPINQALTLM from CDV-F;
xii. EPINQALTLMTKNVKPL from CDV-F;
xiii. TLMTKNVKPLQSLGSGR from CDV-F;
xiv. KPLQSLGSGRRQRRFAG from CDV-F;
xv. SGRRQRRFAGWLAGVA from CDV-F;
xvi. FAGWLAGVALGVATAA from CDV-F;
xvii. GVALGVATMQITAGIA from CDV-F;
xviii. GIALHQSNLNAQAIQSL from CDV-F;
xix. NLNAQAIQSLRTSLEQS from CDV-F;
xx. QSLRTSLEQSNKAIEEI from CDV-F;
xxi. EQSNKAIEEIREATQET from CDV-F;
xxii. SSKTQTHTQQDRPPQPS from CDV-F;
xxiii. QPSTELEETRTSRARHS from CDV-F;
xxiv. RHSTTSAQRSTHYDPRT from CDV-F;
xxv. PRTSDRPVSYTMNRTRS from CDV-F;
xxvi. TRSRKQTSHRLKNIPVH from CDV-F;
xxvii. TELLSIFGPSLRDPISA from CDV-F;
xxviii. PRYIATNGYLISNFDES from CDV-F;
xxix. CIRGDTSSCARTLVSGT from CDV-F;
xxx. DESSCVFVSESAICSQN from CDV-F;
xxxi. TSTIINQSPDKLLTFIA from CDV-F;
xxxii. SPDKLLTFIASDTCPLV from CDV-F;
xxxiii. STAPPAHGVTSAPDTRAPGSTAPP from MUC-1;
xxxiv. GVTSAPDTRPAPGSTASSL from MUC-1;
xxxv. GVTSAPDTRPAPGSTASL from MUC-1;
xxxvi. TAPPAHGVTSAPDTRPAPGSTAPPKKG from MUC-1;
xxxvii. STAPPAHGVTSAPDTRPAPGSTAPPK from MUC-1;
xxxviii. GVAE from FMDV-VP3 protein;
xxxix. TASGVAEIIN from FMDV-VP3 protein (residues 170 to 179); and
xl. TAKSKKFPSYTATYQF from FMDV.

The T-helper epitopes disclosed herein are included for the purposes of exemplification only. Using standard peptide synthesis techniques known to the skilled artisan, the T-helper epitopes referred to herein mat be readily substituted for a different T-helper epitope to adapt the SPA of the invention for use in a different species. Accordingly, additional T-helper epitopes known to the skilled person to be useful in eliciting or enhancing an immune response in any species species of interest are not to be excluded.

Additional T-helper epitopes may be identified by a detailed analysis, using *in vitro* T-cell stimulation techniques of component proteins, protein fragments and peptides to identify appropriate sequences (Goodman and Sercarz (1983) Ann. Rev. Immunol. 1:465); (Berzofsky (1986): "The Year in Immunology, Vol. 2, page 151, Karger, Basel) and (Livingstone and Fathman (1987) Ann. Rev. Immunol.5:477).

Cytotoxic T Lymphocyte (CTL) Target Epitopes

The CTL epitope may conveniently be derived from the amino acid sequence of an immunogenic protein, lipoprotein, or glycoprotein of a virus, prokaryotic or eukaryotic organism, including but not limited to a CTL epitope derived from a mammalian subject or a bacterium, fungus, protozoan, or parasite that infects said subject. Mimotopes of the CTL epitopes are specifically included within the scope of the invention.

The CTL epitope will be capable of eliciting a T cell response when administered to a mammal, preferably by activating CD8+ T cells specific for the epitope or antigen from which the epitope was derived, and more preferably, by inducing cell mediated immunity against the pathogen or tumour cell from which the epitope is derived. Shorter CTL epitopes are preferred, to facilitate peptide synthesis. The length of the CTL epitope should not exceed about 30 amino acids in length; for example, the CTL epitope may be 30, 29, 28, 27, 26, 25, 24, 23, 22, 21, 20, 19, 18, 17, 16, 15, 14, 13, 12, 11, 10, 9, 8, 7, 6, or 5 amino acids in length. In a non-limiting example, the CTL epitope may less than about 25, or less than about 20 amino acid residues. In another example, the CTL epitope is 8-12 amino acid residues in length.

CTL epitopes may be obtained from parasites, for example but not limited to those associated with leishmania, malaria, trypanosomiasis, babesiosis, or schistosomiasis. For example, a CTL epitope may be from an antigen of a parasite selected from the group consisting of: *Plasmodium falciparum; Circumsporozoa; Leishmania donovani; Toxoplasma gondii; Schistosoma mansoni; Schistosoma japonicum; Schisfosoma hematoblum;* and *Trypanosoma brucei.*

Particular examples of CTL epitopes of *P. falciparum* may be those derived from an antigen selected from the group consisting of: circumsporozoite protein (CSP), sporozoite surface protein 2 (PfSSP2), liver stage antigen 1 (LSA1), merozoite surface protein 1 (MSP 1), serine repeat antigen (SERA) and AMA-1 antigen (Amante et al. (1997) J. Immunol. 159:5535; Chaba et al. (1998) J. Immunopharm. 20:259; Shi et al. (1999) PNAS 96:1615; Wang et al. (1998) Science 282:476; and Zevering et al. (1998) Immunol. 94:445). Particular examples of CTL epitopes of *L. donovani* may be those derived from the Repetitive Peptide (Liew et al. (1990) J. Exp. Med. 172:1359). Particular examples of CTL epitopes of *T. gondii* may be those derived from the P30 surface protein (Darcy et al. (1992) J. Immunol. 149:3636). Particular examples of CTL epitopes of *S*. *mansoni* may be those derived from the Sm-28GST antigen (Wolowxzuk et al. (1991) J. Immunol. 146:1987).

CTL epitopes may be, for example, but not limited to virus-specific derived from Rotaviruses, Herpes viruses, Corona viruses, Picornaviruses (e.g., Apthovirus), Respiratory Synctial virus, Influenza Virus, Parainfluenza virus, Adenovirus, Pox viruses, Bovine herpes virus Type I, Bovine viral diarrhea virus, Bovine rotaviruses, Canine Distemper Virus (CDV), Foot and Mouth Disease Virus (FMDV), Measles Virus (MV), Human Immunodeficiency Viruses (HIV), Feline Immunodeficiency Viruses (FIV), Epstein-Barr virus (EBV), Human Cytomegalovirus (HCMV), hepatitis viruses, Hepatitis B virus, Hepatitis C virus, Herpes Simplex - 1 virus, Herpes simplex - 2 virus, Hepatitis B virus, Human Herpes Virus 6, Infectious Bursal Disease Virus, Mumps virus, Human papilloma virus type 16, Human papilloma virus type 18, Influenza A virus, Influenza B virus, Influenza C virus, Porcine Reproductive and Respiratory Syndrome Virus, Rabies Virus, Rhinovirus, Smallpox (Variola) Virus, Vaccinia Virus, Zoster virus (chicken pox), and the like.

Particular examples of CTL epitopes of HIV-1 may be those derived from the env, gag, or pol proteins.

Particular examples of CTL epitopes of influenza virus may be those derived from the nucleoprotein (Taylor et al. (1989) Immunogenetics 26:267 (1989); Townsend et al. (1983) Nature 348:674), matrix protein (Bednarek et al. (1991) J. Immunol. 147:4047) or polymerase protein (Jameson et al. (1998) J. Virol. 72:8682; and Gianfrani et al. (2000) Human Immunol. 61:438).

Particular examples of CTL epitopes of Lymphocytic choriomeningitis virus (LCMV) may be those derived from glycoprotein-1 antigen (Zinkernagel et al. (1974) Nature 248:701).

Particular examples of CTL epitopes of cytomegalovirus may be those derived from an antigen selected from the group consisting of: of pp28, pp50, pp65, pp71, pp150, gB, gH, IE-1, IE 2, US2, US3, US6, US11, and UL18 (Longmate et al. (2000) Immunogenet. 52:165; Wills etal. (1996) J. Virol. 70:7569; Solache et al. (1999) J. Immunol. 163, 5512; Diamond et al. (1997) Blood 90:1751; Kern et al. (1998) Nature Med. 4:975; Weekes et al. (1999) J. Virol. 73, 2099; Retiere et al. (2000) J. Virol. 74:3948; and Salquin et al. (2000) Eur. J. Immunol. 30:2531).

Particular examples of CTL epitopes of Measles Virus may be those derived from the fusion glycoprotein (MV-F), particularly from residues 438-446 thereof (Herberts et al. (2001) J. Gen Virol. 82:2131).

Particular examples of CTL epitopes ofEpstein-Barr virus (EBV) may be those derived from a latent nuclear antigen (EBNA) or to latent membrane protein (LMP) of EBV, such as, for example, EBNA 2A, EBNA 3A, EBNA 4A, or EBNA 14a from EBV type A; EBNA 2B, EBNA 3B, EBNA 4B, or EBNA 14b from EBV type B; LMP1; or LMP2 (PCT/AU95/00140; PCT/AU97/00328; and PCT/AU98/00531).

CTL epitopes may be, for example, but not limited to bacteria-specific CTL epitopes derived from *Pasteurella, Actinobacillus, Haemophilus, Listeria monocytogenes, Mycobacterium tuberculosis, Staphylococcus, Neisseria gonorrhoeae, Helicobacter pylori, Streptococcus pneumoniae, Salmonella enterica, Escherichia coli, Shigella,* and the like. Suitable bacterial CTL epitopes include, but are not limited to, those CTL epitopes derived from the *Mycobacterium tuberculosis* 65Kd protein (Lamb et al. (1987) EMBO J. 6:1245); *M. tuberculosis* ESAT-6 protein (Morten et al. (1998) Infect. Immun. 66:717); *Staphylococcus aureus* nuclease protein (Finnegan et al. (1986) J. Exp. Med. 164:897); *Escherichia coli* heat stable enterotoxin (Cardenas et al. (1993) Infect. Immunity 61:4629); and *Escherichia coli* heat labile enterotoxin (Clements et al. (1986) Infect. Immunity 53:685).

CTL epitopes may be, for example, but not limited to CTL epitopes from mammalian subjects derived from and/or capable of generating T cell responses against a tumor CTL antigen. Tumor-specific CTL epitopes are usually native or foreign CTL epitopes, the expression of which is correlated with the development, growth, presence or recurrence of a tumor. In as much as such CTL epitopes are useful in differentiating abnormal from normal tissue, they are useful as targets for therapeutic intervention. Such CTL epitopes are well known in the art. Non-limiting examples of tumor CTL epitopes may be those derived from carcinoembryonic antigen (CEA), prostate specific antigen (PSA), melanoma antigen (PAGE, SAGE, GAGE), and mucins, such as MUC-1. Particular examples of CTL epitopes for administration to a cancer patient may be those derived from a protein that induces cancer, such as, for example, an oncoprotein (e.g., p53, ras, etc.).

In a non-limiting example, the CTL epitope may comprise an amino acid sequence selected from the group consisting of:
i. TYQRTRALV from the NP of PRO virus;
ii. KPKDELDYENDIEKKICKMEKCS of *P*. *falciparum* CSP;
iii. DIEKKICKMEKCSSVFNWNS from *P.falciparurn* COP;
iv. KPIVQYDNF from P. falciparum LSAT;
v. GISWEKVLAKYKDDLE from *P. falciparum* MSP 1;
vi. EFTYMINFGRGQNYWEHPYQKS of *P. falciparum* AMA-1;
vii. DQPKQYEQHLTDYEKIKEG from *P. falciparum* AMA-1;
viii. NMWQEVGKAM from HIV-1 env protein;
ix. APTKAKRRW from HIV-1 env protein;
x. CTRPNNNTRKfrom HIV-1 env protein;
xi. TVYYGVPVWK from HIV-1 env protein;
xii. RPWSTQLL from HIV-1 env protein;
xiii. SLYNTVATLY from HIV-1 gag protein;
xiv. ELRSLYNTVA from HIV-1 gag protein;
xv. KIRLRPGGKK from HIV-1 gag protein;
xvi. IRLRPGGKKK from HIV-1 gag protein;
xvii. RLRPGGKKK from HIV-1 gag protein;
xviii. GPGHKARVLA from HIV-1 gag protein;
xix. SPIETVPVKL from HIV-1 pol protein;
xx. ILKEPVHGVY from HIV-1 pol protein;
xxi. AIFQSSMTK from HIV-1 pol protein;
xxii. SPAIFQSSMT from HIV-1 pol protein;
xxiii. QVRDQAEHLK from HIV-1 pol protein;
xxiv. GPKVKQWPLT from HIV-1 pol protein;
xxv. TYQRTRALV from influenza virus nucleoprotein;
xxvi. TYQRTRALVRTGMDP from influenza nucleoprotein;
xxvii. IASNENMDAMESSTL from influenza virus nucleoprotein;
xxviii. KAWNFATM from LCMV gp1;
xxix. QVKWRMTTL from EBV;
xxx. VFSDGRVAC from EBV;
xxxi. VPAPAGPIV from EBV;
xxxii. TYSAGIVQI from EBV;
xxxiii. LLDFVRFMGV from EBV ;
xxxiv. QNGALAINTF from EBV;
xxxv. VSSDGRVAC from EBV;
xxxvi. VSSEGRVAC from EBV;
xxxvii. VSSDGRVPC from EBV;
xxxviii. VSSDGLVAC from EBV;
xxxix. VSSDGQVAC from EBV;
xl. VSSDGRWC from EBV;
xli. VPAPPVGPIV from EBV;
xlii. VEITPYEPTG from EBV;
xliii. VEITPYEPTW from EBV;
xliv. VELTPYKPTW from EBV;
xlv. RRIYDLIKL, from EBV;
xlvi. RKIYDLIEL from EBV;
xlvii. PYLFWLAGI from EBV;
xlviii. TSLYNLRRGTALA from EBV;
xlix. DTPLIPLTIF from EBV;
l. TVFYNIPPMPL from EBV;
li. VEITPYKPTW from EBV;
lii. VSFIEFVGW from EBV;
liii. FRKAQIQGL from EBV;
liv. FLRGRAYGL from EBV;
lv. QAKWRLQTL from EBV;
lvi. SVRDRLARL from EBV;
lvii. YPLHEQHGM from EBV
lviii. HLMQGMAY from EBV;
lix. RPPIFIRRL from EBV;
lx. RLRAEAGVK from EBV;
lxi. IVTDFSVIK from EBV;
lxii. AVFDRKSDAK from EBV;
lxiii. NPTQAPVIQLVHAVY from EBV;
lxiv. LPGPQVTAVLLHEES from EBV;
lxv. DEPASTEPVHDQLL from EBV;
lxvi. RYSIFFDY from EBV;
lxvii. AVLLHEESM from EBV;
lxviii. RRARSLSAERY from EBV;
lxix. EENLLDFVRF from EBV;
lxx. KEHVIQNAF from EBV;
lxxi. RRIYDLIEL from.EBV;
lxxii. QPRAPIRPI from EBV;
lxxiii. EGGVGWRHW from EBV;
lxxiv. CLGGLLTMV from EBV;
lxxv. RRRWRRLTV from EBV;
lxxvi. RAKFKQLL from EBV;
lxxvii. RKCCRAKFKQLLQHYR from EBV;
lxxviii. YLLEMLWRL from EBV;
lxxix. YFLEILWGLfrom EBV;
lxxx. YLLEILWRL from EBV;
lxxxi. YLQQNWWTL from EBV;
lxxxii. LLLALLFWL from EBV;
lxxxiii. LLVDLLWLL from EBV;
lxxxiv. LLLIALWNL from EBV;
lxxxv. WLLLFLAIL from EBV;
lxxxvi. TLLVDLLWL from EBV;
lxxxvii. LLWLLLFLA from EBV;
lxxxviii. ILLIIALYL from EBV;
lxxxix. VLFIFGCLL from EBV;
xc. RLGATIWQL from EBV;
xci. ILYFIAFAL from EBV;
xcii. SLVIVIIFV from EBV;
xciii. LMIIPLINV from EBV;
xciv. ILFIGSHW from EBV;
xcv. LIPETVPYI from EBV;
xcvi. VLQWASLAV from EBV;
xcvii. QLTPHTKAV from EBV;
xcviii. SVLGPISGHVLK from HCMV pp65;
xcix. FTSQYRIQGKL from HCMV pp65;
c. FVFPTKDVALR from HCMV pp65;
ci. FPTKDVAL from HCMV pp65;
cii. NLVPMVAIV from HCMV pp65;
ciii. MLNIPSINV from HCMV pp65;
civ. RIFAELEGV from HCMV pp65;
cv. TPRVTGGGGAM from HCMV pp65;
cvi. RPHERNGFTVL from HCMV pp65;
cvii. RLLQTGIHV from HCMV pp65;
cviii. VIGDQYVKV from HCMV pp65;
cix. ALFFFDIDL from HCMV pp65;
cx. YSEHPTFTSQY from HCMV pp65;
cxi. VLCPKNMII from HCMV pp65;
cxii. DIYRIFAEL from HCMV pp65;
cxiii. ILARNLVPMV from HCMV pp65;
cxiv. EFFWDANDIY from HCMV pp65;
cxv. IPSINVHHY from HCMV pp65;
cxvi. YILEETSVM from HCMV IE-1;
cxvii. CVETMCNEY from HCMV IE- 1;
cxviii. RRIEEICMKfrom HCMV IE-1;
cxix. TTWPPSSTAK from HCMV pp150;
cxx. RRYPDAWL from Measles Virus Fusion glycoprotein;
cxxi. GYKDGNEYI from *Listeria monocytogenes;*
cxxii. SIINFEKL from ovalbumin; and
cxxiii. DLMGYIPLV from the core protein of hepatitis C virus.
cxxiv. (MAGE-A1) [96-104] melanoma;
cxxv. (MAGE-A10) [254-262] melanoma;
cxxvi. gp100 [614-622] melanoma; and
cxxvii. six HLA cross-reactive tumor associated CTL epitopes from (Kawashima et al. (1998) Hum. Immunol. 59:1).

It is to be understood that the compositions and methods of the present invention are amenable for use with these and other known peptides and carbohydrates that have been implicated as CTL epitopes involved in disease states of interest. Clearly, the present invention is intended to encompass any other such peptide or carbohydrate that may in future be disclosed that may be used as the CTL target epitope according to the principles of the present invention.

B Cell Target Epitopes

The B cell epitope may conveniently be derived from the amino acid sequence of an immunogenic protein, lipoprotein, or glycoprotein of a virus, prokaryotic or eukaryotic organism, including but not limited to an antigen derived from a mammalian subject or a bacterium, fungus, protozoan, or parasite that infects said subject. Idiotypic and anti-idiotypic B cell epitopes against which an immune response is desired are specifically included, as are lipid-modified B cell epitopes. Alternatively, the B cell epitope may be a carbohydrate antigen, such as, for example, an ABH blood group antigen, transplantation antigen (eg. Gal-α-[1,3]-Gal-β-[1,4]-GlcNAc (Sandrin et al. (1993) PNAS 90:11391; (Galili et al. (1987) PNAS 84:1369; Schofield et al. (2002) Nature 418:785), or a conjugate thereof.

The B-cell epitope should be capable of eliciting the production of antibodies when administered to a mammal; for example, neutralizing antibody may be produced; in a further example, a high titer neutralizing antibody may be produced.

Shorter B cell epitopes may be used, to facilitate peptide synthesis. For example, the length of the B cell epitope should not exceed about 30 amino acids in length; for example, the B cell epitope may be 30, 29, 28, 27, 26, 25, 24, 23, 22, 21, 20, 19, 18, 17, 16, 15, 14, 13, 12, 11, 10, 9, 8, 7, 6, or 5 amino acids in length. In a non-limiting example, the B cell epitope may less than about 25, or less than about 20 amino acid residues. In another example, the B cell epitope is 5-20 amino acid residues in length.

The peptides may assume a conformation that mimics the conformation of the native polypeptide from which the B cell epitope is derived.

B cell epitopes may be, for example, but not limited to from parasites and may be those associated with leishmania, malaria, trypanosomiasis, babesiosis, or schistosomiasis. Without wishing to be limiting in any manner, the B cell epitope may be selected from the group consisting of:
i. a B cell epitope of *Plasmodiumfalciparum* (NANP) 3 (Good et al. (1986) J. Exp. Med. 164:655);
ii. a B cell epitope of *Circumsporozoa* (Good et al. (1987) Protein Sci. 235:1059);
iii. a B cell epitope comprising amino acid residues 326-343 of *Leishmania donovani* Repetitive Peptide (Liew et al. (1990) J. Exp. Med. 172:1359);
iv. a B cell epitope of *Toxoplasma gondii* P30 surface protein (Darcy et al. (1992) J. Immunol. 149:3636); and
v. a B cell epitope of *schistosoma mansoni* Sm-28GST antigen (Wolowxzuk et al. (1991) J. Immunol. 146:1987).

B cell epitopes may be, for example, but not limited to derived from and/or capable of generating antibodies against Rotaviruses, Herpes viruses, Corona viruses, Picornaviruses (e.g., Apthovirus), Respiratory Synctial virus, Influenza Virus, Parainfluenza virus, Adenovirus, Pox viruses, Bovine herpes virus Type I, Bovine viral diarrhea virus, Bovine rotaviruses, Canine Distemper Virus (CDV), Foot and Mouth Disease Virus (FMDV), Measles Virus (MV), Human Immunodeficiency Viruses (HIV), Feline Immunodeficiency Viruses (FIV), Epstein-Barr virus (EBV), Human Cytomegalovirus (HCMV), hepatitis viruses, Hepatitis B virus, Hepatitis C virus, Herpes Simplex - 1 virus, Herpes simplex - 2 virus, Hepatitis B virus, Human Herpes Virus 6, Infectious Bursal Disease Virus, Mumps virus, Human papilloma virus type 16, Human papilloma virus type 18, Influenza A virus, Influenza B virus, Influenza C virus, Porcine Reproductive and Respiratory Syndrome Virus, Rabies Virus, Rhinovirus, Smallpox (Variola) Virus, Vaccinia Virus, Zoster virus (chicken pox), and the like.

Suitable viral B cell epitopes include, but are not limited to epitopes selected from the group consisting of:
i. HIV gp120 V3 loop, amino acid residues 308-331 (Jatsushita et al. (1988) J. Virol. 62:2107);
ii. HIV gp120 amino acid residues 428-443 (Ratner et al. (1985) Nature 313:277);
iii. HIV gp120 amino acid residues 112-124 (Berzofsky et al. (1988) Nature 334:706);
iv. a B cell epitope of HIV Reverse transcriptase (Hosmalin et al. (1990) PNAS 87:2344);
v. Influenza virus nucleoprotein amino acid residues 335-349 (Townsend et al. Cell 44, 959 (1986));
vi. Influenza virus nucleoprotein amino acid residues 366-379 (Townsend et al. (1986) Cell 44:959);
vii. Influenza virus hemagglutinin amino acid residues 48-66 (Mills et al. (1986) J. Exp. Med. 163:1477);
viii. Influenza virus hemagglutinin amino acid residues 111-120 (Hackett et al. (1983) J. Exp. Med. 158:294);
ix. Influenza virus hemagglutinin amino acids 114- 131 (Lamb and Green (1983) Immunology 50:659);
x. Epstein-Barr LOP amino acid residues 43-53 (Thorley-Lawson et al. (1987) PNAS 84:5384);
xi. Hepatitis B virus surface antigen amino acid residues 95-109 (Milich et al. (1985) J. Immunol. 134:4203);
xii. Hepatitis B virus surface antigen amino acid residues 140-154;
xiii. Hepatitis B virus Pre-S antigen amino acid residues 120-132 (Milich et al. (1986) J. Exp. Med. 164:532);
xiv. Herpes simplex virus gD protein amino acid residues 5-23 (Jayaraman et al. (1993) J. Immunol. 151:5777);
xv. Herpes simplex virus gD protein amino acid residues 241-260 (Wyckoff et al. (1988) Immunobiol. 177:134);
xvi. Rabies glycoprotein amino acid residues/ 32-44 (MacFarlan et al. (1984) J. Immuno/. 133:2748);
xvii. The major FMDV epitope comprising at least amino acid residues 134- 168 or 137-160 or residues 142-160 or residues 137-162 or residues 145- 150 of the VP1 capsid protein of FMDV serotype O, or the corresponding amino acid residues of another serotype, such as, for example, serotypes A, C, SAT1, SAT2, SAT3, or ASIA1 (US 5,864,008 and US 6,107, 021);
xviii. The hypervariable region-1 (HVR1) of the E2 protein of hepatitis C virus (HCV) variant AD78 (Zibert et al. (1997) Virol. 71:4123- 4127);
xix. Sequences of Hepatitis B virus selected from:
   surface antigen (Kobayashi and Kolke (1984) Gene 30:227), for example LVLLDYQGMLPVCPL and TKPSDGNCTCIPIPS; and precursor surface antigen MQWNSTTFHQALL;
xx. Sequences from Influenza virus selected from:
   Nucleoprotein (Gregory et al. (2001) J. Gen. Virol. 82:1397), for example MFEDLRVSSFIRGT and SNENMETMDSSTLE; Hemagglutinin, for example HPLILDTCTIEGLIYGNPS; YQRIQIFPDT; and IQIFPDTIWNVSYSGTSK; and
xxi. Sequence from Hepatitis C virus, for example
   GGPTRTIGGSQAQTASGLVSMFSVGPSQK

Particular examples of bacteria-specific B cell epitopes may be those derived from and/or capable of generating antibodies against *Pasteurella, Actinobacillus, Haemophilus, Listeria monocytogenes, Mycobacteria, Staphylococci, E. coli, Shigella,* and the like. Suitable bacterial B cell epitopes include, but are not limited to epitopes selected from the group consisting of:
i. *Mycobacterium tuberculosis* 65Kd protein amino acid residues 112-126 (Lamb et al. (1987) EMBOJ. 6:1245);
ii. *M tuberculosis* 65Kd protein amino acid residues 163-184 (Lamb et al. (1987) EMBOJ. 6:1245);
iii. *M tuberculosis* 65Kd protein amino acid residues 227-243 (Lamb et al. (1987) EMBOJ. 6:1245);
iv. *M. tuberculosis* 65Kd protein amino acid residues 242-266 (Lamb et al. (1987) EMBOJ. 6:1245);
v. *M tuberculosis* 65Kd protein amino acid residues 437-459 (Lamb et al. (1987) EMBOJ. 6:1245);
vi. *M tuberculosis* ESAT-6 protein residues 3-15 (Morten et al., Infect Immun. 66, 717-723, 1998);
vii. *M tuberculosis* ESAT-6 protein residues 40-62 (Morten et al. (1998) Infect. Immun. 66:717);
viii. *Mycobacterium scrofulaceum* α-antigen residues 279-290 (Mikiko et al. (1997) Microb. Path. 23:95);
ix. *Staphylococcus aureus* nuclease protein amino acid residues 61-80 (Finnegan et al. (1986) J. Exp. Med. 164:897);
x. a B cell epitope of *Escherichia coli* heat stable enterotoxin (Cardenas et al. (1993) Infect. Immunity 61:4629);
xi. a B cell epitope of Escherichia coli heat labile enterotoxin (Clements et al. (1986) Infect. Immunity 53:685);
xii. a B cell epitope of *Shigella sonnei* form I antigen (Formal et al. (1981) Infect Immunity 34:746);
xiii. a B cell epitope from Group A Streptococcus, preferably derived from the M protein, more preferably from the C-terminal half of the M protein so and more preferably a minimum, helical, non-host-cross-reactive peptide derived from the conserved C-terminal half of the M protein and comprising a non-M-protein peptide designed to maintain helical folding and antigenicity displayed within said minimum, helical, non-host-cross-reactive peptide. For example, the non-M-protein peptide (e. g., peptide J14) may be linked to one or more serotypic M protein peptides using chemistry that enables the immunogen to display all the individual peptides pendant from a (alkane) backbone, thereby conferring excellent immunogenicity and protection (US 6,174,528) and (Brandt et al. (2000) Nat. Med. 6: 455);
xiv. a B cell epitope of the Cholera toxin B subunit (CTB), such as, for example described in (Kazemi and Finkelstein (1991) Mol. Immunol. 28:865);
xv. a B cell epitope of a protein of *Bacillus anthracis* (anthrax), such as, for example, a B cell epitope derived from a protein of the outer exosporium of anthrax such as the 250 kDa glycoprotein (Sylvestre et al. (2001) In: Proc. 4th Int. Conf. Anthrax, St. John's College, Annapolis, MD, June 10-13, Abstract 31 B); and
xvi. a B cell epitope from a protein of tetanus, such as, for example, the tetanus toxoid protein.

Particular non-limiting examples of B cell epitopes from mammalian subjects may be those derived from and/or capable of generating antibodies against a tumor antigen. Tumor antigens are usually native or foreign antigens, the expression of which is correlated with the development, growth, presence or recurrence of a tumor. In as much as tumor antigens are useful in differentiating abnormal from normal tissue, they are useful as a target for therapeutic intervention. Tumor antigens are well known in the art. Non-limiting examples of tumor antigens include, but are not limited to carcinoembryonic antigen (CEA), prostate specific antigen (PSA), CA-125, CA-19-9, CA-15-3, CA-549, CA-72-4, CA-50, Friedenreich Antigen (T), Le^{b} Antigen, Forssman Antigen, melanoma antigens (MAGE, BAGE, GAGE) and mucins, such as MUC-1. Tumor antigens may also be carbohydrates such as globo-H, Tn, and sialyl Le^{a}.

In particular non-limiting examples, peptides comprising B cell target epitopes may comprise amino acid sequences selected from sequences from prostate specific antigen (PSA, U.S. 6,326,471) selected from the group consisting of:
LYTKWHYRKWIKDTIVANP;
AVKVMDLPQEPALGTTCYA;
IVGGWECEKHSQPWQVLVAS;
CAQVHPQKVTKFML;
YLMLLRLSEPAELTDDAVKVM;
LLKNRFLRPGDDSSHDLMLLY; and
ILLGRHSLFHPEDTGQVFQVY,
or a sequence from carcinoembryonic antigen (CEA) PPAQYSWLIDGN.

It is to be understood that the compositions and methods of the present invention are amenable for use with these and other known peptides and carbohydrates that have been implicated as B cell epitopes involved in disease states of interest. Clearly, the present invention is intended to encompass any other such peptide or carbohydrate that may in future be disclosed that may be used as the B cell target epitope according to the principles of the present invention.

Suppressive Target Epitopes

The suppressive target epitopes as used in the present invention, may be epitopes derived from peptide sequences or carbohydrates involved in any one or more autoimmune diseases or disorders, including, but not limited to: diabetes mellitus, arthritis (including rheumatoid arthritis, juvenile rheumatoid arthritis, osteoarthritis, psoriatic arthritis), multiple sclerosis, myasthenia gravis, systemic lupus erythematosis (SLE), autoimmune thyroiditis, dermatitis (including atopic dermatitis and eczematous dermatitis), psoriasis, Sjogren's Syndrome, including keratoconjunctivitis sicca secondary to Sjogren's Syndrome, alopecia areata, allergic responses due to arthropod bite reactions, Crohn's disease, aphthous ulcer, iritis, conjunctivitis, keratoconjunctivitis, ulcerative colitis, asthma, allergic asthma, cutaneous lupus erythematosus, scleroderma, vaginitis, proctitis, drug eruptions, leprosy reversal reactions, erythema nodosum leprosum, autoimmune uveitis, allergic encephalomyelitis, acute necrotizing hemorrhagic encephalopathy, idiopathic bilateral progressive sensorineural hearing loss, aplastic anemia, pure red cell anemia, idiopathic thrombocytopenia, polychondritis, Wegener's granulomatosis, chronic active hepatitis, Stevens-Johnson syndrome, idiopathic sprue, lichen planus, Graves ophthalmopathy, sarcoidosis, primary biliary cirrhosis, uveitis posterior, and interstitial lung fibrosis.

Examples of known antigens involved in autoimmune diseases include, but are not limited to, myelin basic protein, myelin oligodendrocyte glycoprotein and myelin proteolipid protein (involved in multiple sclerosis), acetylcholine receptor components (involved in myasthenia gravis), collagen and Mycobacterial hsp peptide 180-188 (involved in arthritis), laminin and p53 peptide (involved in systemic lupus erythematosis).

In a particular non-limiting example, the suppressive target epitope may be a myelin basic protein fragment, for the treatment of multiple sclerosis. In a further example, the myelin basic protein peptide having for example the sequence disclosed in U.S. 6,489,299, denoted herein as: Pro-Lys-Tyr-Val-Lys-Gin-Asn-Thr-Leu-Lys-Leu-Ala-Thr (MBP 87-99).

The suppressive target epitope may also be, for example and not wishing to be limiting, acetylcholine receptor antigen or one of its peptides for the treatment of myasthenia gravis. In a specific non-limiting example, the acetylcholine receptor peptides used may be the p259 peptide (Zisman et al. (1995) Hum. Immunol. 44:121) and (brocks et al. (1990) Immunology 69:495). In another example, the acetylcholine receptor peptides used may be fragments which comprise the amino acid residues 61-76 of the hAChR or fragments which comprise the amino acid residues 184-210 of the hAChR.

The suppressive target epitope may also be, in a non-limiting example, a collagen fragment for the treatment of arthritis. In a further example, the collagen fragment may be, for example, a collagen type C11 peptide 245-270 having the sequence disclosed in U.S. 6,423,315 and denoted herein as:
SPTGPLGPKGQTGELGIAGFKGEQGPK.

In a particular non-limiting example, the suppressive target epitope may be a laminin fragment for the treatment of systemic lupus erythematosis. Peptides derived from the C-terminal or N-terminal of mouse laminin chain may be used. In a specific, non-limiting example, the suppressive target epitope may be an amino acid sequence of laminin fragments are disclosed for example in U.S. Patent No. 6,228,363, including:
RPVRHAQCRVCDGNSTNPRERH;
KNLEISRSTEDLLRNSYGVRK;
TSLRKALLHAPTGSYSDGQ;
KATPMLKMRTSFHGCIK;
DGKWHTVKTEYIKRKAF;
KEGYKVRLDLNITLEFRTTSK; and
KQNCLSSRASFRGCVRNLRLSR.

It is to be understood that the compositions and methods of the present invention are amenable for use with these and other known peptides and carbohydrates that have been implicated as epitopes involved in autoimmunity. Clearly, the present invention is intended to encompass any other such peptide or carbohydrate that may in the future be disclosed that may be used as the suppressive target epitope according to the principles of the present invention.

Synthetic Methodology

Retrosythetic analysis (Corey and Cheng (1995) The Logic of Chemical Synthesis, John Wiley and Sons, New York: Chapter 1) applied to the generalized antigen specific SPA reveals two general methods for construction of the SPAs of the present invention.

In a non-limiting example and for purposes of illustration only, a generalized stimulatory *mono*SPA (for example, as shown in Figure 3) is used to demonstrate the first method. It will be recognized by the skilled artisan that this methodology can be employed to synthesize all categories of SPA described herein.

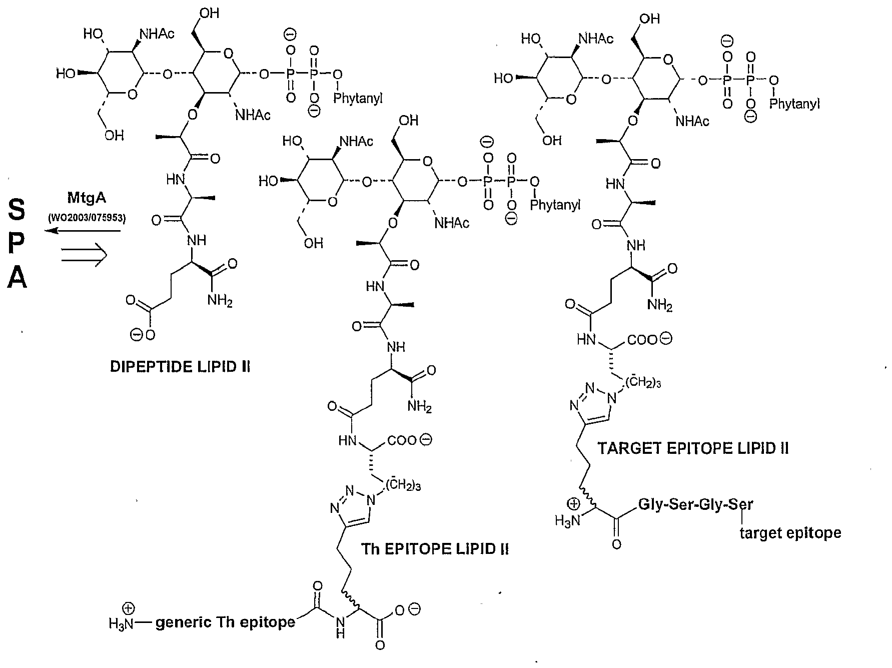

The first retrosynthetic disconnection (open arrow, left to right above) affords three different lipids II, immediate precursors of the SPA. In the synthetic direction (line arrow, right to left above) the action of MtgA and cofactors in aqueous solution at room temperature (as described in WO 2003/075953, which is incorporated herein by reference in its entirety) may produce the random copolymer SPA with repeat units in the same mole fraction as mole fractions of the lipids II starting materials.

Dipeptide lipid II may be synthesized utilizing the methodology described in WO 2003/075953.

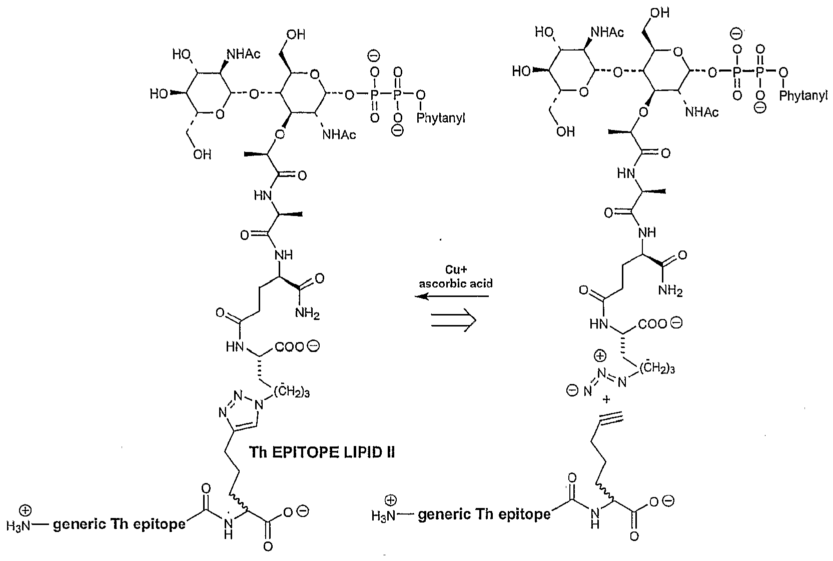

A second retrosynthetic disconnection (open arrow, left to right above), applied to the Th epitope lipid II, affords a tripeptide lipid II (Ala-D-iso-Gln-bisnor-azidolysine) and a generic Th epitope that is C-terminally modified by the unnatural amino acid rac-4-pentynylglycine. The two components may be assembled in the synthetic direction (line arrow, right to left above) by the action of cuprous ion and ascorbic acid in aqueous solution (Rostovtsev et al. (2002) Angew. Chem. Int. Ed. 114:2708).

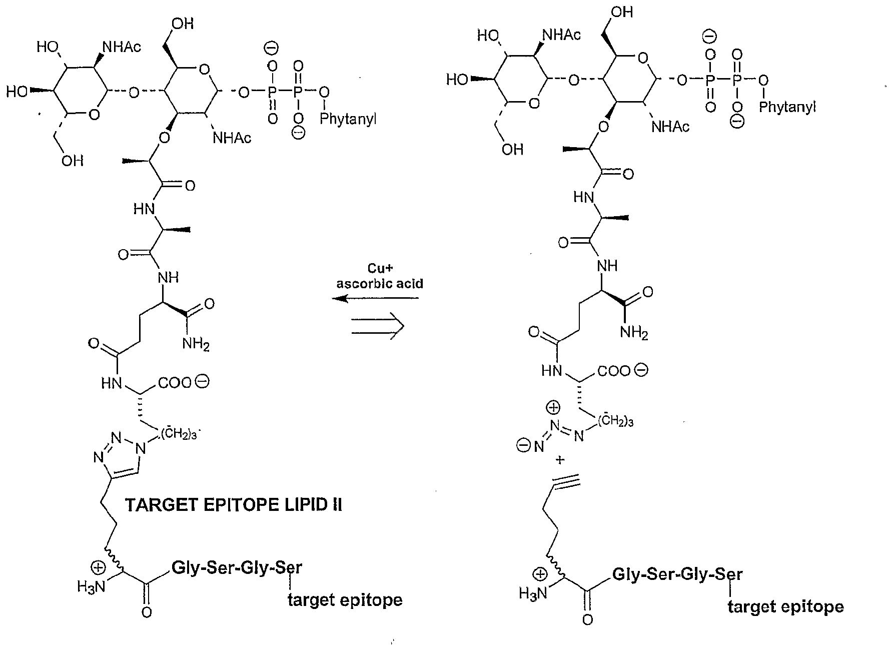

In a similar manner, a third retrosynthetic disconnection (open arrow, left to right above), applied to the target epitope(s) lipid(s) II, afford(s) the same tripeptide lipid II (Ala-D-iso-Gln-bisnor-azidolysine) and target epitope or epitopes that is/are C-tenninally modified by the unnatural amino acid *rac*-4-pentynylglycine. The components may be assembled in the synthetic direction (line arrow, right to left above) by the action of cuprous ion and ascorbic acid in aqueous solution (Rostovtsev et al. (2002) Angew. Chem. Int. Ed. 114:2708).

The azido lipid II is synthesized utilizing the methodology described in WO 2003/075953. The *bisnor*-azidolysine component may be prepared by standard methodology via displacement of the *homo*-serine *p*-toluenesulfonate by azide ion in dipolar aprotic solvent.

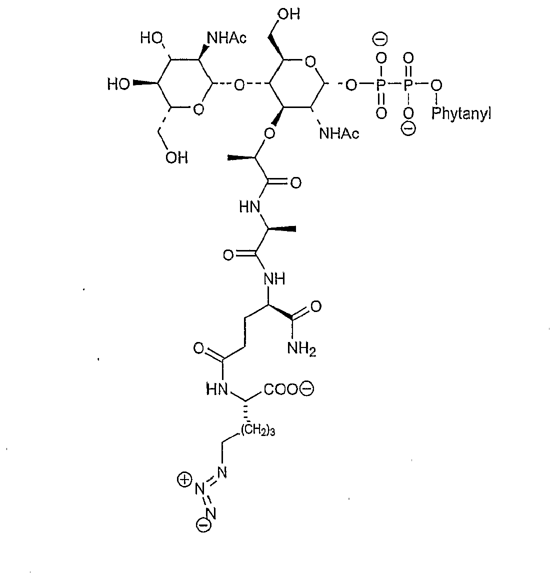

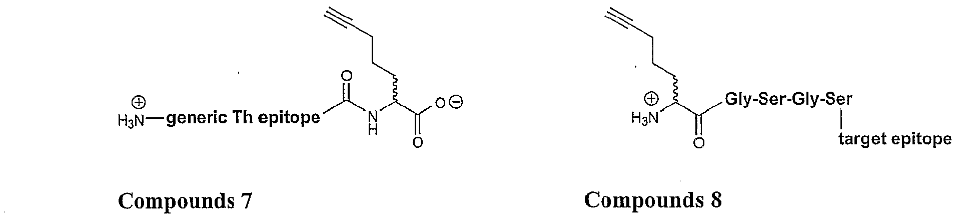

The C-terminally modified Th epitopes and N-terminally modified peptidic target epitopes may be synthesized by standard solid-phase peptide synthesis techniques (Atherton and Shepard (1989) Solid Phase Peptide Synthesis: A Practical Approach, Irl Pr Publishing). Carbohydrate target epitopes as their alkeneoxy (e.g., allyl, 4-pentenyl) glycosides may be ozonized to the corresponding aldehydes and reductively condensed with the ε-amino group of suitably protected lysine (Slovin et al. (1999) PNAS 96:5710). The carbohydrate-derivatized lysines thus obtained may then be incorporated into standard peptide synthesis methodology.

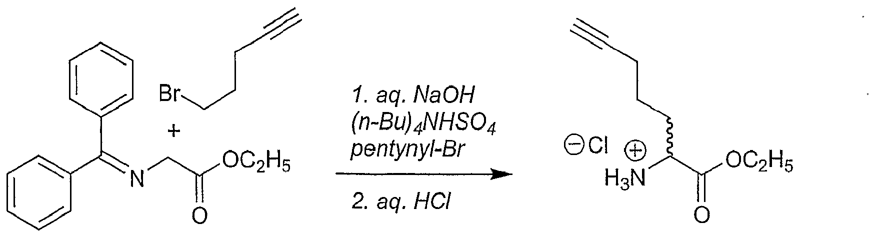

The unnatural alkyne amino acid may be prepared in the racemic modification by the glycine-imine method (O'Donnell et al. J. Am. Chem. Soc. (1989) 111 :2353) from commercially available materials.

A generalized suppressive monoSPA (for example, as shown in Figure 7) is used as a non-limiting example and for purposes of illustration only, to demonstrate an alternative synthetic route. A person of skill in the art will recognize that this methodology can be employed to synthesize all categories of SPA described herein. The first alternative retrosynthetic disconnection reveals a pre-SPA carbohydrate polymer substituted appropriately to accept any epitope with the required N-tenninal sequence: [tethered alkynyl Gly-Gly-Ser-Gly-Ser-target epitope], i.e., **Compound(s) 8.**

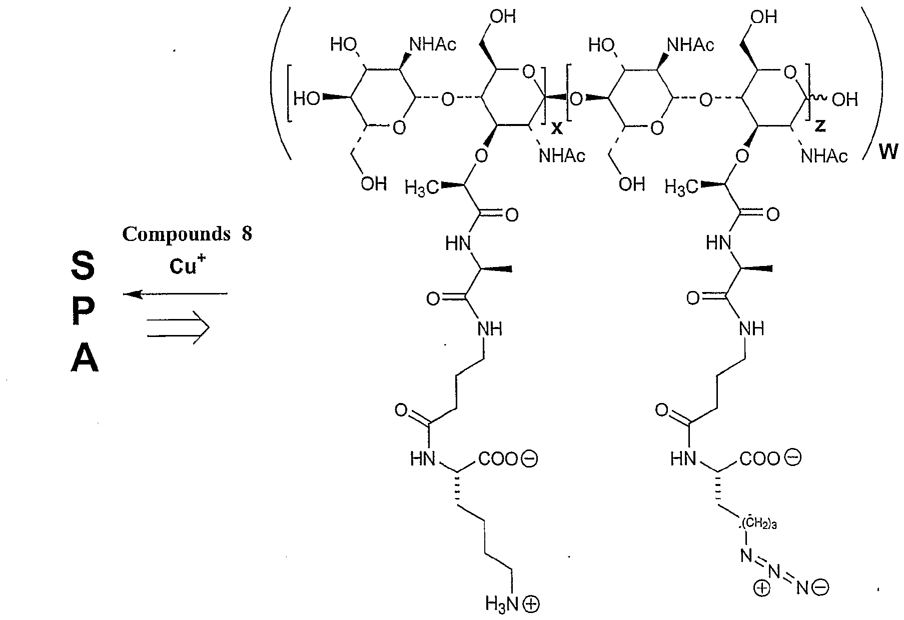

The azide-containing polymeric precursor may be prepared from the component lipids II by the usual method (WO 2003/075953, which is incorporated herein by reference in its entirety).

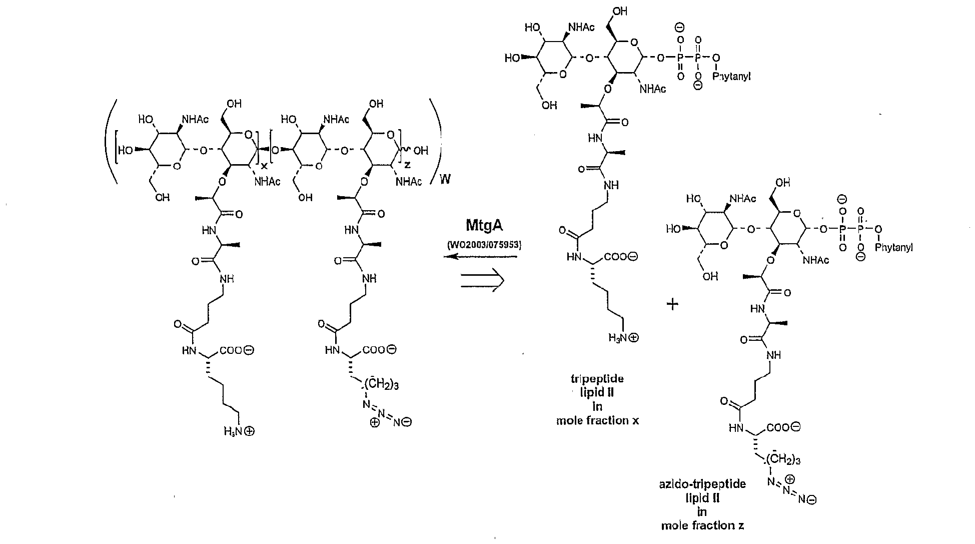

Finally, the component lipids II are synthesized by the established methodology disclosed in (WO 2003/075953). It will be further recognized by the skilled artisan that the first alternative synthetic route and the second alternative synthetic route could each be preferred, depending on the precise SPA to be synthesized.

It should be appreciated that the examples described above are for illustrative purposes only, and are not meant to limit the scope of the present invention.

Pharmaceutical Compositions and Their Formulation

Depending on their structure, the *mono*- and *poly*SPAs disclosed herein can be used either to prevent or treat inflammatory pathologies or to induce inflammation in connection with various disease states or conditions in which such inflammation provides a beneficial treatment or prophylactic effect in humans and other animals. Thus, in one aspect, the present invention provides pharmaceutical compositions for human and veterinary medical use comprising a *mono-* and *poly*SPAs, or a pharmaceutically acceptable salt thereof, together with one or more pharmaceutically or physiologically acceptable buffers, carriers, excipients, or diluents, and optionally, other therapeutic agents. It should be noted that compounds of the present invention may be administered individually, or in mixtures comprising two or more compounds. The present invention also encompasses the use of *mono-* and *poly*SPAs, or a pharmaceutically acceptable salt thereof, for the preparation of a medicament for the prevention or treatment of an inflammatory pathology, or a disease state or condition in which an inflammatory immune response is beneficial. Choice of a pro-inflammatory SPA or a suppressive SPA for these uses depends upon which type of immune response is desired for therapeutic purposes.

The compounds of the present invention can be administered in pharmaceutically or physiologically acceptable solutions that can contain pharmaceutically or physiologically acceptable concentrations of salts, buffering agents, preservatives, compatible carriers, diluents, excipients, dispersing agents, etc., and optionally, other therapeutic ingredients. For example, **Compound 1 and Compound 2** are soluble up to ca. 20 mg/mL in water at neutral pH. Furthermore, aqueous solutions of this compound can accommodate low (about 0.5 to about 5) weight percentages of glycerol, sucrose, and other such pharmaceutically acceptable excipient materials. The SPAs of the present invention can thus be formulated in a variety of standard pharmaceutically acceptable parenteral formulations.

Net Charge and Aggregation

Balanced charge zwitterionic molecules of the present invention having equal numbers of positive and negative charges per repeat unit can, over time, aggregate with one another and/or compress intramolecularly due to charge-charge attractive forces. For example, **Compound 1** disclosed herein is a representative balanced charge zwitterionic molecule that exhibits desirable anti-inflammatory activity. Retention of anti-inflammatory immunomodulatory activity overtime by molecules of this type, and by suppressive *mono*- and *poly*SPAs, in pharmaceutical compositions can be optimized by formulation techniques that minimize aggregation, such as the inclusion of surfactants or dispersing agents, e.g., polyethylene glycol, glycerol, sucrose, etc.

Advantageously, linear polymers of the present invention possessing a net positive or negative charge per repeat unit at physiological pH due to their peptidic moieties maintain charge-charge repulsion. Such molecules therefore exhibit ideal solution behavior, i.e., an extended solution state with minimal intramolecular or intermolecular aggregation, events which may diminish immunological activity over time, especially at low ionic strength. Therefore, molecules of the present invention with a net positive or negative charge per repeat unit will behave as polyelectrolytes, and possess the advantage that they will exhibit enhanced solution, and therefore storage, behavior. The polyelectrolyte charge-charge repulsion phenomenon has been observed directly by atomic force microscopy (AFM) for poly(2-vinylpyridine) (Minko et al. (2002) J. Am. Chem. Soc. 124:3218). Furthermore, the immunomodulatory activities of synthetic polysaccharide antigens of *mono-* and *poly*SPAs exhibiting a net positive or negative charge per repeat unit are significantly enhanced by the intra- and intermolecular charge-charge repulsive forces that keep these molecules from aggregating, facilitating proper display of their structural features to cellular receptors.

The pharmaceutical compositions of the present invention may contain an effective amount of the presently disclosed compounds, optionally included in a pharmaceutically or physiologically acceptable buffer, carrier, excipient, or diluent. The term "pharmaceutically or physiologically acceptable buffer, carrier, excipient, or diluent" means one or more than one compatible solid or liquid fillers, dilutants, or encapsulating substances that are suitable for administration to a human or other animal. The term "carrier" denotes an organic or inorganic ingredient, natural or synthetic, with which the active ingredient is combined to facilitate the application. The components of the pharmaceutical compositions are capable of being commingled with the polymers of the present invention, and with each other, in a manner such that there is no interaction that would substantially impair the desired pharmaceutical efficiency of the active compound(s).

Compositions suitable for parenteral administration conveniently comprise sterile aqueous preparations, which can be isotonic with the blood of the recipient. Among the acceptable vehicles and solvents are water, Ringer's solution, and isotonic sodium chloride solution. In addition, sterile, fixed oils are conventionally employed as a solvent or suspending medium. For this purpose, any bland fixed oil can be employed, including synthetic mono- or diglycerides. In addition, fatty acids such as oleic acid are useful in the preparation of injectables. Carrier formulations suitable for subcutaneous, intramuscular, intraperitoneal, intravenous, etc. administrations can be found in Remington: The Science and Practice of Pharmacy, 19th Edition, A.R. Gennaro, ed., Mack Publishing Co., Easton, Pa., (1995).

The compositions can be conveniently presented in unit dosage form or dosage unit form, and can be prepared by any of the methods well known in the art of pharmacy. All methods include the step of bringing the compound into association with a carrier that constitutes one or more accessory ingredients. In general, the compositions are prepared by uniformly and intimately bringing the compound into association with a liquid carrier, a finely divided solid carrier, or both, and then, if necessary, shaping the product. Compounds of the present invention can be stored lyophilized.

Other delivery systems can include time-release, delayed-release, or sustained- release delivery systems. Such systems can avoid repeated administrations of the anti-inflammatory or inflammatory agent, increasing convenience to the subject and the physician. Many types of release delivery systems are available and known to those of ordinary skill in the art, including polymer-based systems such as poly(lactide-glycolide), copolyoxalates, polycaprolactones, polyesteramides, polyorthoesters, polyhydroxybutyric acid, and polyanhydrides.

Microcapsules of the foregoing polymers containing drugs are described in, for example, U.S. Patent 5,075,109, which is incorporated herein by reference. Delivery systems also include non-polymer systems such as: lipids, including sterols such as cholesterol, cholesterol esters, and fatty acids or neutral fats such as mono-, di-, and tri-glycerides; hydrogel release systems; silastic systems; peptide-based systems; wax coatings; compressed tablets using conventional binders and excipients; partially fused implants; and the like. Specific examples include, but are not limited to: (a) erosional systems in which an agent of the invention is contained in a form within a matrix such as those described in U.S. Patent Nos. 4,452,775, 4,675,189, and 5, 736,152, which are incorporated herein by reference, and (b) diffusional systems in which an active component permeates at a controlled rate from a polymer such as described in U.S. Patent Nos. 3,854,480, 5,133,974 and 5, 407,686, which are incorporated herein by reference. In addition, pump-based hardware delivery systems can be used, some of which are adapted for implantation.

Dosing, Treatment Regimen, and Administration

Appropriately selected compounds of the present invention can be administered in an effective amount for either inducing protection against a wide variety of different inflammation-based pathologies, including post-surgical adhesions and intra-abdominal abscesses associated with bacterial infection, or for inducing inflammation in connection with various disease states or disorders in which such inflammation provides a beneficial treatment or prophylactic effect. For such purposes, an effective amount is that amount of an anti-inflammatory or inflammatory compound of the present invention that will, alone or together with further doses or additional therapeutic compounds, either inhibit, ameliorate, or prevent the inflammation-based pathology, or stimulate a therapeutically beneficial inflammatory response, respectively. The dose range can be from about one picogram/kilogram bodyweight to about one milligram/kilogram bodyweight, or from about one nanogram/kilogram bodyweight to about one microgram/kilogram bodyweight. The absolute amount will depend upon a variety of factors, including the nature of the disease or disorder to be treated, whether the administration is in conjunction with elective surgery or emergency surgery, concurrent treatment, the number of doses, individual patient parameters including age, physical condition, size and weight, and the severity of the disease or disorder to be treated, and can be determined by the medical practitioner with no more than routine experimentation. It is generally preferred that a maximum dose be used, that is, the highest safe dose according to sound medical judgment. Multiple doses of the pharmaceutical compositions of the invention are contemplated.

Determination of the optimal amount of compound to be administered to human or animal patients in need of prevention or treatment of an inflammation-based pathology, or a disease or disorder which benefits from immune system stimulation, as well as methods of administering therapeutic or pharmaceutical compositions comprising such compounds, is well within the skill of those in the pharmaceutical, medical, and veterinary arts. Dosing of a human or animal patient is dependent on the nature of inflammation-based pathology or other disease or disorder to be treated, the patient's condition, body weight, general health, sex, diet, time, duration, and route of administration, rates of absorption, distribution, metabolism, and excretion of the compound, combination with other drugs, severity of the inflammation-based pathology or other disease or disorder to be treated, and the responsiveness of the pathology or disease state being treated, and can readily be optimized to obtain the desired level of effectiveness. The course of treatment can last from several days to several weeks or several months, or until a cure is effected or an acceptable diminution or prevention of the disease state is achieved. Optimal dosing schedules can be calculated from measurements of drug accumulation in the body of the patient in conjunction with the effectiveness of the treatment. Persons of ordinary skill can easily determine optimum dosages, dosing methodologies, and repetition rates. Optimum dosages can vary depending on the potency of the immunomodulatory polymeric compound, and can generally be estimated based on ED50 values found to be effective in in vitro and in vivo animal models. Effective amounts of the present compounds for the treatment or prevention of inflammation-based pathologies or other diseases or disorders to be treated, delivery vehicles containing these compounds, agonists, and treatment protocols, can be determined by conventional means. For example, the medical or veterinary practitioner can commence treatment with a low dose of the compound in a subj ect or patient in need thereof, and then increase the dosage, or systematically vary the dosage regimen, monitor the effects thereof on the patient or subject, and adjust the dosage or treatment regimen to maximize the desired therapeutic effect. Further discussion of optimization of dosage and treatment regimens can be found in Benet et al., in Goodman & Gihnan's The Pharmacological Basis of Therapeutics, Ninth Edition, Hardman et al., Eds., McGraw-Hill, New York, (1996), Chapter 1, pp. 3-27, and L.A. Bauer, in Pharmacotherapy, A Pathophysiologic Approach, Fourth Edition, DiPiro et al., Eds., Appleton & Lange, Stamford, Connecticut, (1999), Chapter 3, pp.21-43, and the references cited therein, to which the reader is referred.

A variety of administration routes are available. The particular mode selected will depend upon which compound is selected, the particular condition being treated, and the dosage required for therapeutic efficacy. Generally speaking, the methods of the present invention can be practiced using any mode of administration that is medically acceptable, meaning any mode that produces effective levels of an immune response without causing clinically unacceptable adverse effects. Preferred modes of administration are parenteral routes, although oral administration can also be employed. The term "parenteral" includes subcutaneous, intravenous, intramuscular, or intraperitoneal injection, or infusion techniques.

In the context of the present invention, the terms "treatment," "therapeutic use," or "treatment regimen" as used herein are meant to encompass prophylactic, palliative, and therapeutic modalities of administration of the immunomodulatory polymers of the present invention, and include any and all uses of the presently claimed compounds that remedy a disease state, condition, symptom, sign, or disorder caused by an inflammation-based pathology or other disease or disorder to be treated, or which prevents, hinders, retards, or reverses the progression of symptoms, signs, conditions, or disorders associated therewith. Thus, any prevention, amelioration, alleviation, reversal, or complete elimination of an undesirable disease state, symptom, condition, sign, or disorder associated with an inflammation-based pathology, or other disease or disorder that benefits from stimulation of the body's immune response, is encompassed by the present invention.

For purposes of the present invention, the meaning of the terms "treating," "treatment," and the like as applied to cancer therapy is broad, and includes a wide variety of different concepts generally accepted in the art. Thus, as used herein, this term includes, but is not limited to, prolongation of time to progressive disease; tumor reduction; disease remission; relief of suffering; improvement in life quality; extension of life; amelioration or control of symptoms such as pain, difficulty breathing, loss of appetite and weight loss, fatigue, weakness, depression and anxiety, confusion, etc.; improvement in patient comfort, etc. A separate goal may even be to cure the disease entirely.

The term "cancer" has many definitions. According to the American Cancer Society, cancer is a group of diseases characterized by uncontrolled growth (and sometimes spread) of abnormal cells. Although often referred to as a single condition, it actually consists of more than 200 different diseases. Cancerous growths can kill when such cells prevent normal function of vital organs, or spread throughout the body, damaging essential systems.

The present invention provides a method of treating susceptible neoplasms in a mammal that comprises administering to a mammal in need of said treatment an oncolytically effective amount of a compound of the present invention.

Non-limiting examples of different types of cancers against which compounds of the present invention may be effective as therapeutic agents include, but are not limited to: carcinomas, such as neoplasms of the central nervous system, including glioblastoma multiforme, astrocytoma, oligodendroglial tumors, ependymal and choroid plexus tumors, pineal tumors, neuronal tumors, medulloblastoma, schwannoma, meningioma, and meningeal sarcoma; neoplasms of the eye, including basal cell carcinoma, squamous cell carcinoma, melanoma, rhabdomyosarcoma, and retinoblastoma; neoplasms of the endocrine glands, including pituitary neoplasms, neoplasms of the thyroid, neoplasms of the adrenal cortex, neoplasms of the neuroendocrine system, neoplasms of the gastroenteropancreatic endocrine system, and neoplasms of the gonads; neoplasms of the head and neck, including head and neck cancer, neoplasms of the oral cavity, pharynx, and larynx, and odontogenic tumors; neoplasms of the thorax, including large cell lung carcinoma, small cell lung carcinoma, non-small cell lung carcinoma, malignant mesothelioma, thymomas, and primary germ cell tumors of the thorax; neoplasms of the alimentary canal, including neoplasms of the esophagus, stomach, liver, gallbladder, the exocrine pancreas, the small intestine, veriform appendix, and peritoneum, adneocarcinoma of the colon and rectum, and neoplasms of the anus; neoplasms of the genitourinary tract, including renal cell carcinoma, neoplasms of the renal pelvis, ureter, bladder, urethra, prostate, penis, testis; and female reproductive organs, including neoplasms of the vulva and vagina, cervix, adenocarcinoma of the uterine corpus, ovarian cancer, gynecologic sarcomas, and neoplasms of the breast; neoplasms of the skin, including basal cell carcinoma, squamous cell carcinoma, dermatofibrosarcoma; Merkel cell tumor, and malignant melanoma; neoplasms of the bone and soft tissue, including osteogenic sarcoma, malignant fibrous histiocytoma, chondrosarcoma, Ewing's sarcoma, primitive neuroectodermal tumor, and angiosarcoma; neoplasms of the hematopoietic system, including myelodysplastic sydromes, acute myeloid leukemia, chronic myeloid leukemia, acute lymphocytic leukemia, HTLV-1 and 5 T-cell leukemia/lymphoma, chronic lymphocytic leukemia, hairy cell leukemia, Hodgkin's disease, non-Hodglcin's lynphomas, and mast cell leukemia; and neoplasms of children, including acute lymphoblastic leukemia, acute myelocytic leukemias, neuroblastoma, bone tumors, rhabdomyosarcoma, lymphomas, and renal tumors.

A particular treatment regimen can last for a period of time which may vary depending upon the nature of the particular inflammation-based pathology or other disease or disorder to be treated, its severity, and the overall condition of the patient, and may involve administration of compound-containing compositions from once to several times daily for several days, weeks, months, or longer. Following treatment, the patient is monitored for changes in his/her condition and for alleviation of the symptoms, signs, or conditions of the disorder or disease state. The dosage of the composition can either be increased in the event the patient does not respond significantly to current dosage levels, or the dose can be decreased if an alleviation of the symptoms of the disorder or disease state is observed, or if the disorder or disease state has been ablated.

An optimal dosing schedule is used to deliver a therapeutically effective amount of the compounds of the present invention. For the purposes of the present invention, the terms "effective amount" or "therapeutically effective amount" with respect to the compounds disclosed herein refers to an amount of compound that is effective to achieve an intended purpose, preferably without undesirable side effects such as toxicity, irritation, or allergic response. Although individual patient needs may vary, determination of optimal ranges for effective amounts of pharmaceutical compositions is within the skill of the art. Human doses can be extrapolated from animal studies (A.S. Katocs, Remington: The Science and Practice of Pharmacy, 19th Ed., A.R. Gennaro, ed., Mack Publishing Co., Easton, Pa., (1995), Chapter 30). Generally, the dosage required to provide a therapeutically effective amount of a pharmaceutical composition, which can be adjusted by one skilled in the art, will vary depending on the age, health, physical condition, weight, type and extent of the disease or disorder of the recipient, frequency of treatment, the nature of concurrent therapy (if any), and the nature and scope of the desired effect(s) (Nies et al., Goodman & Gilman's The Pharmacological Basis of Therapeutics, 9th Ed., Hardman et al., eds., McGraw-Hill, New York, N.Y., 1996, Chapter 3).

Prophylactic modalities for high risk individuals are also encompassed by the present invention. As used herein, the term "high risk individual" is meant to refer to an individual for whom it has been determined, via, e.g., individual or family history or genetic testing, living or working environment or conditions, etc., that there is a significantly higher than normal probability of being susceptible to an inflammation-based pathology or the onset or recurrence of an associated disease or disorder, or a disease/disorder that will benefit from a stimulation of the body's immune response. For example, a patient could have a personal and/or family medical history that includes frequent occurrences of a particular disease or disorder. As another example, a patient could have had such a susceptibility determined by genetic screening according to techniques known in the art (see, e.g., U.S. Congress, Office of Technology Assessment, Chapter 5 In: Genetic Monitoring and Screening in the Workplace, OTA-BA-455, U.S. Government Printing Office, Washington, D. C., 1990, pages 75-99). In the case of viral diseases, environment can be a predisposing factor. In the case of cancer, both genetics and environment can be predisposing factors. As part of a treatment regimen for a high risk individual, the individual can be prophylactically treated to prevent inflammation-based pathologies or the onset or recurrence of the disease, disorder, sign, symptom, or condition, or diseases/disorders that will benefit from an enhanced immune response. The term "prophylactically effective amount" is meant to refer to an amount of a pharmaceutical composition of the present invention that produces an effect observed as the prevention of infection or inflammation, or the onset or recurrence of an inflammatory disease, symptom, sign, condition, or disorder, or a disease/disorder that benefits from a stimulation of the body's immune response. Prophylactically effective amounts of a pharmaceutical composition are typically determined by the effect they have compared to the effect observed when a second pharmaceutical composition lacking the active agent is administered to a similarly situated individual.

For therapeutic use, the immunomodulatory compounds disclosed herein can be administered to a patient suspected of suffering from an infectious disease or cancer based pathology in an amount effective to reduce the symptomology of the disease, symptom, sign, condition, or disorder, or suffering from a disease or disorder that will benefit from an enhanced immune response. One skilled in the art can determine optimum dosages and treatment schedules for such treatment regimens by routine methods.

The present invention is useful whenever it is desirable to prevent bacterial abscess or adhesion formation in a human or animal subject. This includes prophylactic treatment to prevent such conditions in planned surgical procedures, as well as in emergency situations. Any regimen that results in an enhanced immune response to bacterial infection/contamination and subsequent abscess/adhesion formation can be used, although optimal doses and dosing regimens are those which would not only inhibit the development of abscess and/or adhesion formation, but also would result in a complete protection against abscess or adhesion formation by a particular bacterial organism or a variety of bacterial organisms. Desired time intervals for delivery of multiple doses of a particular polymer can be determined by one of ordinary skill in the art employing no more than routine experimentation.

The present methods are also useful in connection with diseases that predispose a subject to abscess formation such as pelvic inflammatory disease, inflammatory bowel disease, urinary tract infections, and colon cancer. The present methods are therefore useful with abscesses of virtually any tissue or organ, including specifically, but not limited to, dermal abscesses such as acne. Those of ordinary skill in the art to which this invention pertains will readily recognize the range of conditions and procedures in which the present invention is applicable.

The doses for administration may range from about one picogram/kilogram bodyweight to about one milligram/kilogram bodyweight, or from about one nanogram/kilogram bodyweight to about one microgram/kilogram bodyweight, will be effective, depending upon the mode of administration. The absolute amount will depend upon a variety of factors (including whether the administration is in conjunction with elective surgery or emergency surgery, concurrent treatment, number of doses, and individual patient parameters including age, physical condition, size and weight), and can be determined via routine experimentation. It is preferred generally that a maximum dose be used, that is, the highest safe dose according to sound medical judgment.

Multiple doses of the pharmaceutical compositions of the present invention are contemplated for inducing protection against adhesion formation. Such multiple doses can be administered over a three day period beginning on the day preceding surgery. Further doses can be administered post surgery as well. Any regimen that results in a reduced postoperative surgical adhesion formation can be used, although optimum doses and dosing regimens are those which would not only inhibit the development of postoperative surgical adhesion formation, but would also result in complete protection against postoperative surgical adhesion formation. Desired time intervals for delivery of multiple doses of one of the present immunomodulatory polymers can be determined by one of ordinary skill in the art employing no more than routine experimentation.

The compounds of the present invention can be administered systemically, or locally into the site at which it is desirable to reduce the likelihood of adhesion formation. The compounds of the present invention can be administered as an aqueous solution, as a crosslinked gel, or as any temporal or physical combination of aqueous solution and crosslinked gel forms. The immunomodulatory polymer can also be effective when given subcutaneously locally at the site, or apart from the site at which adhesions are likely to form.

The preparations of the present invention can be administered "in conjunction with" infection, meaning close enough in time with the surgery, trauma, or diseases that predispose the host to abscess or adhesion formation so that a protective effect against abscess or adhesion formation is obtained. The preparations can be administered long before surgery in the case of elective surgery (i.e., weeks or even months), preferably with booster administrations closer in time to (and even after) the surgery. Particularly in emergency situations, the preparations can be administered immediately before (minutes to hours) and/or after the trauma or surgery. It is important only that the preparation be administered close enough in time to the surgery so as to enhance the subject's immune response against bacterial infection/contamination, thereby increasing the chances of a successful host response and reducing the likelihood of abscess or adhesion formation.

Those of ordinary skill in the art to which this invention pertains will recognize that the present methods can be applied to a wide range of diseases, symptoms, conditions, signs, disorders, and procedures. Besides abscesses and adhesions, other inflammatory processes and pathologies to which the anti-inflammatory *mono*- and *poly*SPAs, compositions, and methods of the present invention can be applied include:

Allergic diseases such as (generalized) anaphylaxis, serum sickness, generalized drug reactions, food allergies, insect venom allergies, and mastocytosis; airway allergies such as allergic rhinitis, asthma, and hypersensitivity pneumonitis; skin allergies such as urticaria, angioedema, eczema, atopic dermatitis, allergic contact dermatitis, infectious dermatitis, erythema multiforme and Stevens-Johnson syndrome; and ocular allergies such as allergic conjunctivitis, atopic keratoconjunctivitis, venereal keratoconjunctivitis, giant papillary conjunctivitis, and contact allergy.

Organ specific autoimmune diseases include, but are not limited to those of the:

Endocrine system, including: (thyroid gland) Hashimoto's thyroiditis, Graves' disease, thyroiditis with hyperthyroidism; Type I autoimmune polyglandular syndrome, Type II autoimmune polyglandular syndrome, insulin-dependent diabetes mellitus, immune-mediated infertility, and autoimmune Addison's disease.

Skin, including: pemphigus vulgaris, pemphigus foliaceus, paraneoplastic pemphigus, bullus pemphigoid, dermatitis herpetifonnis, linear IgA disease epidermolysis bullosa acquisita, autoimmune alopecia, erythema nodosa, pemphigoid gestationis, cicatricial pemphigoid, and chronic bullous disease of childhood.

Hematologic system, including: autoimmune hemolytic anemia, autoimmune thrombo-cytopenic purpura (idiopathic and drug-related), and autoimmune neutropenia.

Neuromuscular system, including: myasthenia gravis, Eaton-Lambert myasthenic syndrome, Stiff-man syndrome, acute disseminated encephalomyelitis, multiple sclerosis, Guillain-Barré syndrome, chronic inflammatory demyelinating polyradiculoneuropathy, multifocal motor neuropathy with conduction block, and chronic neuropathy with monoclonal gammopathy.

Paraneoplastic neurologic disorders, including: opsoclonus-myoclonus syndrome, cerebellar degeneration, encephalomyelitis, retinopathy.

Hepatobiliary system, including: autoimmune chronic active hepatitis, primary biliary sclerosis, and sclerosing cholangitis.

Gastrointestinal tract, including: gluten-sensitive enteropathy, pernicious anemia, and inflammatory bowel disease.

Organ nonspecific autoimmune diseases including, but not limited to:

Connective tissue diseases, including systemic lupus erythematosus, rheumatoid arthritis, systemic sclerosis (scleroderma), ankylosing spondylitis, reactive arthritides, polymyositis/dermatomyositis, Sjögren's syndrome, mixed connective tissue disease, Behçet's syndrome, and psoriasis.

Vasculitic syndromes, including: systemic necrotizing vasculitides, including classic polyarteritis nodosa, allergic angiitis and granulomatosis (Churg-Strauss disease), and polyangiitis overlap syndrome; hypersensitivity vasculitis, Wegener's granulomatosis, temporal arteritis, Takayasu's arteritis, Kawasaki's disease, isolated vasculitis of the central nervous system, thromboangiitis obliterans, and miscellaneous vasculitides; sarcoidosis, graft-versus-host disease, and cryopathies.

Other diseases and conditions in which anti-inflammatory compounds of the present invention are useful include sepsis; colitis; coronary artery disease; hepatic fibrosis; acute respiratory distress syndrome; acute inflammatory pancreatitis; endoscopic retrograde cholangiopancreatography-induced pancreatitis; burns; atherogenesis of coronary, cerebral, and peripheral arteries; appendicitis; cholecystitis; diverticulitis; visceral fibrotic disorders (liver, lung, intestinal); wound healing; skin scarring disorders (keloids, hidradenitis suppurativa); granulomatous disorders (sarcoidosis, primary biliary cirrhosis); pyoderma gangrenosum; Sweet's syndrome; cell, tissue, or organ transplantation; Alzheimer's disease; Parkinson's disease; atherosclerosis; obesity; and cancer.

Diseases and pathologies to which the inflammatory compounds of inflammatory *mono*- and *poly*SPAs, compositions thereof, and methods employing these compounds and compositions can be applied include antiviral therapy, for example treatment or prevention of hepatitis B virus and hepatitis C virus infections; anticancer therapy; and use as vaccine adjuvants.

Multiple doses of the pharmaceutical compositions of the present invention are contemplated for inducing protection against postoperative surgical adhesion formation. Such multiple doses can be administered over a three day period beginning on the day preceding surgery. Further doses can be administered post surgery as well. Any regimen that results in a reduced postoperative surgical adhesion formation can be used, although optimum doses and dosing regimens are those which would not only inhibit the development of postoperative surgical adhesion formation, but would also result in complete protection against postoperative surgical adhesion formation. Desired time intervals for delivery of multiple doses of one of the present immunomodulatory polymers can be determined by one of ordinary skill in the art employing no more than routine experimentation.

Diseases and pathologies to which the inflammatory *mono-* and *poly*SPAs, compositions thereof, and methods employing these compounds and compositions can be applied include antiviral therapy, for example treatment or prevention of hepatitis B virus and hepatitis C virus infections; antibacterial therapy; antifungal therapy; antiparasitic therapy; anticancer therapy; and use as vaccine adjuvants.

The foregoing descriptions provide a comprehensive overview of the many aspects of the present invention. The following examples illustrate various aspects thereof and are not intended, nor should they be construed, to be limiting thereof in any way. The present invention is not to be limited in scope by the specific examples described herein. Functionally-equivalent products, compositions and methods are clearly within the scope of the invention, as described herein.

The present invention is performed without undue experimentation using, unless otherwise indicated, conventional techniques ofmolecular biology, microbiology, virology, recombinant DNA technology, peptide synthesis in solution, solid phase peptide synthesis, and immunology. Such procedures are described, for example, in the following texts that are incorporated by reference:
1. Sambrook, Fritsch & Maniatis, Molecular Cloning: A Laboratory Manual, Cold Spring Harbor Laboratories, New York, Second Edition (1989), whole of Vols I, II, and III;
2. DNA Cloning: A Practical Approach, Vols. I and 11 (D. N. Glover, ed., 1985), IRL Press, Oxford, whole of text;
3. Oligonucleotide Synthesis: A Practical Approach (M. J. Gait, ed., 1984) IRL Press, Oxford, whole of text, and particularly the papers therein by Gait, pp. 1-22; Atkinson et al., pp. 35-81; Sproat et al., pp. 83-115; and Wu et al., pp. 135-151;
4. Nucleic Acid Hybridization: A Practical Approach (B. D. Hames & S. J. Higgins, eds., 1985) IRL Press, Oxford, whole of text;
5. Animal Cell Culture: Practical Approach, Third Edition (John R.W. Masters, ed., 2000), ISBN 0199637970, whole of text;
6. Immobilized Cells and Enzymes: A Practical Approach (1986) IRL Press, Oxford, whole of text;
7. Perbal, B., A Practical Guide to Molecular Cloning (1984);
8. Methods In Enzymology (S. Colowick and N. Kaplan, eds., Academic Press, Inc.), whole of series;
9. J.F. Ramalho Ortigao, "The Chemistry ofPeptide Synthesis" In: Knowledge database of Access to Virtual Laboratory website (Interactive, Germany);
10. Sakakibara, D., Teichman, J., Lien, E. Land Fenichel, R.L. (1976) Biochem. Biophys. Res. Commun. 73:336);
11. Merrifield, R.B. (1963) J. Am. Chem. Soc. 85:2149;
12. Barany, G. and Merrifield, R.B. (1979) in The Peptides (Gross, E. and Meienhofer, J. eds.), vol. 2, pp.1-284, Academic Press, New York;
13. Wunsch, E., ed. (1974) Synthese von Peptiden in Houben-Weyls 25 Methoden der Organischen Chemie (Muler, E., ed.), vol. 15, 4th edn., Parts 1 and 2, Thieme, Stuttgart;
14. Bodanszky, M. (1984) Principles of Peptide Synthesis, Springer- Verlag, Heidelberg;
15. Bodanszky, M. & Bodanszky, A. (1984) The Practice of Peptide Synthesis, Springer-Verlag, Heidelberg;
16. Bodanszky, M. (1985) Int. J. Peptide Protein Res. 25:449; and
17. Handbook of Experimental Immunology, Vols. 1-IV, D. M. Weir and C. C. Black-well, eds., 1986, Blackwell Scientific Publications.

The present invention will be further illustrated in the following examples.

### Example 1: Preparation of Compounds of monoSPA and polySPA

Polymerization of the disaccharide units from lipid II precursors may be executed either before or after covalent attachment of the epitope(s). The physicochemical properties of the epitope fragments will usually be the factor that determines the choice in route selection. If the peptide or peptide/carbohydrate fragments are soluble, construction of fully elaborated lipids II may be accomplished prior to polymerization. If epitope solubility is an issue, *poly*SPAs may be generated with appropriate epitope attachment points, *i.e*., alkyl azides, pre-installed. Both alternatives are illustrated in general. Illustrative examples feature suppressive *mono*/*poly*SPAs; stimulatory *mono*/*poly*SPAs are prepared in precisely the same manner.

### Example 1A: Epitope Fragments are Soluble: Fully Elaborated Lipid II Prior to Polymerisation

Synthesis of peptide precursor to *bisnor*-azido-Lys lipid II. Details will be clear to those skilled in the art of organic synthesis.

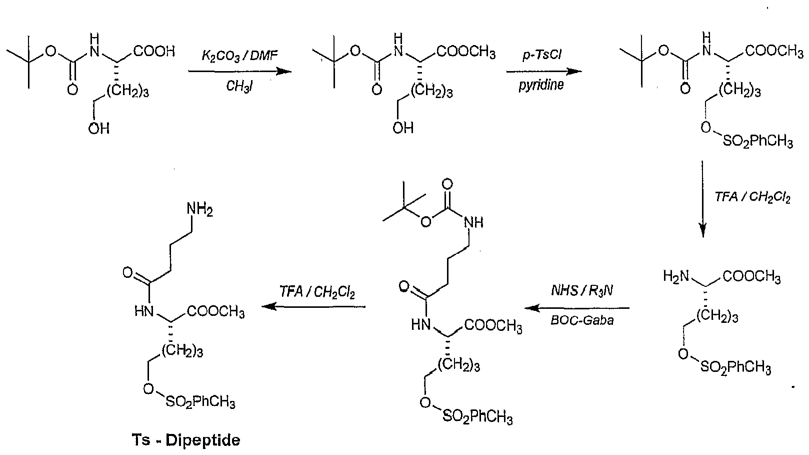

Synthesis of *bisnor*-azido-Lys lipid II. Details will be clear to those skilled in the art of organic synthesis. Where indicated, precisely the same processes taught in WO03075953 are used wherein Ts -Dipeptide is substituted for Compound 5.

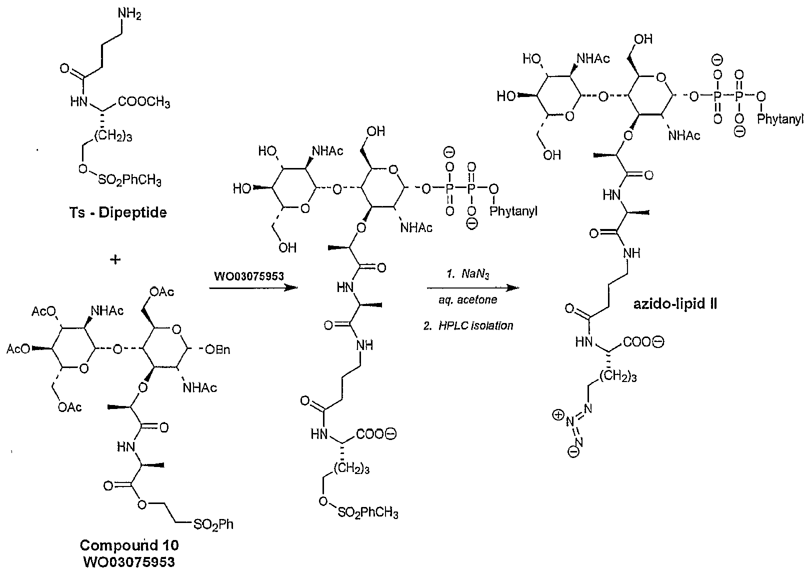

Epitope(s), mono or poly, are attached to the azido-lipid II precisely as described in the literature (Rostovtsev et al. (2002) Angew. Chem. Int. Ed. 114:2708, which in incorporated herein by reference).

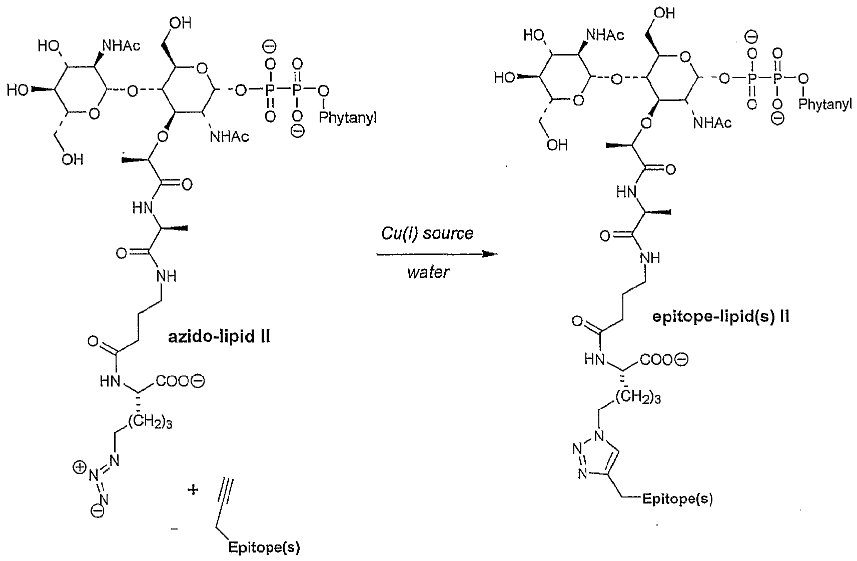

Co-polymerization of an unsubstituted lipid II, e.g., Compound 14 from WO 03/075953, with epitope lipid(s) II using catalytic MtgA under the conditions taught in WO 03/075953 results in a monoSPA or a polySPA, as determined by choice of epitope lipid(s) II. The relative rates of polymerization of unsubstitued and epitope-substituted lipids II are nearly equal when the epitope molecular weight is 1 kDa or less (data not shown). Thus, the epitopes are nearly evenly distributed along the polysaccharide backbone.

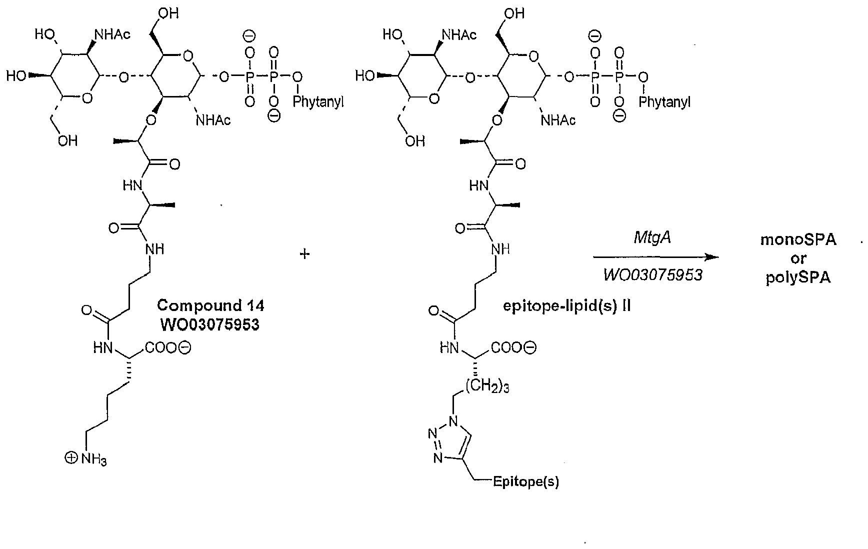

### Example 1B: Alternative Preparation of Compounds of monoSPA and polySPA

Co-polymerization of an unsubstituted lipid II, *e.g*., Compound 14 from WO 03/075953, with azido-lipid II using catalytic MtgA under the conditions taught in WO 03/075953 results in an SPA with alkyl azides, attachment points for epitopes, distributed along the polysaccharide backbone at a linear density determined by the mole fraction of azido-lipid II in the mixture (mole fraction of unsubstitued lipid II + mole fraction of azido-lipid II = 1.00). The relative rates of polymerization of unsubstitued and azide-substituted lipids II are virtually identical.

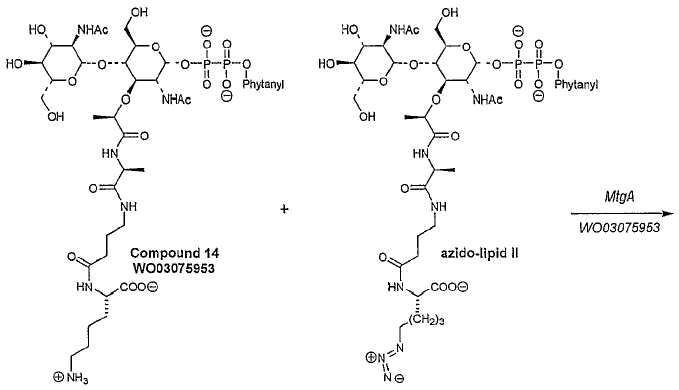

The azides are substituted with epitope(s) after polymerization. Clearly, monoSPA or polySPA will result depending on the number of epitopes selected. The epitopes are applied according to Rostovtsev et al. (2002) Angew. Chem. Int. Ed. 114:2708 and the SPA are purified as described for Compound 15 in WO03075953.

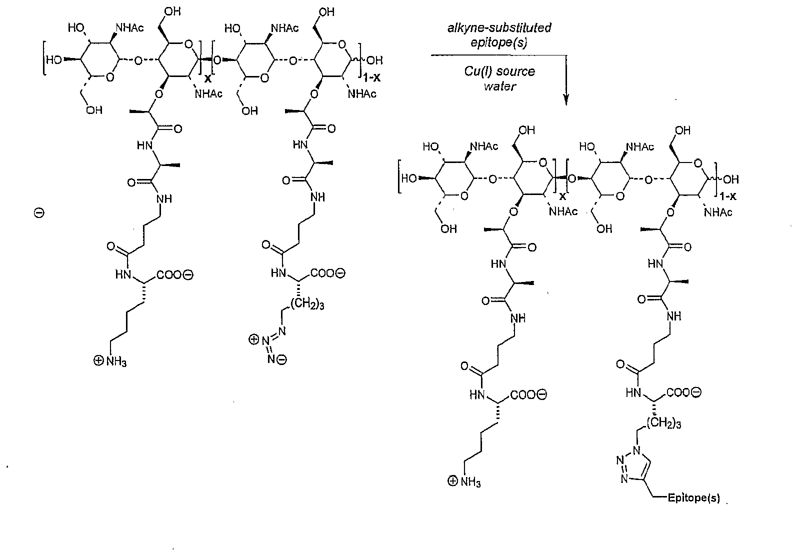

### Example 2: Characterization of Compounds of monoSPA and polySPA

The polysaccharide portion of all SPA, including *mono*SPA and *poly*SPA, are equisitely sensitive to lysozyme digestion. Advantage of this phenomenon may be taken to develop analytical protocols for SPA. Picomolar concentrations of lysozyme react at room temperature to degrade the macromolecular polysaccharide backbone to smaller polysaccharide fragments. Micromolar lysozyme (hen eggwhite or bacteriophage T4) at 37°C degrades an SPA completely and specifically to its component disaccharide-peptides. These disaccharide peptides are then quantified to relative abundance by HPLC peak integration. HPLC in combination with electrospray ionization mass spectrometry and Fourier transform mass spectrometry are then used to identify and rigorously characterize the disaccharide peptide fragments of the SPA.

Like other macromolecular biomolecules, *e.g*., antibody, DNA, protein, *etc.,* SPA cannot simply be lyophilized and weighed to determine concentration in aqueous solution. SPA contains a very large but indeterminant amount of structural water that cannot be measured or removed by lyophilization. An indirect method is used to determine concentrations of SPA accurately. Authentic samples of the disaccharide peptide components of an SPA is independently synthesized with anomeric substitution that allows these independently synthesized fragments to be chromatographically differentiated from the corresponding lysozyme digestion fragments. When a standard curve of mass as a function of HPLC peak area is determined for the anomerically tagged fragments, the absolute concentration of any given SPA in solution are determined. This technique is useful for precise determination of dose in animal model and clinical studies of SPA.

### Examples 3: Differential Induction of TNF-a in Human PBMCs by Compounds of Formulae V, VI, and mono- and polySPAs

The ability of compounds of Formulae V and VI, and of *mono-* and *poly*SPAs to induce the production of the pro-inflammatory cytokine TNF-α by human peripheral blood mononuclear cells (PBMCs) is determined as described below. This test may be used to establish whether an SPA of any structure disclosed herein stimulates TLR2 and, thus, has the ability to deliver antigen-specific suppressive or pro-inflammatory effects.

This protocol can also be used to determine the epitope density that will be tolerated and still allow binding to TLR2, because production of TNF- α by human PBMCs, is dependent on ligation of and signaling through TLR2. Production of TNF- α as a function of epitope density on an SPA is monitored and the inflection, if any, is determined. It is presumed that the maximum epitope density that allows signal in the inflammation-based assay below will be the same epitope density that will allow suppression in appropriately derivatized SPAs.

PBMCs from a human donor are isolated by density gradient centrifugation over Ficoll (Pharmacia, Uppsala, Sweden) plated at a density of 1.0x10⁶ cells/ml in RPMI medium containing 10% FBS (both from Invitrogen Corporation, Carlsbad, CA), and separately incubated at 37°C in a 5 % CO₂ atmosphere for 18 hr either in the presence or absence of **Compound 2, Compound 1**, or of suppressive or pro-inflammatory *mono*- and *poly*SPAs. Separate control cells are incubated under the same conditions as above with 10 ng/ml *S. aureus* peptidoglycan (Sigma). After incubation, the tissue culture medium is removed from the various cells by pipetting, and the amount of TNF-α present therein is determined using a commercially available sandwich ELISA kit that utilizes a monoclonal antibody to TNF-α (BD OptEIA™ Set Human TNF, Pharmingen, Inc.). This ELISA assay has a limit of detection for TNF-α of 7.8 pg/ml.

Incubation with 500 mg/ml, 100 mg/ml and 1 mg/ml of **Compound 2** for 18 hr induces the production of 64.0 pg/ml, 17.6 pg/ml and 1.82 pg/ml TNF-α, respectively, whereas no detectible TNF-a is observed using the same concentrations of Compound 1 with these donor cells. Incubation with 10 ng/ml of S. aureus peptidoglycans induces 26 pg/ml TNF- α in these donor cells.

### Example 4: Amelioration of Proteolipid Protein139-151-Induced Experimental Autoimmune Encephalitis (EAE) in SJL/J Mice

Multiple sclerosis (MS) is a chronic autoimmune inflammatory disease of the central nervous system affecting young adults. Experimental autoimmune encephalitis (EAE), an animal model of MS, is induced in mice by administration of peptides derived from myelin proteins, i.e., proteolipid protein (PLP) 139-151, myelin oligodendrocyte glycoprotein (MOG) 35-55, or myelin basic protein (MBP) 85-99. Without wishing to be bound by theory, in this model, self-reactive CD4+ T cells produce the pro-inflammatory cytokine IFN-γ that mediate the disease. Suppressive cytokines such as IL-4 and IL-10 have been shown to reduce its severity (Stern et al. (2004) PNAS 101:11743).

The efficacies of various compounds of the present invention in the animal model of MS (EAE) induced in SJL/J mice with PLP 139-151 are demonstrated by using three protocols: (i) simultaneous administration of autoantigen and compound (prevention); (ii) pre-treatment with compound (vaccination); and (iii) administration of compound after disease onset (treatment). As the skilled artisan will appreciate, the mouse models are conducted with mouse peptide sequences; human medicine requires human peptide sequences. The scope of the present invention is not limited by the use of species-specific peptides.

*Co-immunization of mice with PLP 139-151 and compound protects against EAE*

SJL/J mice are immunized subcutaneously with 50 µg of PLP 139-151 and compound in a range of doses. In the PLP-immunized group, the first clinical signs of EAE appear in about eight days with a mortality of 100% by day sixteen. Mice co-immunized with an effective anti-inflammatory *mono-* or *poly*SPA of the present invention (dose groups, about 10 ng to about 100 µg) develop EAE at delayed time points followed by recovery at various dose-dependent rates (minimal positive result), or develop essentially no disease (maximal positive result), after the forty-five day experiment duration.

*Pre-immunization of mice protects against PLP 139-151-Induced EAE*

SJL/J mice are immunized subcutaneously with compound (dose groups, about 10 ng to about 100 µg) two days before administration of 50 µg of PLP 139-151 in complete Freund's adjuvant (CFA). All control mice (PLP 139-151/CFA) develop EAE and go on to 100% mortality. Pre-injection with an effective anti-inflammatory *mono-* or *poly*SPA of the present invention on day -2 results in amelioration of symptoms, with no mortality. Efficacy ranking of compounds is demonstrated in this way.

*Treatment of Established PLP 139-151-Induced EAE with Compound Reduces Disease Burden*

SJL/J mice are immunized subcutaneously with PLP 139-151 (50 µg in CFA). On or about day 10, when all mice have developed mild clinical symptoms of EAE (limp tail), a *mono-* or *poly*SPA of the present invention (dose groups, about 10 ng to about 100 µg) is administered subcutaneously for five consecutive days. All control mice (PLP 139-151/CFA) develop severe EAE clinical symptoms and go on to 100% mortality. Suppression of clinical symptoms in treatment groups is evaluated.

*EAE*/*MS Test Compounds and Clinical Hypotheses*

The first compound in the test scheme is Copaxone (Cop1, glatiramer acetate), a random peptide copolymer (Y, E, A, K)n. It serves as a positive control and is the current standard of care for MS relapses in human medicine.

The second compound in the test scheme is based on **Compound 1**. As outlined above, **Compound 1** exhibits a generalized suppressive effect on inflammatory pathologies, and is expected to have some level of efficacy in the model.

The third compound in the test scheme is the suppressive *mono*SPA (see below) based on the myelin basic protein epitope. This compound tests the relative level of efficacy in suppressive activity that might result from a single epitope.

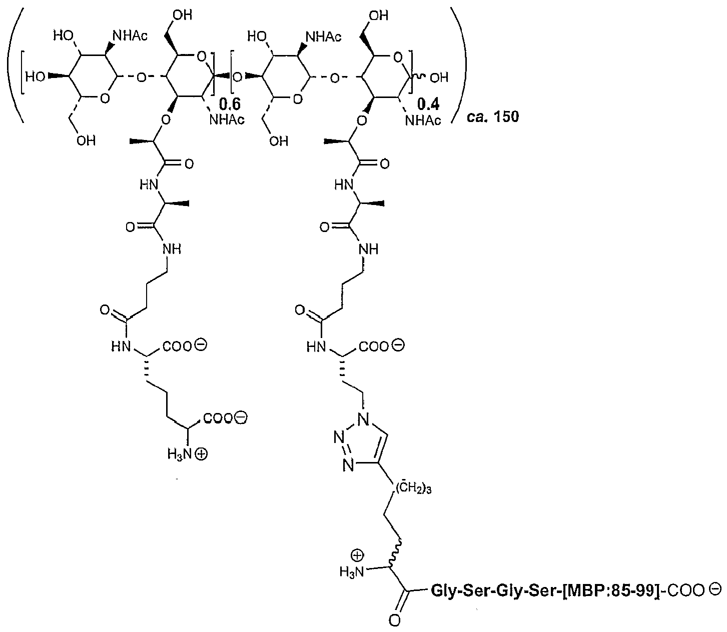

The third compound in the test scheme is the suppressive *poly*SPA (see below) based on three MS (EAE) related epitopes. This compound tests the relative level of efficacy in suppressive activity that might result from multiple MS- (EAE)-related epitopes.

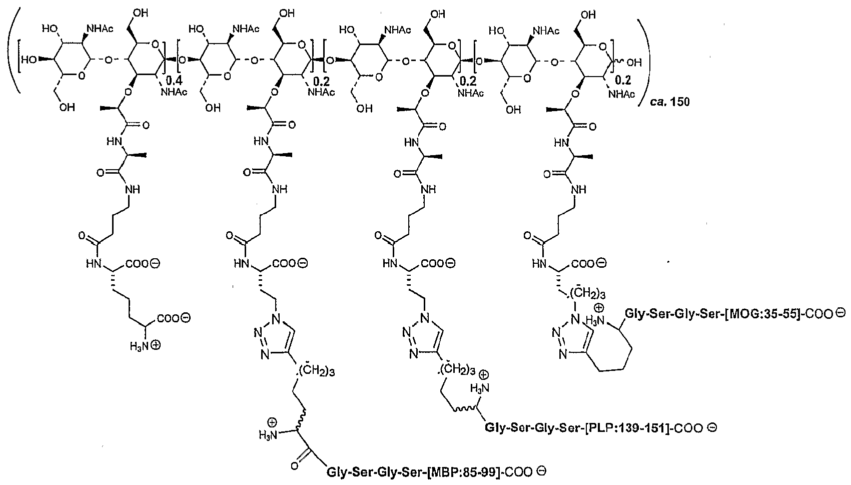

### Example 5: Induction of Immunologic Responses to Human Tumor Antigens

The ability of the pro-inflammatory polySPA, comprising multiple T-helper and target epitopes, to induce immunologic responses to human melanoma immunogens is assessed (Chianese-Bullock et al. (2005) J. Immunol. 174:3080, Slingluff et al. (2001) Clin. Cancer Res. 7:3012). The prototype stimulatory *poly*SPA construct contains three T-helper epitopes: two from canine distemper virus-F (TLMTKNVKPLQSLGSGR, KLIPNASLEINCTLAEL) and one from tetanus toxoid (AQTIKANSKFIGITEL) to augment the activity of three HLA-A2-restricted CTL target epitopes: tyrosinase₃₆₉₋₃₇₇ (YMDGTMSQV), Gp100₂₀₉₋₂₁₇ (IMDQVPFSV) and MAGE-A10₂₅₄₋₂₆₃ (GLYDGMEHL).

A *poly*SPA is prepared by co-polymerization of two lipids II whose stem peptides comprise Ala-D-*iso*-Gln and Ala-D-*iso*-Gln-*bisnor*-azido-Lys in mole fractions of 0.7 and 0.3, respectively. Thus, 30% of the stem peptides in the resulting *poly*SPA will contain C-terminal azide residues. The various epitopes are synthesized by a commercial vendor using standard solid-phase techniques such that the final sequences contain the spacer residues Ser-Gly-Ser-Gly-propargylGly C-terminal to the relevant T-helper peptides, *e.g*., AQTIKANSKFIGITELSGSG(propargylG) and the final sequences of the target epitopes contain the spacer residues (propargylGly)-Gly-Ser-Gly-Ser N-terminal to the relevant CTL peptides, *e.g*., (propargylGly)GSGSYMDGTMSQV. All peptides are homogeneous by HPLC analysis as determined by the commercial supplier. An aqueous solution is prepared in such a manner that the final peptide sequences, three T-helper and three CTL, are present in slight stoichiometric excess over the azide-containing units of the *poly*SPA. Ascorbic acid and copper metal powder are added and the slurry is stirred at room temperature for 14 hr. (Rostovtsev et al. (2002) Angew. Chem. Int. Ed. 114:2708). After the copper is removed by centrifugation, the supernatant is placed in a stirred-cell concentrator and subjected to concentration dilution cycles using water for injection until the effluent conductance is near zero. An aliquot is removed and lyophilized to determine the approximate *poly*SPA concentration. This *poly*SPA is a specific example of the generalized diagram in Fig. 5. A second aliquot is treated exhaustively with hen eggwhite lysozyme to dismantle specifically the polysaccharide backbone of the SPA and reveal the constituent disaccharide-peptide components. The precise composition and relative abundance of the six relevant disaccharide peptide components is determined by HPLC/electrospray mass spectrometry and HPLC/Fourier transform mass spectrometry. The remainder of the *poly*SPA solution is taken on to evaluation *in vivo.*

HLA-A2 transgenic mice (Jackson Laboratories, Bar Harbor, ME) are immunized with the *poly*SPA in three dose groups: 1µg, 10 µg and 100 µg. Vaccination in each dose group is executed subcutaneously at the nape on days 0, +21, and +35. On day +38, peripheral blood is analyzed by standard techniques to determine antibody titer(s) specific for any or all of the target epitopes. The animals are sacrificed on day +39 and the draining lymph nodes are harvested. CD8+ T cell (CTL) activity from the lymph nodes is determined using standard tetramer, ⁵¹Cr release, and Elispot assays that are familiar to those experienced in the practice of cancer immunology. A positive result is antigen-specific CTL activity observed over background in any or all of the assays.

Cancer testis antigens (CTA) such as those illustrated in the present example are expressed in a multiple cancers including melanoma, non-small cell lung, bladder, breast, and prostate (Scanlan et al. (2004) Cancer Immun. 4:1). Thus, the present example provides a basis for broad application in oncology clinical settings. The stimulatory sPGNs are particularly useful as a self-adjuvant platform for presentation of antigen in the context of cancer chemotherapy because the stimulatory sPGNs, which include the *mono-* and *poly*SPAs of the present invention, themselves, by virtue of their ability to signal through TLR2 and independent of attached epitopes, induce the production of TNF-α from peripheral blood mononuclear cells (WO 2005/0305588). TNF-α is well known to inhibit unregulated melanocyte proliferation without affecting normal cells. TNF-α, also acts as a tumor-specific inhibitor of angiogenesis: it inhibits tumor growth by restricting blood and oxygen supply through the surrounding epithelial cells (Goldsby, Kindt, Osborne, and Kuby; Immunology, 5th Edition; W. H. Freeman and Company, New York: 2003, pp. 216-217).

All citations are hereby incorporated by reference.

The present invention has been described with regard to one or more embodiments. However, it will be apparent to persons skilled in the art that a number of variations and modifications can be made without departing from the scope of the invention as defined in the claims.
The present invention also relates to the following sections:
§1. A pro-inflammatory synthetic polysaccharide antigen (SPA), comprising:
   a TLR2-targeting synthetic peptidoglycan (PGN) moiety onto which a first epitope and a second epitope are each covalently attached;
   the first epitope comprising one or more than one generic T helper epitope, the second epitope comprising one or more than one target epitope; the first and second epitopes are present in one or more copies each within the SPA,
   wherein each target epitope is a peptide sequence or a carbohydrate moiety, and wherein each target epitope is an immunogen to CD8+ T cells or B cells, or a pharmaceutically acceptable salt thereof.
§2. The pro-inflammatory SPA according to § 1, or a pharmaceutically acceptable salt thereof, comprising a single species of target epitope in one or more copies each within the SPA.
§3. The pro-inflammatory SPA according to § 1, or a pharmaceutically acceptable salt thereof, comprising more than one species of target epitope, in one or more copies each within the SPA.
§4. The pro-inflammatory SPA according to § 2, wherein the SPA is selected from 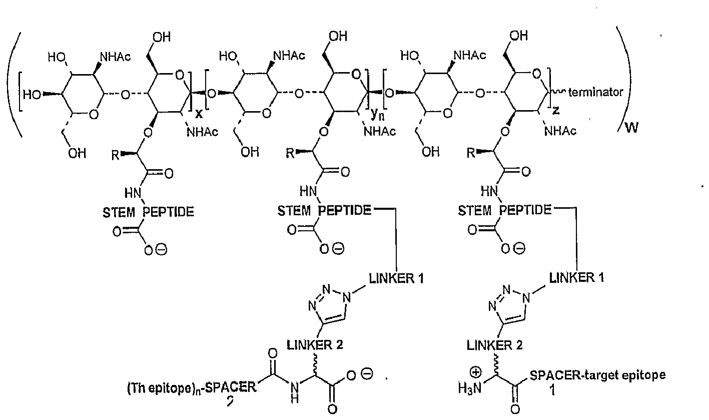 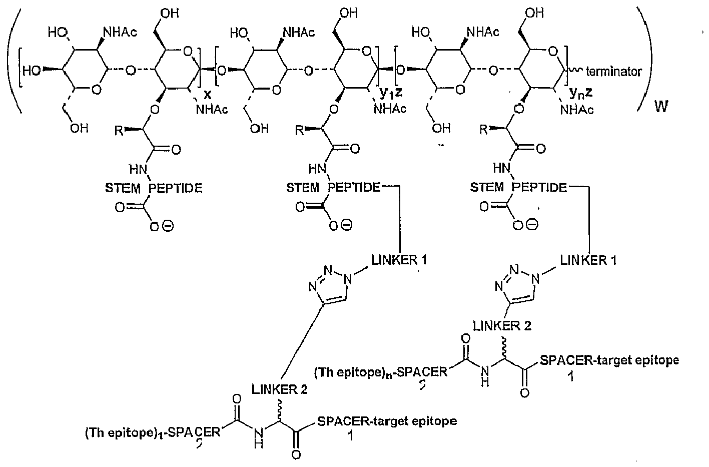 and pharmaceutically acceptable salts thereof, wherein
   W is the total number of monomeric units in the SPA and is an integer in the range of about 10 to about 375;
   R are independantly selected from H or lower alkyl;
   x is the mole fraction of unsubstituted repeat units (UR) in the SPA;
   yₙ is mole fraction of the nth species of Th epitope repeat units (ThR) in the SPA;
   z is the mole fraction of target epitope repeat unit (TR) in the SPA;
   yₙz is mole fraction of the nth species of combined Th epitope / target epitope repeat units (Th/TR) in the SPA;
   STEM PEPTIDE are independantly selected, and comprise about 2 to about 5 amino acids, wherein the amino acids are independently joined at the α or γ carboxyl groups, and at the α or E amino groups, or any combination thereof, provided that a pendant carboxylate or carboxamide group is present;
   LINKER 1 and LINKER2 are independantly selected, and comprise about 1 to about 6 segments, each segment selected from -CH₂-, -CHR-, =CH-, and ≡CH-, -O-, -NH-, -NR-, -S-, -SO-, and -SO₂- provided that there are no contiguous heteroatom segments and that the heteroatom segments are not in segments 1 and 2, where R is a lower alkyl;
   SPACER1 is a peptide of about 1 to about 10 amino acids in length;
   SPACER2 is 0 to about 10 amino acids in length;
   target epitope is a peptide sequence or carbohydrate moiety that is an immunogen to CD8+ T cells or to B cells; and
   (Th epitope)ₙ is a number n of different Th epitopes, each Th epitope is independantly selected and comprises a generic T helper epitope.
§5. The pro-inflammatory SPA according to § 3, wherein the SPA is selected from 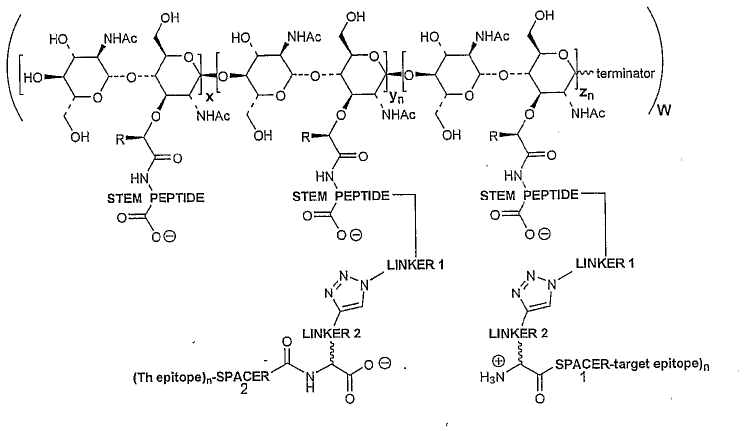 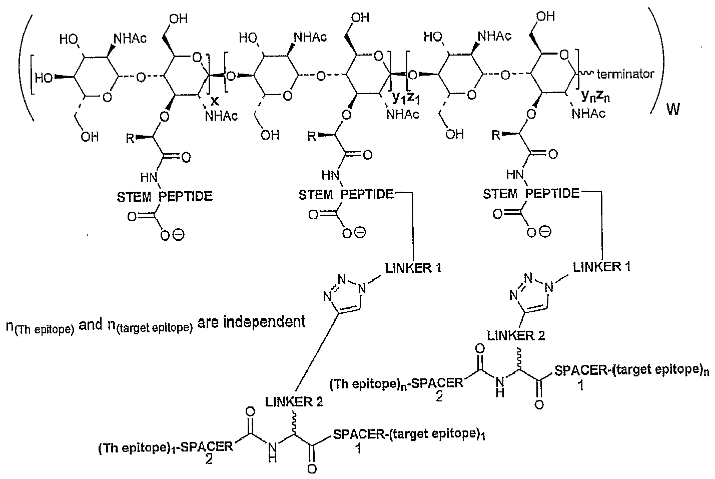 and pharmaceutically acceptable salts thereof, wherein
   W is the total number of monomeric units in the SPA and is an integer in the range of about 10 to about 375;
   R are independantly selected from H or lower alkyl;
   x is the mole fraction of unsubstituted repeat units (UR) in the SPA;
   yₙ is mole fraction of the nth species of Th epitope repeat units (ThR) in the SPA;
   zₙ is the mole fraction of the nth species of target epitope repeat unit (TR) in the SPA;
   yₙzₙ is mole fraction of the nth/nth species of combined Th epitope / target epitope repeat units (Th/TR) in the SPA;
   STEM PEPTIDE are independantly selected, and comprise about 2 to about 5 amino acids, wherein the amino acids are independantly joined at the α or γ carboxyl groups, and at the α or E amino groups, or any combination thereof, provided that a pendant carboxylate or carboxamide group is present;
   LINKER 1 and LINKER2 are independantly selected, and comprise about 1 to about 6 segments, each segment selected from -CH₂-, -CHR-, =CH-, and ≡CH-, -O-, -NH-, -NR-, -S-, -SO-, and -SO₂- provided that there are no contiguous heteroatom segments and that the heteroatom segments are not in segments 1 and 2, where R is a lower alkyl;
   SPACER1 is a peptide of about 1 to about 10 amino acids in length;
   SPACER2 is 0 to about 10 amino acids in length ;
   (target epitope)ₙ is a is a number n of different target epitopes, each target epitope is independantly selected and is peptide sequence or carbohydrate moiety that is an immunogen to CD8+ T cells or to B cells; and
   (Th epitope)ₙ is a number n of different Th epitopes, each Th epitope is independantly selected and comprises a generic T helper epitope.
§6. The pro-inflamatory SPA according to § 3 or 5, wherein the SPA comprises about 2 to about 180 target epitopes and about 1 to about 180 Th helper epitopes, in one or more copies each.
§7. A suppressive synthetic polysaccharide antigen (SPA), comprising:
   a TLR2-targeting synthetic peptidoglycan (PGN) moiety onto which one or more than one target epitope is covalently attached, in one or more copies each, within the SPA,
   wherein each species of target epitope is a peptide sequence or carbohydrate moiety,
   or a pharmaceutically acceptable salt thereof.
§8. The suppressive SPA according to § 7, or a pharmaceutically acceptable salt thereof, comprising a single target epitope in one or more copies within the SPA.
§9. The suppressive SPA according to § 7, or a pharmaceutically acceptable salt thereof, comprising more than one target epitope, in one or more copies each within the SPA.
§10. The suppressive SPA according to § 8, wherein the SPA is 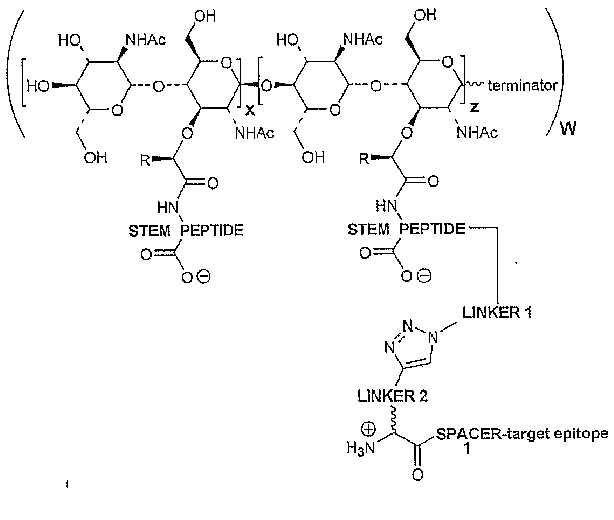 or a pharmaceutically acceptable salt thereof, wherein
   W is the total number of monomeric units in the SPA and is an integer in the range of about 10 to about 375;
   R are independantly selected from H or lower alkyl;
   x is the mole fraction of unsubstituted repeat units (UR) in the SPA;
   z is the mole fraction of target epitope repeat unit (TR) in the SPA;
   STEM PEPTIDE are independantly selected, and comprise about 2 to about 5 amino acids, wherein the amino acids are independantly joined at the α or γ carboxyl groups, and at the α or E amino groups, or any combination thereof, provided that there is no pendant carboxylate or carboxamide group;
   LINKER 1 and LINKER2 are independantly selected, and comprise about 1 to about 6 segments, each segment selected from -CH₂-, -CHR-, =CH-, and ≡CH-, -O-, -NH-, -NR-, -S-, -SO-, and -SO₂- provided that there are no contiguous heteroatom segments and that the heteroatom segments are not in segments 1 and 2, where R is a lower alkyl;
   SPACER1 is a peptide of about 1 to about 10 amino acids in length; and target epitope is a peptide sequence or carbohydrate moiety.
§11. The suppressive SPA according to § 9, wherein the SPA is 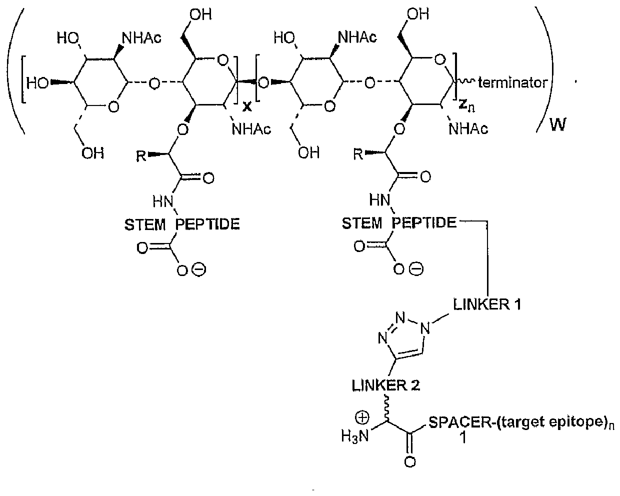 or a pharmaceutically acceptable salt thereof, wherein
   W is the total number of monomeric units in the SPA and is an integer in the range of about 10 to about 375;
   R are independantly selected from H or lower alkyl;
   x is the mole fraction of unsubstituted repeat units (UR) in the SPA;
   zₙ is the mole fraction of the nth species of target epitope repeat unit (TR) in the SPA;
   STEM PEPTIDE are independantly selected, and comprise about 2 to about 5 amino acids, wherein the amino acids are independantly joined at the α or γ carboxyl groups, and at the α or E amino groups, or any combination thereof, provided that there is no pendant carboxylate or carboxamide group;
   LINKER 1 and LINKER2 are independantly selected, and comprise about 1 to about 6 segments, each segment selected from -CH₂-, -CHR-, =CH-, and ≡CH-, -O-, -NH-, -NR-, -S-, -SO-, and -SO₂- provided that there are no contiguous heteroatom segments and that the heteroatom segments are not in segments 1 and 2, where R is a lower alkyl;
   SPACER1 is a peptide of about 1 to about 10 amino acids in length; and (target epitope)ₙ is a is a number n of different target epitopes, each target epitope is independantly selected and is peptide sequence or carbohydrate moiety.
§12. The suppressive SPA according to § 9 or 11 , wherein the SPA comprises about 2 to about 180 target epitope, in one or more copies each.
§13. A synthetic polysaccharide antigen, wherein the SPA is a polymer comprising the sequence:

   X¹-[-MO-]_{w}-X²

   wherein
   X¹ and X² are independently H or a terminator;
   W represents the number of monomeric units (MO) in the polymer, and may be an integer in the range of from about 2 to about 375;
   each MO is a monomeric unit selected from the group comprising unsubstituted repeat units (UR), one or more more than one species of Th epitope repeat units (ThR), one or more more than one species of target epitope repeat units (TR), one or more than one species of combined Th/target epitope repeat unit (Th/TR), and a combination thereof,
   or a pharmaceutically acceptable salt thereof.
§14. The synthetic polysaccharide antigen of § 13, wherein the SPA is a random copolymer.
§15. The synthetic polysaccharide antigen of § 13, wherein the SPA is a block copolymer.
§16. The synthetic polysaccharide antigen of § 13, wherein the SPA is an alternating copolymer.
§17. The synthetic polysaccharide antigen of § 13, or a pharmaceutically acceptable salt thereof, wherein the SPA is a pro-inflammatory synthetic polysaccharide antigen comprising a TLR2-targeting synthetic peptidoglycan (PGN) moiety onto which a first epitope and a second epitope are covalently attached; the first epitope comprising one or more than one generic T helper epitope; the second epitope comprising one or more than one target epitope, and the first and second eptiope are present in one or more copies each, within the SPA; wherein each target epitope is a peptide sequence or a carbohydrate moiety, and wherein each target epitope is an immunogen to CD8+ T cells or B cells.
§18. The synthetic polysaccharide antigen of § 13, or a pharmaceutically acceptable salt thereof, wherein the SPA is a suppresive synthetic polysaccharide antigen comprising, a TLR2-targeting synthetic peptidoglycan (PGN) moiety onto which one or more than one target epitope is covalently attached, in one or more copies each within the SPA, wherein each target epitope is a peptide sequence or carbohydrate moiety.
§19. A pro-inflammatory synthetic polysaccharide antigen (SPA) comprising from about 10 to about 375 monomeric units, the monomeric units independantly selected from 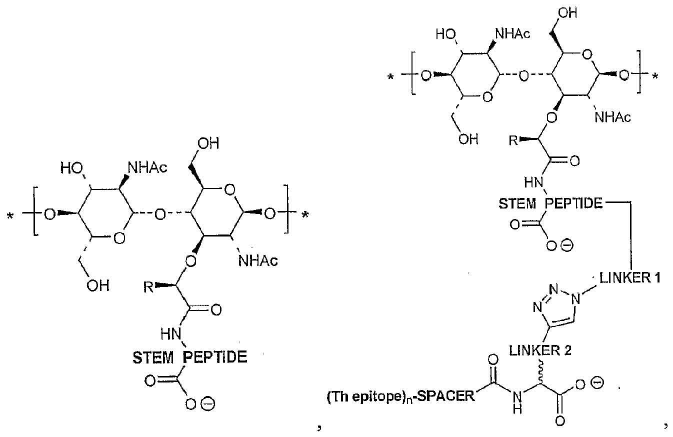 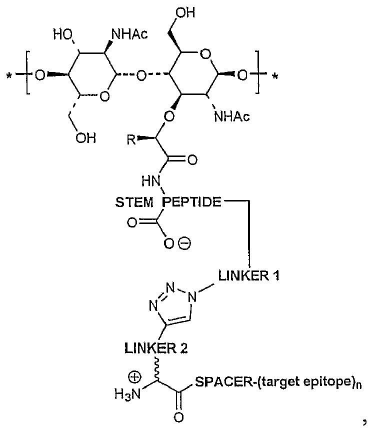 and pharmaceutically acceptable salts thereof,
   wherein
   R are independantly selected from H or lower alkyl;
   STEM PEPTIDE are independantly selected, and comprise about 2 to about 5 amino acids, wherein the amino acids are independantly joined at the α or γ carboxyl groups, and at the α or E amino groups, or any combination thereof, provided that a pendant carboxylate or carboxamide group is present;
   LINKER 1 and LINKER2 are independantly selected, and comprise about 1 to about 6 segments, each segment selected from -CH₂-, -CHR-, =CH-, and ≡CH-, -O-, -NH-, -NR-, -S-, -SO-, and -SO₂- provided that there are no contiguous heteroatom segments and that the heteroatom segments are not in segments 1 and 2, where R is a lower alkyl;
   SPACER1 is a peptide of about 1 to about 10 amino acids in length;
   SPACER2 is 0 to about 10 amino acids in length;
   (target epitope)ₙ is a is a number n of different target epitopes, each target epitope is independantly selected and is peptide sequence or carbohydrate moiety that is an immunogen to CD8+ T cells or to B cells; and
   (Th epitope)ₙ is a number n of different Th epitopes, each Th epitope is independantly selected and comprises a generic T helper epitope.
§20. A pro-inflammatory synthetic polysaccharide antigen (SPA) comprising from about 10 to about 375 monomeric units, the monomeric units independantly selected from 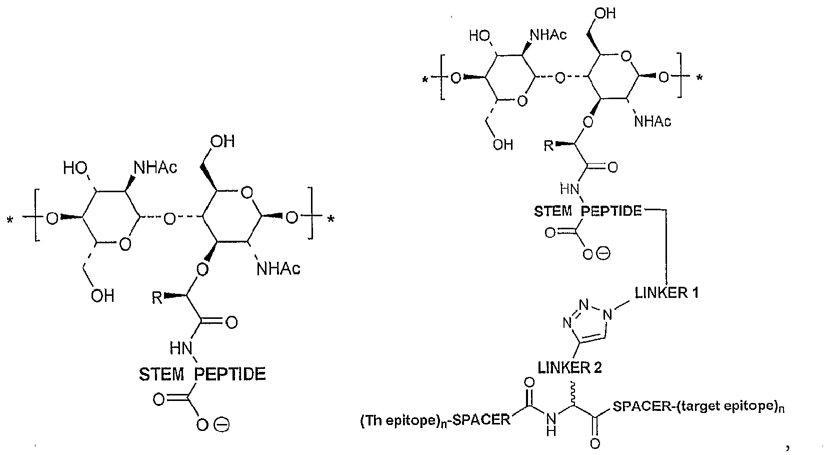 and pharmaceutically acceptable salts thereof,
   wherein
   R are independantly selected from H or lower alkyl;
   STEM PEPTIDE are independantly selected, and comprise about 2 to about 5 amino acids, wherein the amino acids are independantly joined at the α or γ carboxyl groups, and at the α or E amino groups, or any combination thereof, provided that a pendant carboxylate or carboxamide group is present;
   LINKER 1 and LINKER2 are independently selected, and comprise about 1 to about 6 segments, each segment selected from -CH₂-, -CHR-, =CH-, and ≡CH-, -O-, -NH-, -NR-, -S-, -SO-, and -SO₂- provided that there are no contiguous heteroatom segments and that the heteroatom segments are not in segments 1 and 2, where R is a lower alkyl;
   SPACER1 is a peptide of about 1 to about 10 amino acids in length;
   SPACER2 is 0 to about 10 amino acids in length;
   (target epitope)ₙ is a is a number n of different target epitopes, each target epitope is independantly selected and is peptide sequence or carbohydrate moiety that is an immunogen to CD 8+ T cells or to B cells; and
   (Th epitope)ₙ is a number n of different Th epitopes, each Th epitope is independantly selected and comprises a generic T helper epitope.
§21. A suppressive synthetic polysaccharide antigen (SPA) comprising from about 10 to about 375 monomeric units, the monomeric units independantly selected from 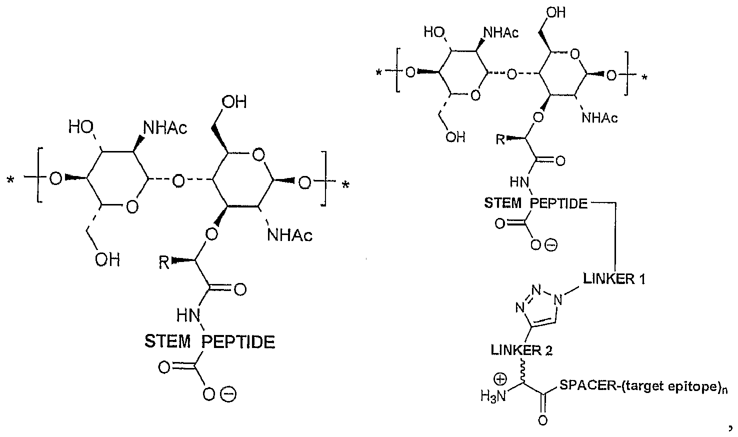 and pharmaceutically acceptable salts thereof,
   wherein
   R are independantly selected from H or lower alkyl;
   STEM PEPTIDE are independantly selected, and comprise about 2 to about 5 amino acids, wherein the amino acids are independantly joined at the α or γ carboxyl groups, and at the α or E amino groups, or any combination thereof, provided there is no pendant carboxylate or carboxamide group;
   LINKER 1 and LINKER2 are independantly selected, and comprise about 1 to about 6 segments, each segment selected from -CH₂-, -CHR-, =CH-, and ≡CH-, -O-, -NH-, -NR-, -S-, -SO-, and -SO₂- provided that there are no contiguous heteroatom segments and that the heteroatom segments are not in segments 1 and 2, where R is a lower alkyl;
   SPACER1 is a peptide of about 1 to about 10 amino acids in length; and (target epitope)ₙ, is a is a number n of different target epitopes, each target epitope is independantly selected and is peptide sequence or carbohydrate moiety.
§22. A pharmaceutical composition comprising the synthetic polysaccharide of any one of § 1 to 21, or a pharmaceutically acceptable salt thereof, and a pharmaceutically acceptable diluent, excipient or carrier.
§23. A use of a synthetic polysaccharide antigen or pharmaceutically acceptable salt thereof of any one of § 1 to 21 as a medicament.
§24. A use of the compound of any one of § 1 to 21, or a pharmaceutically acceptable salt thereof, for the preparation of a medicament for the prevention or treatment of a disease or disorder susceptible to treatment with an immunomodulator.
§25. A method of treating or preventing a disease or disorder susceptible to treatment with an immunomodulator, comprising administering to a patient in need thereof an effective amount of a compound of any one of § 1 to 21 or a pharmaceutically acceptable salt thereof.
§26. A method of inducing an immune response in a mammal, comprising administering to said mammal an effective amount of a synthetic polysaccharide antigen of any one of § 1 to 21, or a pharmaceutically acceptable salt thereof.

## Claims

1. A suppressive synthetic polysaccharide antigen (SPA), comprising:
a TLR2-targeting synthetic peptidoglycan (PGN) moiety onto which one or
more than one target epitope is covalently attached, in one or more copies each, within the SPA,
wherein each species of target epitope is a peptide sequence or carbohydrate moiety,
or a pharmaceutically acceptable salt thereof.

2. The suppressive SPA according to claim 1, or a pharmaceutically acceptable salt thereof, comprising a single target epitope in one or more copies within the SPA.

3. The suppressive SPA according to claim 1, or a pharmaceutically acceptable salt thereof, comprising more than one target epitope, in one or more copies each within the SPA.

4. The suppressive SPA according to claim 2, wherein the SPA is 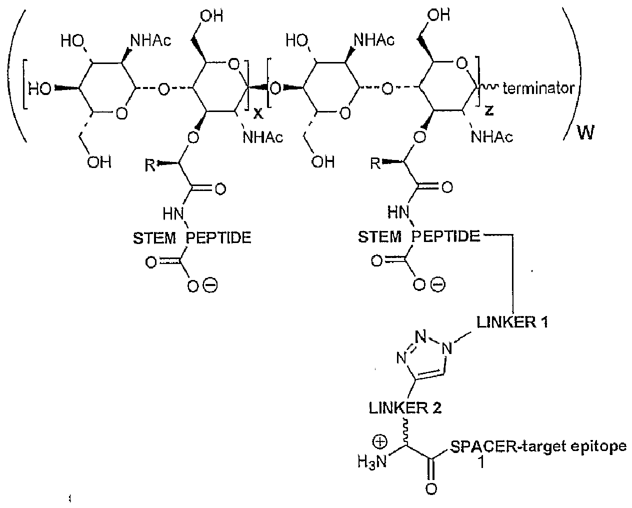 or a pharmaceutically acceptable salt thereof, wherein
W is the total number of monomeric units in the SPA and is an integer in the range of about 10 to about 375;
R are independently selected from H or lower alkyl;
x is the mole fraction of unsubstituted repeat units (UR) in the SPA; z is the mole fraction of target epitope repeat unit (TR) in the SPA; STEM PEPTIDE are independently selected, and comprise about 2 to about 5 amino acids, wherein the amino acids are independently joined at the α or γ carboxyl groups, and at the α or E amino groups, or any combination thereof,
provided that there is no pendant carboxylate or carboxamide group; LINKER 1 and LINKER 2 are independently selected, and comprise about 1 to about 6 segments, each segment selected from -CH₂-, -CHR-, =CH-, and ≡CH-, -0-, -NH-, -NR-, -S- -SO-, and -SO₂- provided that there are no contiguous heteroatom segments and that the heteroatom segments are not in segments 1 and 2, where R is a lower alkyl;
SPACER1 is a peptide of about 1 to about 10 amino acids in length; and target epitope is a peptide sequence or carbohydrate moiety.

5. The suppressive SPA according to claim 3, wherein the SPA is 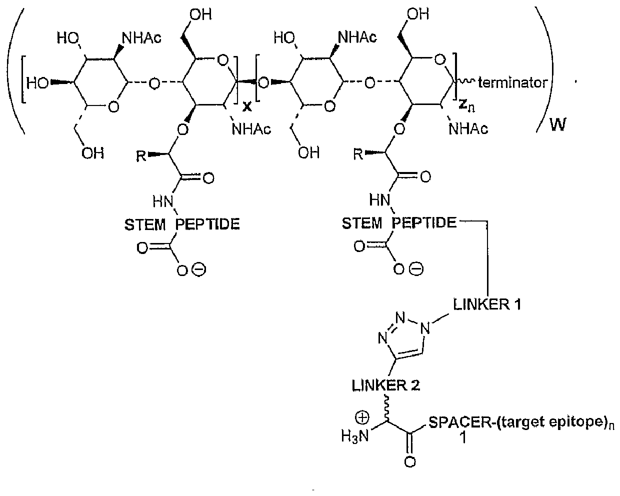 or a pharmaceutically acceptable salt thereof, wherein
W is the total number of monomeric units in the SPA and is an integer in the range of about 10 to about 375;
R are independently selected from H or lower alkyl;
x is the mole fraction of unsubstituted repeat units (UR) in the SPA;
Zₙ is the mole fraction of the nth species of target epitope repeat unit (TR) in the SPA;
STEM PEPTIDE are independently selected, and comprise about 2 to about 5 amino acids, wherein the amino acids are independently joined at the α or γ carboxyl groups, and at the α or E amino groups, or any combination thereof, provided that there is no pendant carboxylate or carboxamide group; LINKER 1 and LINKER 2 are independently selected, and comprise about 1 to about 6 segments, each segment selected from -CH₂-, -CHR-, =CH-, and ≡CH-, -O-, -NH-, -NR-, -S-, -SO-, and -SO₂- provided that there are no contiguous heteroatom segments and that the heteroatom segments are not in segments 1 and 2, where R is a lower alkyl;
SPACER1 is a peptide of about 1 to about 10 amino acids in length; and (target epitope)ₙ is a number n of different target epitopes, each target epitope is independently selected and is peptide sequence or carbohydrate moiety.

6. The suppressive SPA according to claim 3 or 5, wherein the SPA comprises about 2 to about 180 target epitope, in one or more copies each.

7. A pharmaceutical composition comprising the suppressive synthetic polysaccharide antigen of any one of claims 1 to 6, or a pharmaceutically acceptable salt thereof, and a pharmaceutically acceptable diluent, excipient or carrier.

8. The suppressive synthetic polysaccharide antigen or pharmaceutically acceptable salt thereof of any one of claims 1 to 6 for use as a medicament.

9. A use of the compound of any one of claims 1 to 6, or a pharmaceutically acceptable salt thereof, for the preparation of a medicament for the prevention or treatment of a disease or disorder susceptible to treatment with an immunomodulator.

10. A method of treating or preventing a disease or disorder susceptible to treatment with an immunomodulator, comprising administering to a patient in need thereof an effective amount of a compound of any one of claims 1 to 6 or a pharmaceutically acceptable salt thereof.

11. A method of inducing an immune response in a mammal, comprising administering to said mammal an effective amount of a synthetic polysaccharide antigen of any one of claims 1 to 6, or a pharmaceutically acceptable salt thereof.

12. The suppressive synthetic polysaccharide antigen or pharmaceutically acceptable salt thereof of any one of claims 1 to 6 for use in the prevention or treatment of a disease or disorder susceptible to treatment with an immunomodulator.

13. The suppressive synthetic polysaccharide antigen or pharmaceutically acceptable salt thereof of any one of claims 1 to 6 for use in antimicrobial, antiviral or anticancer therapy.


SEQUENCE LISTING

<110>  Eli Lilly and Company

<120>  Monovalent And Polyvalent Synthetic Polysaccaride Antigens For
       Immunological Intervention In Disease

<130>  13287 EP PAB/KT

<140>  EP 06758412.8
<141>  2006-04-19

<150>  US 60/672,807
<151>  2005-04-19

<160>  191

<170>  PatentIn version 3.5

<210>  1
<211>  4
<212>  PRT
<213>  Artificial Sequence

<220>
<223>  SPACER1

<400>  1

Gly Ser Gly Ser
1


<210>  2
<211>  16
<212>  PRT
<213>  Artificial Sequence

<220>
<223>  fragment from influenza virus haemagglutinin Is (IV-H)

<400>  2

Gly Ala Leu Asn Asn Arg Phe Gln Ile Lys Gly Val Glu Leu Lys Ser
1               5                   10                  15


<210>  3
<211>  15
<212>  PRT
<213>  Artificial Sequence

<220>
<223>  fragment from IV-H

<400>  3

Ala Leu Asn Asn Arg Phe Gln Ile Lys Gly Val Glu Leu Lys Ser
1               5                   10                  15

<210>  4
<211>  15
<212>  PRT
<213>  Artificial Sequence

<220>
<223>  fragment from measles virus fusion protein (MV-F)

<400>  4

Leu Ser Glu Ile Lys Gly Val Ile Val His Arg Leu Glu Gly Val
1               5                   10                  15


<210>  5
<211>  16
<212>  PRT
<213>  Artificial Sequence

<220>
<223>  fragment from canine distemper virus fusion protein (CDV-F)

<400>  5

Thr Met Gln Ile Thr Ala Gly Ile Ala Leu His Gln Ser Asn Leu Asn
1               5                   10                  15


<210>  6
<211>  17
<212>  PRT
<213>  Artificial Sequence

<220>
<223>  fragment from CDV-F

<400>  6

Ile Gly Thr Asp Asn Val His Tyr Lys Ile Met Thr Arg Pro Ser His
1               5                   10                  15


Gln




<210>  7
<211>  17
<212>  PRT
<213>  Artificial Sequence

<220>
<223>  fragment from CDV-F

<400>  7

Tyr Lys Ile Met Thr Arg Pro Ser His Gln Tyr Leu Val Ile Lys Leu
1               5                   10                  15

Ile

<210> 8
<211> 17
<212> PRT
<213> Artificial Sequence

<220>
<223> fragment from CDV-F

<400> 8

Ser His Gln Tyr Leu Val Ile Lys Leu Ile Pro Asn Ala Ser Leu Ile
1               5                   10                  15

Glu

<210> 9
<211> 17
<212> PRT
<213> Artificial Sequence

<220>
<223> fragment from CDV-F

<400> 9

Lys Leu Ile Pro Asn Ala Ser Leu Ile Glu Asn Cys Thr Lys Ala Glu
1               5                   10                  15

Leu

<210> 10
<211> 17
<212> PRT
<213> Artificial Sequence

<220>
<223> fragment from CDV-F

<400> 10

Leu Ile Glu Asn Cys Thr Lys Ala Glu Leu Gly Glu Tyr Glu Lys Leu
1               5                   10                  15

Leu

<210> 11

<211>  17
<212>  PRT
<213>  Artificial Sequence

<220>
<223>  fragment from CDV-F

<400>  11

Ala Glu Leu Gly Glu Tyr Glu Lys Leu Leu Asn Ser Val Leu Glu Pro
1               5                   10                  15

Ile

<210>  12
<211>  17
<212>  PRT
<213>  Artificial Sequence

<220>
<223>  fragment from CDV-F

<400>  12

Lys Leu Leu Asn Ser Val Leu Glu Pro Ile Asn Gln Ala Leu Thr Leu
1               5                   10                  15

Met

<210>  13
<211>  17
<212>  PRT
<213>  Artificial Sequence

<220>
<223>  fragment from CDV-F

<400>  13

Glu Pro Ile Asn Gln Ala Leu Thr Leu Met Thr Lys Asn Val Lys Pro
1               5                   10                  15

Leu

<210>  14
<211>  17
<212>  PRT
<213>  Artificial Sequence

<220>
<223>  fragment from CDV-F

<400>  14

Thr Leu Met Thr Lys Asn Val Lys Pro Leu Gln Ser Leu Gly Ser Gly
1               5                   10                  15

Arg

<210>  15
<211>  17
<212>  PRT
<213>  Artificial Sequence

<220>
<223>  fragment from CDV-F

<400>  15

Lys Pro Leu Gln Ser Leu Gly Ser Gly Arg Arg Gln Arg Arg Phe Ala
1               5                   10                  15

Gly

<210>  16
<211>  16
<212>  PRT
<213>  Artificial Sequence

<220>
<223>  fragment from CDV-F

<400>  16

Ser Gly Arg Arg Gln Arg Arg Phe Ala Gly Trp Leu Ala Gly Val Ala
1               5                   10                  15

<210>  17
<211>  16
<212>  PRT
<213>  Artificial Sequence

<220>
<223>  fragment from CDV-F

<400>  17

Phe Ala Gly Trp Leu Ala Gly Val Ala Leu Gly Val Ala Thr Ala Ala
1               5                   10                  15

<210>  18
<211>  16
<212>  PRT

<213>  Artificial Sequence

<220>
<223>  fragment from CDV-F

<400>  18

Gly Val Ala Leu Gly Val Ala Thr Met Gln Ile Thr Ala Gly Ile Ala
1               5                   10                  15


<210>  19
<211>  17
<212>  PRT
<213>  Artificial Sequence

<220>
<223>  fragment from CDV-F

<400>  19

Gly Ile Ala Leu His Gln Ser Asn Leu Asn Ala Gln Ala Ile Gln Ser
1               5                   10                  15


Leu



<210>  20
<211>  17
<212>  PRT
<213>  Artificial Sequence

<220>
<223>  fragment from CDV-F

<400>  20

Asn Leu Asn Ala Gln Ala Ile Gln Ser Leu Arg Thr Ser Leu Glu Gln
1               5                   10                  15


Ser



<210>  21
<211>  17
<212>  PRT
<213>  Artificial Sequence

<220>
<223>  fragment from CDV-F

<400>  21

Gln Ser Leu Arg Thr Ser Leu Glu Gln Ser Asn Lys Ala Ile Glu Glu
1               5                   10                  15

Ile

<210>  22
<211>  17
<212>  PRT
<213>  Artificial Sequence

<220>
<223>  fragment from CDV-F

<400>  22

Glu Gln Ser Asn Lys Ala Ile Glu Glu Ile Arg Glu Ala Thr Gln Glu
1               5                   10                  15

Thr

<210>  23
<211>  17
<212>  PRT
<213>  Artificial Sequence

<220>
<223>  fragment from CDV-F

<400>  23

Ser Ser Lys Thr Gln Thr His Thr Gln Gln Asp Arg Pro Pro Gln Pro
1               5                   10                  15

Ser

<210>  24
<211>  17
<212>  PRT
<213>  Artificial Sequence

<220>
<223>  fragment from CDV-F

<400>  24

Gln Pro Ser Thr Glu Leu Glu Glu Thr Arg Thr Ser Arg Ala Arg His
1               5                   10                  15

Ser

<210>  25

<211>  17
<212>  PRT
<213>  Artificial Sequence

<220>
<223>  fragment from CDV-F

<400>  25

Arg His Ser Thr Thr Ser Ala Gln Arg Ser Thr His Tyr Asp Pro Arg
1               5                   10                  15

Thr

<210>  26
<211>  17
<212>  PRT
<213>  Artificial Sequence

<220>
<223>  fragment from CDV-F

<400>  26

Pro Arg Thr Ser Asp Arg Pro Val Ser Tyr Thr Met Asn Arg Thr Arg
1               5                   10                  15

Ser

<210>  27
<211>  17
<212>  PRT
<213>  Artificial Sequence

<220>
<223>  fragment from CDV-F

<400>  27

Thr Arg Ser Arg Lys Gln Thr Ser His Arg Leu Lys Asn Ile Pro Val
1               5                   10                  15

His

<210>  28
<211>  17
<212>  PRT
<213>  Artificial Sequence

<220>
<223>  fragment from CDV-F

<400>  28

Thr Glu Leu Leu Ser Ile Phe Gly Pro Ser Leu Arg Asp Pro Ile Ser
1               5                   10                  15

Ala

<210>  29
<211>  17
<212>  PRT
<213>  Artificial Sequence

<220>
<223>  fragment from CDV-F

<400>  29

Pro Arg Tyr Ile Ala Thr Asn Gly Tyr Leu Ile Ser Asn Phe Asp Glu
1               5                   10                  15

Ser

<210>  30
<211>  17
<212>  PRT
<213>  Artificial Sequence

<220>
<223>  fragment from CDV-F

<400>  30

Cys Ile Arg Gly Asp Thr Ser Ser Cys Ala Arg Thr Leu Val Ser Gly
1               5                   10                  15

Thr

<210>  31
<211>  17
<212>  PRT
<213>  Artificial Sequence

<220>
<223>  fragment from CDV-F

<400>  31

Asp Glu Ser Ser Cys Val Phe Val Ser Glu Ser Ala Ile Cys Ser Gln
1               5                   10                  15

Asn

<210> 32
<211> 17
<212> PRT
<213> Artificial Sequence

<220>
<223> fragment from CDV-F

<400> 32

Thr Ser Thr Ile Ile Asn Gln Ser Pro Asp Lys Leu Leu Thr Phe Ile
1               5                   10                  15

Ala

<210> 33
<211> 17
<212> PRT
<213> Artificial Sequence

<220>
<223> fragment from CDV-F

<400> 33

Ser Pro Asp Lys Leu Leu Thr Phe Ile Ala Ser Asp Thr Cys Pro Leu
1               5                   10                  15

Val

<210> 34
<211> 24
<212> PRT
<213> Artificial Sequence

<220>
<223> fragment from MUC-1

<400> 34

Ser Thr Ala Pro Pro Ala His Gly Val Thr Ser Ala Pro Asp Thr Arg
1               5                   10                  15

Ala Pro Gly Ser Thr Ala Pro Pro
            20

<210> 35

<211>  19
<212>  PRT
<213>  Artificial Sequence

<220>
<223>  fragment from MUC-1

<400>  35

Gly Val Thr Ser Ala Pro Asp Thr Arg Pro Ala Pro Gly Ser Thr Ala
1               5                   10                  15

Ser Ser Leu

<210>  36
<211>  18
<212>  PRT
<213>  Artificial Sequence

<220>
<223>  fragment from MUC-1

<400>  36

Gly Val Thr Ser Ala Pro Asp Thr Arg Pro Ala Pro Gly Ser Thr Ala
1               5                   10                  15

Ser Leu

<210>  37
<211>  27
<212>  PRT
<213>  Artificial Sequence

<220>
<223>  fragment from MUC-1

<400>  37

Thr Ala Pro Pro Ala His Gly Val Thr Ser Ala Pro Asp Thr Arg Pro
1               5                   10                  15

Ala Pro Gly Ser Thr Ala Pro Pro Lys Lys Gly
            20                  25

<210>  38
<211>  26
<212>  PRT
<213>  Artificial Sequence

<220>
<223>  fragment from MUC-1

<400>  38

Ser Thr Ala Pro Pro Ala His Gly Val Thr Ser Ala Pro Asp Thr Arg
1               5                   10                  15

Pro Ala Pro Gly Ser Thr Ala Pro Pro Lys
            20                  25

<210>  39
<211>  4
<212>  PRT
<213>  Artificial Sequence

<220>
<223>  fragment from foot and mouth disease virus (FMDV-VP3 protein)

<400>  39

Gly Val Ala Glu
1

<210>  40
<211>  10
<212>  PRT
<213>  Artificial Sequence

<220>
<223>  fragment from FMDV-VP3 protein (residues 170 to 179)

<400>  40

Thr Ala Ser Gly Val Ala Glu Ile Ile Asn
1               5                   10

<210>  41
<211>  16
<212>  PRT
<213>  Artificial Sequence

<220>
<223>  fragment from FMDV

<400>  41

Thr Ala Lys Ser Lys Lys Phe Pro Ser Tyr Thr Ala Thr Tyr Gln Phe
1               5                   10                  15

<210>  42
<211>  9
<212>  PRT
<213>  Artificial Sequence

<220>
<223>  from the NP of PRO virus

<400>  42

Thr Tyr Gln Arg Thr Arg Ala Leu Val
1               5


<210>  43
<211>  23
<212>  PRT
<213>  Artificial Sequence

<220>
<223>  fragment from P. falciparum CSP

<400>  43

Lys Pro Lys Asp Glu Leu Asp Tyr Glu Asn Asp Ile Glu Lys Lys Ile
1               5                   10                  15


Cys Lys Met Glu Lys Cys Ser
            20


<210>  44
<211>  20
<212>  PRT
<213>  Artificial Sequence

<220>
<223>  fragement from P. falciparum COP

<400>  44

Asp Ile Glu Lys Lys Ile Cys Lys Met Glu Lys Cys Ser Ser Val Phe
1               5                   10                  15


Asn Trp Asn Ser
            20


<210>  45
<211>  9
<212>  PRT
<213>  Artificial Sequence

<220>
<223>  fragment from P. falciparum LSAT

<400>  45

Lys Pro Ile Val Gln Tyr Asp Asn Phe
1               5


<210>  46
<211>  16
<212>  PRT

<213>  Artificial Sequence

<220>
<223>  fragment from P. falciparum MSP1

<400>  46

Gly Ile Ser Trp Glu Lys Val Leu Ala Lys Tyr Lys Asp Asp Leu Glu
1               5                   10                  15


<210>  47
<211>  22
<212>  PRT
<213>  Artificial Sequence

<220>
<223>  fragment from P. falciparum AMA-1

<400>  47

Glu Phe Thr Tyr Met Ile Asn Phe Gly Arg Gly Gln Asn Tyr Trp Glu
1               5                   10                  15


His Pro Tyr Gln Lys Ser
            20


<210>  48
<211>  19
<212>  PRT
<213>  Artificial Sequence

<220>
<223>  fragment from P. falciparum AMA-1

<400>  48

Asp Gln Pro Lys Gln Tyr Glu Gln His Leu Thr Asp Tyr Glu Lys Ile
1               5                   10                  15


Lys Glu Gly



<210>  49
<211>  10
<212>  PRT
<213>  Artificial Sequence

<220>
<223>  fragment from HIV-1 env protein

<400>  49

Asn Met Trp Gln Glu Val Gly Lys Ala Met
1               5                   10

<210>  50
<211>  9
<212>  PRT
<213>  Artificial Sequence

<220>
<223>  fragment from HIV-1 env protein

<400>  50

Ala Pro Thr Lys Ala Lys Arg Arg Trp
1               5


<210>  51
<211>  10
<212>  PRT
<213>  Artificial Sequence

<220>
<223>  fragment from HIV-1 env protein

<400>  51

Cys Thr Arg Pro Asn Asn Asn Thr Arg Lys
1               5                   10


<210>  52
<211>  10
<212>  PRT
<213>  Artificial Sequence

<220>
<223>  fragment from HIV-1 env protein

<400>  52

Thr Val Tyr Tyr Gly Val Pro Val Trp Lys
1               5                   10


<210>  53
<211>  8
<212>  PRT
<213>  Artificial Sequence

<220>
<223>  fragment from HIV-1 env protein

<400>  53

Arg Pro Trp Ser Thr Gln Leu Leu
1               5


<210>  54
<211>  10
<212>  PRT

<213>  Artificial Sequence

<220>
<223>  fragment from HIV-1 gag protein

<400>  54

Ser Leu Tyr Asn Thr Val Ala Thr Leu Tyr
1               5                   10


<210>  55
<211>  10
<212>  PRT
<213>  Artificial Sequence

<220>
<223>  fragment from HIV-1 gag protein

<400>  55

Glu Leu Arg Ser Leu Tyr Asn Thr Val Ala
1               5                   10


<210>  56
<211>  10
<212>  PRT
<213>  Artificial Sequence

<220>
<223>  fragment from HIV-1 gag protein

<400>  56

Lys Ile Arg Leu Arg Pro Gly Gly Lys Lys
1               5                   10


<210>  57
<211>  10
<212>  PRT
<213>  Artificial Sequence

<220>
<223>  fragment from HIV-1 gag protein

<400>  57

Ile Arg Leu Arg Pro Gly Gly Lys Lys Lys
1               5                   10


<210>  58
<211>  9
<212>  PRT
<213>  Artificial Sequence

<220>
<223>  fragment from HIV-1 gag protein

<400>  58

Arg Leu Arg Pro Gly Gly Lys Lys Lys
1               5


<210>  59
<211>  10
<212>  PRT
<213>  Artificial Sequence

<220>
<223>  fragment from HIV-1 gag protein

<400>  59

Gly Pro Gly His Lys Ala Arg Val Leu Ala
1               5                   10


<210>  60
<211>  10
<212>  PRT
<213>  Artificial Sequence

<220>
<223>  fragment from HIV-1 pol protein

<400>  60

Ser Pro Ile Glu Thr Val Pro Val Lys Leu
1               5                   10


<210>  61
<211>  10
<212>  PRT
<213>  Artificial Sequence

<220>
<223>  fragment from HIV-1 pol protein

<400>  61

Ile Leu Lys Glu Pro Val His Gly Val Tyr
1               5                   10


<210>  62
<211>  9
<212>  PRT
<213>  Artificial Sequence

<220>
<223>  fragment from HIV-1 pol protein

<400>  62

Ala Ile Phe Gln Ser Ser Met Thr Lys

1               5


<210>  63
<211>  10
<212>  PRT
<213>  Artificial Sequence

<220>
<223>  fragment from HIV-1 pol protein

<400>  63

Ser Pro Ala Ile Phe Gln Ser Ser Met Thr
1               5                   10


<210>  64
<211>  10
<212>  PRT
<213>  Artificial Sequence

<220>
<223>  fragment from HIV-1 pol protein

<400>  64

Gln Val Arg Asp Gln Ala Glu His Leu Lys
1               5                   10


<210>  65
<211>  10
<212>  PRT
<213>  Artificial Sequence

<220>
<223>  fragment from HIV-1 pol protein

<400>  65

Gly Pro Lys Val Lys Gln Trp Pro Leu Thr
1               5                   10


<210>  66
<211>  9
<212>  PRT
<213>  Artificial Sequence

<220>
<223>  fragment from influenza virus nucleoprotein

<400>  66

Thr Tyr Gln Arg Thr Arg Ala Leu Val
1               5


<210>  67

<211>  15
<212>  PRT
<213>  Artificial Sequence

<220>
<223>  fragment from influenza nucleoprotein

<400>  67

Thr Tyr Gln Arg Thr Arg Ala Leu Val Arg Thr Gly Met Asp Pro
1               5                   10                  15


<210>  68
<211>  15
<212>  PRT
<213>  Artificial Sequence

<220>
<223>  fragment from influenza virus nucleoprotein

<400>  68

Ile Ala Ser Asn Glu Asn Met Asp Ala Met Glu Ser Ser Thr Leu
1               5                   10                  15


<210>  69
<211>  8
<212>  PRT
<213>  Artificial Sequence

<220>
<223>  fragment from Lymphocytic choriomeningitis virus (LCMV gp1)

<400>  69

Lys Ala Trp Asn Phe Ala Thr Met
1               5


<210>  70
<211>  9
<212>  PRT
<213>  Artificial Sequence

<220>
<223>  fragment from Epstein-Barr virus (EBV)

<400>  70

Gln Val Lys Trp Arg Met Thr Thr Leu
1               5


<210>  71
<211>  9
<212>  PRT
<213>  Artificial Sequence

<220>
<223>  fragment from EBV

<400>  71

Val Phe Ser Asp Gly Arg Val Ala Cys
1               5


<210>  72
<211>  9
<212>  PRT
<213>  Artificial Sequence

<220>
<223>  fragment from EBV

<400>  72

Val Pro Ala Pro Ala Gly Pro Ile Val
1               5


<210>  73
<211>  9
<212>  PRT
<213>  Artificial Sequence

<220>
<223>  fragment from EBV

<400>  73

Thr Tyr Ser Ala Gly Ile Val Gln Ile
1               5


<210>  74
<211>  10
<212>  PRT
<213>  Artificial Sequence

<220>
<223>  fragment from EBV

<400>  74

Leu Leu Asp Phe Val Arg Phe Met Gly Val
1               5                   10


<210>  75
<211>  10
<212>  PRT
<213>  Artificial Sequence

<220>
<223>  fragment from EBV

<400>  75

Gln Asn Gly Ala Leu Ala Ile Asn Thr Phe
1               5                   10


<210>  76
<211>  9
<212>  PRT
<213>  Artificial Sequence

<220>
<223>  fragment from EBV

<400>  76

Val Ser Ser Asp Gly Arg Val Ala Cys
1               5


<210>  77
<211>  9
<212>  PRT
<213>  Artificial Sequence

<220>
<223>  fragment from EBV

<400>  77

Val Ser Ser Glu Gly Arg Val Ala Cys
1               5


<210>  78
<211>  9
<212>  PRT
<213>  Artificial Sequence

<220>
<223>  fragment from EBV

<400>  78

Val Ser Ser Asp Gly Arg Val Pro Cys
1               5


<210>  79
<211>  9
<212>  PRT
<213>  Artificial Sequence

<220>
<223>  fragment from EBV

<400>  79

Val Ser Ser Asp Gly Leu Val Ala Cys
1               5

<210>  80
<211>  9
<212>  PRT
<213>  Artificial Sequence

<220>
<223>  fragment from EBV

<400>  80

Val Ser Ser Asp Gly Gln Val Ala Cys
1               5


<210>  81
<211>  8
<212>  PRT
<213>  Artificial Sequence

<220>
<223>  fragment from EBV

<400>  81

Val Ser Ser Asp Gly Arg Trp Cys
1               5


<210>  82
<211>  10
<212>  PRT
<213>  Artificial Sequence

<220>
<223>  fragment from EBV

<400>  82

Val Pro Ala Pro Pro Val Gly Pro Ile Val
1               5                   10


<210>  83
<211>  10
<212>  PRT
<213>  Artificial Sequence

<220>
<223>  fragment from EBV

<400>  83

Val Glu Ile Thr Pro Tyr Glu Pro Thr Gly
1               5                   10


<210>  84
<211>  10
<212>  PRT

<213>  Artificial Sequence

<220>
<223>  fragment from EBV

<400>  84

Val Glu Ile Thr Pro Tyr Glu Pro Thr Trp
1               5                   10


<210>  85
<211>  10
<212>  PRT
<213>  Artificial Sequence

<220>
<223>  fragment from EBV

<400>  85

Val Glu Leu Thr Pro Tyr Lys Pro Thr Trp
1               5                   10


<210>  86
<211>  9
<212>  PRT
<213>  Artificial Sequence

<220>
<223>  fragment from EBV

<400>  86

Arg Arg Ile Tyr Asp Leu Ile Lys Leu
1               5


<210>  87
<211>  9
<212>  PRT
<213>  Artificial Sequence

<220>
<223>  fragment from EBV

<400>  87

Arg Lys Ile Tyr Asp Leu Ile Glu Leu
1               5


<210>  88
<211>  9
<212>  PRT
<213>  Artificial Sequence

<220>
<223>  fragment from EBV

<400>  88

Pro Tyr Leu Phe Trp Leu Ala Gly Ile
1               5


<210>  89
<211>  13
<212>  PRT
<213>  Artificial Sequence

<220>
<223>  fragment from EBV

<400>  89

Thr Ser Leu Tyr Asn Leu Arg Arg Gly Thr Ala Leu Ala
1               5                   10


<210>  90
<211>  10
<212>  PRT
<213>  Artificial Sequence

<220>
<223>  fragment from EBV

<400>  90

Asp Thr Pro Leu Ile Pro Leu Thr Ile Phe
1               5                   10


<210>  91
<211>  11
<212>  PRT
<213>  Artificial Sequence

<220>
<223>  fragment from EBV

<400>  91

Thr Val Phe Tyr Asn Ile Pro Pro Met Pro Leu
1               5                   10


<210>  92
<211>  10
<212>  PRT
<213>  Artificial Sequence

<220>
<223>  fragment from EBV

<400>  92

Val Glu Ile Thr Pro Tyr Lys Pro Thr Trp

1               5                   10


<210>  93
<211>  9
<212>  PRT
<213>  Artificial Sequence

<220>
<223>  fragment from EBV

<400>  93

Val Ser Phe Ile Glu Phe Val Gly Trp
1               5


<210>  94
<211>  9
<212>  PRT
<213>  Artificial Sequence

<220>
<223>  fragment from EBV

<400>  94

Phe Arg Lys Ala Gln Ile Gln Gly Leu
1               5


<210>  95
<211>  9
<212>  PRT
<213>  Artificial Sequence

<220>
<223>  fragment from EBV

<400>  95

Phe Leu Arg Gly Arg Ala Tyr Gly Leu
1               5


<210>  96
<211>  9
<212>  PRT
<213>  Artificial Sequence

<220>
<223>  fragment from EBV

<400>  96

Gln Ala Lys Trp Arg Leu Gln Thr Leu
1               5


<210>  97

<211>  9
<212>  PRT
<213>  Artificial Sequence

<220>
<223>  fragment from EBV

<400>  97

Ser Val Arg Asp Arg Leu Ala Arg Leu
1               5


<210>  98
<211>  9
<212>  PRT
<213>  Artificial Sequence

<220>
<223>  fragment from EBV

<400>  98

Tyr Pro Leu His Glu Gln His Gly Met
1               5


<210>  99
<211>  8
<212>  PRT
<213>  Artificial Sequence

<220>
<223>  fragment from EBV

<400>  99

His Leu Met Gln Gly Met Ala Tyr
1               5


<210>  100
<211>  9
<212>  PRT
<213>  Artificial Sequence

<220>
<223>  fragment from EBV

<400>  100

Arg Pro Pro Ile Phe Ile Arg Arg Leu
1               5


<210>  101
<211>  9
<212>  PRT
<213>  Artificial Sequence

<220>
<223>  fragment from EBV

<400>  101

Arg Leu Arg Ala Glu Ala Gly Val Lys
1               5


<210>  102
<211>  9
<212>  PRT
<213>  Artificial Sequence

<220>
<223>  fragment from EBV

<400>  102

Ile Val Thr Asp Phe Ser Val Ile Lys
1               5


<210>  103
<211>  10
<212>  PRT
<213>  Artificial Sequence

<220>
<223>  fragment from EBV

<400>  103

Ala Val Phe Asp Arg Lys Ser Asp Ala Lys
1               5                   10


<210>  104
<211>  15
<212>  PRT
<213>  Artificial Sequence

<220>
<223>  fragment from EBV

<400>  104

Asn Pro Thr Gln Ala Pro Val Ile Gln Leu Val His Ala Val Tyr
1               5                   10                  15


<210>  105
<211>  15
<212>  PRT
<213>  Artificial Sequence

<220>
<223>  fragment from EBV

<400>  105

Leu Pro Gly Pro Gln Val Thr Ala Val Leu Leu His Glu Glu Ser
1               5                   10                  15

<210>  106
<211>  14
<212>  PRT
<213>  Artificial Sequence

<220>
<223>  fragment from EBV

<400>  106

Asp Glu Pro Ala Ser Thr Glu Pro Val His Asp Gln Leu Leu
1               5                   10

<210>  107
<211>  8
<212>  PRT

<213>  Artificial Sequence

<220>
<223>  fragment from EBV

<400>  107

Arg Tyr Ser Ile Phe Phe Asp Tyr
1               5

<210>  108
<211>  9
<212>  PRT
<213>  Artificial Sequence

<220>
<223>  fragment from EBV

<400>  108

Ala Val Leu Leu His Glu Glu Ser Met
1               5

<210>  109
<211>  11
<212>  PRT
<213>  Artificial Sequence

<220>
<223>  fragment from EBV

<400>  109

Arg Arg Ala Arg Ser Leu Ser Ala Glu Arg Tyr
1               5                   10

<210>  110
<211>  10
<212>  PRT
<213>  Artificial Sequence

<220>
<223>  fragment from EBV

<400>  110

Glu Glu Asn Leu Leu Asp Phe Val Arg Phe
1               5                   10


<210>  111
<211>  9
<212>  PRT
<213>  Artificial Sequence

<220>
<223>  fragment from EBV

<400>  111

Lys Glu His Val Ile Gln Asn Ala Phe
1               5


<210>  112
<211>  9
<212>  PRT
<213>  Artificial Sequence

<220>
<223>  fragment from EBV

<400>  112

Arg Arg Ile Tyr Asp Leu Ile Glu Leu
1               5


<210>  113
<211>  9
<212>  PRT
<213>  Artificial Sequence

<220>
<223>  fragment from EBV

<400>  113

Gln Pro Arg Ala Pro Ile Arg Pro Ile
1               5


<210>  114
<211>  9

<212>  PRT
<213>  Artificial Sequence

<220>
<223>  fragment from EBV

<400>  114

Glu Gly Gly Val Gly Trp Arg His Trp
1               5


<210>  115
<211>  9
<212>  PRT
<213>  Artificial Sequence

<220>
<223>  fragment from EBV

<400>  115

Cys Leu Gly Gly Leu Leu Thr Met Val
1               5


<210>  116
<211>  9
<212>  PRT
<213>  Artificial Sequence

<220>
<223>  fragment from EBV

<400>  116

Arg Arg Arg Trp Arg Arg Leu Thr Val
1               5


<210>  117
<211>  8
<212>  PRT
<213>  Artificial Sequence

<220>
<223>  fragment from EBV

<400>  117

Arg Ala Lys Phe Lys Gln Leu Leu
1               5


<210>  118
<211>  16
<212>  PRT
<213>  Artificial Sequence

<220>

<223>  fragment from EBV

<400>  118

Arg Lys Cys Cys Arg Ala Lys Phe Lys Gln Leu Leu Gln His Tyr Arg
1               5                   10                  15


<210>  119
<211>  9
<212>  PRT
<213>  Artificial Sequence

<220>
<223>  fragment from EBV

<400>  119

Tyr Leu Leu Glu Met Leu Trp Arg Leu
1               5


<210>  120
<211>  9
<212>  PRT
<213>  Artificial Sequence

<220>
<223>  fragment from EBV

<400>  120

Tyr Phe Leu Glu Ile Leu Trp Gly Leu
1               5


<210>  121
<211>  9
<212>  PRT
<213>  Artificial Sequence

<220>
<223>  fragment from EBV

<400>  121

Tyr Leu Leu Glu Ile Leu Trp Arg Leu
1               5


<210>  122
<211>  9
<212>  PRT
<213>  Artificial Sequence

<220>
<223>  fragment from EBV

<400>  122

Tyr Leu Gln Gln Asn Trp Trp Thr Leu
1               5

<210>  123
<211>  9
<212>  PRT
<213>  Artificial Sequence

<220>
<223>  fragment from EBV

<400>  123

Leu Leu Leu Ala Leu Leu Phe Trp Leu
1               5

<210>  124
<211>  9
<212>  PRT
<213>  Artificial Sequence

<220>
<223>  fragment from EBV

<400>  124

Leu Leu Val Asp Leu Leu Trp Leu Leu
1               5

<210>  125
<211>  9
<212>  PRT
<213>  Artificial Sequence

<220>
<223>  fragment from EBV

<400>  125

Leu Leu Leu Ile Ala Leu Trp Asn Leu
1               5

<210>  126
<211>  9
<212>  PRT
<213>  Artificial Sequence

<220>
<223>  fragment from EBV

<400>  126

Trp Leu Leu Leu Phe Leu Ala Ile Leu
1               5

<210>  127
<211>  9
<212>  PRT
<213>  Artificial Sequence

<220>
<223>  fragment from EBV

<400>  127

Thr Leu Leu Val Asp Leu Leu Trp Leu
1               5


<210>  128
<211>  9
<212>  PRT
<213>  Artificial Sequence

<220>
<223>  fragment from EBV

<400>  128

Leu Leu Trp Leu Leu Leu Phe Leu Ala
1               5


<210>  129
<211>  9
<212>  PRT
<213>  Artificial Sequence

<220>
<223>  fragment from EBV

<400>  129

Ile Leu Leu Ile Ile Ala Leu Tyr Leu
1               5


<210>  130
<211>  9
<212>  PRT
<213>  Artificial Sequence

<220>
<223>  fragment from EBV

<400>  130

Val Leu Phe Ile Phe Gly Cys Leu Leu
1               5


<210>  131
<211>  9
<212>  PRT
<213>  Artificial Sequence

<220>
<223>  fragment from EBV

<400>  131

Arg Leu Gly Ala Thr Ile Trp Gln Leu
1               5


<210>  132
<211>  9
<212>  PRT
<213>  Artificial Sequence

<220>
<223>  fragment from EBV

<400>  132

Ile Leu Tyr Phe Ile Ala Phe Ala Leu
1               5


<210>  133
<211>  9
<212>  PRT
<213>  Artificial Sequence

<220>
<223>  fragment from EBV

<400>  133

Ser Leu Val Ile Val Leu Leu Phe Val
1               5


<210>  134
<211>  9
<212>  PRT
<213>  Artificial Sequence

<220>
<223>  fragment from EBVv

<400>  134

Leu Met Ile Ile Pro Leu Ile Asn Val
1               5


<210>  135
<211>  8
<212>  PRT
<213>  Artificial Sequence

<220>
<223>  fragment from EBV

<400>  135

Ile Leu Phe Ile Gly Ser His Trp
1               5


<210>  136
<211>  9
<212>  PRT
<213>  Artificial Sequence

<220>
<223>  fragment from EBV

<400>  136

Leu Ile Pro Glu Thr Val Pro Tyr Ile
1               5


<210>  137
<211>  9
<212>  PRT
<213>  Artificial Sequence

<220>
<223>  fragment from EBV

<400>  137

Val Leu Gln Trp Ala Ser Leu Ala Val
1               5


<210>  138
<211>  9
<212>  PRT
<213>  Artificial Sequence

<220>
<223>  fragment from EBV

<400>  138

Gln Leu Thr Pro His Thr Lys Ala Val
1               5


<210>  139
<211>  12
<212>  PRT
<213>  Artificial Sequence

<220>
<223>  fragment from Human Cytomegalovirus (HCMV pp65)

<400>  139

Ser Val Leu Gly Pro Ile Ser Gly His Val Leu Lys
1               5                   10

<210>  140
<211>  11
<212>  PRT
<213>  Artificial Sequence

<220>
<223>  fragment from HCMV pp65

<400>  140

Phe Thr Ser Gln Tyr Arg Ile Gln Gly Lys Leu
1               5                   10


<210>  141
<211>  11
<212>  PRT
<213>  Artificial Sequence

<220>
<223>  fragment from HCMV pp65

<400>  141

Phe Val Phe Pro Thr Lys Asp Val Ala Leu Arg
1               5                   10


<210>  142
<211>  8
<212>  PRT
<213>  Artificial Sequence

<220>
<223>  fragment from HCMV pp65

<400>  142

Phe Pro Thr Lys Asp Val Ala Leu
1               5


<210>  143
<211>  9
<212>  PRT
<213>  Artificial Sequence

<220>
<223>  fragment from HCMV pp65

<400>  143

Asn Leu Val Pro Met Val Ala Leu Val
1               5


<210>  144
<211>  9

<212>  PRT
<213>  Artificial Sequence

<220>
<223>  fragment from HCMV pp65

<400>  144

Met Leu Asn Ile Pro Ser Ile Asn Val
1               5


<210>  145
<211>  9
<212>  PRT
<213>  Artificial Sequence

<220>
<223>  fragment from HCMV pp65

<400>  145

Arg Ile Phe Ala Glu Leu Glu Gly Val
1               5


<210>  146
<211>  11
<212>  PRT
<213>  Artificial Sequence

<220>
<223>  fragment from HCMV pp65

<400>  146

Thr Pro Arg Val Thr Gly Gly Gly Gly Ala Met
1               5                   10


<210>  147
<211>  11
<212>  PRT
<213>  Artificial Sequence

<220>
<223>  fragment from HCMV pp65

<400>  147

Arg Pro His Glu Arg Asn Gly Phe Thr Val Leu
1               5                   10


<210>  148
<211>  9
<212>  PRT
<213>  Artificial Sequence

<220>

<223>  fragment from HCMV pp65

<400>  148

Arg Leu Leu Gln Thr Gly Ile His Val
1               5


<210>  149
<211>  9
<212>  PRT
<213>  Artificial Sequence

<220>
<223>  fragment from HCMV pp65

<400>  149

Val Ile Gly Asp Gln Tyr Val Lys Val
1               5


<210>  150
<211>  9
<212>  PRT
<213>  Artificial Sequence

<220>
<223>  fragment from HCMV pp65

<400>  150

Ala Leu Phe Phe Phe Asp Ile Asp Leu
1               5


<210>  151
<211>  11
<212>  PRT
<213>  Artificial Sequence

<220>
<223>  fragment from HCMV pp65

<400>  151

Tyr Ser Glu His Pro Thr Phe Thr Ser Gln Tyr
1               5                   10


<210>  152
<211>  9
<212>  PRT
<213>  Artificial Sequence

<220>
<223>  fragment from HCMV pp65

<400>  152

Val Leu Cys Pro Lys Asn Met Ile Ile
1               5

<210>  153
<211>  9
<212>  PRT
<213>  Artificial Sequence

<220>
<223>  fragment from HCMV pp65

<400>  153

Asp Ile Tyr Arg Ile Phe Ala Glu Leu
1               5

<210>  154
<211>  10
<212>  PRT
<213>  Artificial Sequence

<220>
<223>  fragment from HCMV pp65

<400>  154

Ile Leu Ala Arg Asn Leu Val Pro Met Val
1               5                   10

<210>  155
<211>  10
<212>  PRT
<213>  Artificial Sequence

<220>
<223>  fragment from HCMV pp65

<400>  155

Glu Phe Phe Trp Asp Ala Asn Asp Ile Tyr
1               5                   10

<210>  156
<211>  9
<212>  PRT
<213>  Artificial Sequence

<220>
<223>  fragment from HCMV pp65

<400>  156

Ile Pro Ser Ile Asn Val His His Tyr
1               5

<210>  157
<211>  9
<212>  PRT
<213>  Artificial Sequence

<220>
<223>  fragment from HCMV IE-1

<400>  157

Tyr Ile Leu Glu Glu Thr Ser Val Met
1               5


<210>  158
<211>  9
<212>  PRT
<213>  Artificial Sequence

<220>
<223>  fragment from HCMV IE-1

<400>  158

Cys Val Glu Thr Met Cys Asn Glu Tyr
1               5


<210>  159
<211>  9
<212>  PRT
<213>  Artificial Sequence

<220>
<223>  fragment from HCMV IE-1

<400>  159

Arg Arg Ile Glu Glu Ile Cys Met Lys
1               5


<210>  160
<211>  10
<212>  PRT
<213>  Artificial Sequence

<220>
<223>  fragment from HCMV pp150

<400>  160

Thr Thr Trp Pro Pro Ser Ser Thr Ala Lys
1               5                   10


<210>  161
<211>  8
<212>  PRT
<213>  Artificial Sequence

<220>
<223>  fragment from Measles Virus Fusion glycoprotein

<400>  161

Arg Arg Tyr Pro Asp Ala Trp Leu
1               5


<210>  162
<211>  9
<212>  PRT
<213>  Artificial Sequence

<220>
<223>  fragment from Listeria monocytogenes

<400>  162

Gly Tyr Lys Asp Gly Asn Glu Tyr Ile
1               5


<210>  163
<211>  8
<212>  PRT
<213>  Artificial Sequence

<220>
<223>  fragment from ovalbumin

<400>  163

Ser Ile Ile Asn Phe Glu Lys Leu
1               5


<210>  164
<211>  9
<212>  PRT
<213>  Artificial Sequence

<220>
<223>  fragment from the core protein of hepatitis C virus

<400>  164

Asp Leu Met Gly Tyr Ile Pro Leu Val
1               5


<210>  165
<211>  15
<212>  PRT
<213>  Artificial Sequence

<220>
<223>  fragment from Hepatitis B virus

<400>  165

Leu Val Leu Leu Asp Tyr Gln Gly Met Leu Pro Val Cys Pro Leu
1               5                   10                  15


<210>  166
<211>  15
<212>  PRT
<213>  Artificial Sequence

<220>
<223>  fragment from Hepatitis B virus

<400>  166

Thr Lys Pro Ser Asp Gly Asn Cys Thr Cys Ile Pro Ile Pro Ser
1               5                   10                  15


<210>  167
<211>  13
<212>  PRT
<213>  Artificial Sequence

<220>
<223>  fragment from Hepatitis B virus

<400>  167

Met Gln Trp Asn Ser Thr Thr Phe His Gln Ala Leu Leu
1               5                   10


<210>  168
<211>  14
<212>  PRT
<213>  Artificial Sequence

<220>
<223>  fragment from Influenza virus

<400>  168

Met Phe Glu Asp Leu Arg Val Ser Ser Phe Ile Arg Gly Thr
1               5                   10


<210>  169
<211>  14
<212>  PRT
<213>  Artificial Sequence

<220>
<223>  fragment from Influenza virus

<400>  169

Ser Asn Glu Asn Met Glu Thr Met Asp Ser Ser Thr Leu Glu
1               5                   10

<210>  170
<211>  19
<212>  PRT
<213>  Artificial Sequence

<220>
<223>  fragment from Hemagglutinin

<400>  170

His Pro Leu Ile Leu Asp Thr Cys Thr Ile Glu Gly Leu Ile Tyr Gly
1               5                   10                  15

Asn Pro Ser

<210>  171
<211>  10
<212>  PRT
<213>  Artificial Sequence

<220>
<223>  fragment from Hemagglutinin

<400>  171

Tyr Gln Arg Ile Gln Ile Phe Pro Asp Thr
1               5                   10

<210>  172
<211>  18
<212>  PRT
<213>  Artificial Sequence

<220>
<223>  fragment from Hemagglutinin

<400>  172

Ile Gln Ile Phe Pro Asp Thr Ile Trp Asn Val Ser Tyr Ser Gly Thr
1               5                   10                  15

Ser Lys

<210>  173
<211>  29
<212>  PRT
<213>  Artificial Sequence

<220>
<223>  fragment from Hepatitis C virus

<400>  173

Gly Gly Pro Thr Arg Thr Ile Gly Gly Ser Gln Ala Gln Thr Ala Ser
1               5                   10                  15

Gly Leu Val Ser Met Phe Ser Val Gly Pro Ser Gln Lys
            20                  25

<210>  174
<211>  19
<212>  PRT
<213>  Artificial Sequence

<220>
<223>  fragment from prostate specific antigen

<400>  174

Leu Tyr Thr Lys Trp His Tyr Arg Lys Trp Ile Lys Asp Thr Ile Val
1               5                   10                  15

Ala Asn Pro

<210>  175
<211>  19
<212>  PRT
<213>  Artificial Sequence

<220>
<223>  fragment from prostate specific antigen

<400>  175

Ala Val Lys Val Met Asp Leu Pro Gln Glu Pro Ala Leu Gly Thr Thr
1               5                   10                  15

Cys Tyr Ala

<210>  176
<211>  20
<212>  PRT
<213>  Artificial Sequence

<220>
<223>  fragment from prostate specific antigen

<400>  176

Ile Val Gly Gly Trp Glu Cys Glu Lys His Ser Gln Pro Trp Gln Val
1               5                   10                  15

Leu Val Ala Ser
            20


<210>  177
<211>  14
<212>  PRT
<213>  Artificial Sequence

<220>
<223>  fragment from prostate specific antigen

<400>  177

Cys Ala Gln Val His Pro Gln Lys Val Thr Lys Phe Met Leu
1               5                   10


<210>  178
<211>  21
<212>  PRT
<213>  Artificial Sequence

<220>
<223>  fragment from prostate specific antigen

<400>  178

Tyr Leu Met Leu Leu Arg Leu Ser Glu Pro Ala Glu Leu Thr Asp Asp
1               5                   10                  15


Ala Val Lys Val Met
            20


<210>  179
<211>  21
<212>  PRT
<213>  Artificial Sequence

<220>
<223>  fragment from prostate specific antigen

<400>  179

Leu Leu Lys Asn Arg Phe Leu Arg Pro Gly Asp Asp Ser Ser His Asp
1               5                   10                  15


Leu Met Leu Leu Tyr
            20


<210>  180
<211>  21
<212>  PRT
<213>  Artificial Sequence

<220>

<223>  fragment from prostate specific antigen

<400>  180

Ile Leu Leu Gly Arg His Ser Leu Phe His Pro Glu Asp Thr Gly Gln
1               5                   10                  15

Val Phe Gln Val Tyr
            20

<210>  181
<211>  12
<212>  PRT
<213>  Artificial Sequence

<220>
<223>  fragment from carcinoembryonic antigen

<400>  181

Pro Pro Ala Gln Tyr Ser Trp Leu Ile Asp Gly Asn
1               5                   10

<210>  182
<211>  13
<212>  PRT
<213>  Artificial Sequence

<220>
<223>  Myelin basic protein peptide

<400>  182

Pro Lys Tyr Val Lys Gln Asn Thr Leu Lys Leu Ala Thr
1               5                   10

<210>  183
<211>  27
<212>  PRT
<213>  Artificial Sequence

<220>
<223>  fragment from a collagen type CII peptide 245-270

<400>  183

Ser Pro Thr Gly Pro Leu Gly Pro Lys Gly Gln Thr Gly Glu Leu Gly
1               5                   10                  15

Ile Ala Gly Phe Lys Gly Glu Gln Gly Pro Lys
            20                  25

<210>  184
<211>  22

<212>  PRT
<213>  Artificial Sequence

<220>
<223>  fragment from laminin

<400>  184

Arg Pro Val Arg His Ala Gln Cys Arg Val Cys Asp Gly Asn Ser Thr
1               5                   10                  15

Asn Pro Arg Glu Arg His
            20

<210>  185
<211>  21
<212>  PRT
<213>  Artificial Sequence

<220>
<223>  fragment from laminin

<400>  185

Lys Asn Leu Glu Ile Ser Arg Ser Thr Glu Asp Leu Leu Arg Asn Ser
1               5                   10                  15

Tyr Gly Val Arg Lys
            20

<210>  186
<211>  19
<212>  PRT
<213>  Artificial Sequence

<220>
<223>  fragment from laminin

<400>  186

Thr Ser Leu Arg Lys Ala Leu Leu His Ala Pro Thr Gly Ser Tyr Ser
1               5                   10                  15

Asp Gly Gln

<210>  187
<211>  17
<212>  PRT
<213>  Artificial Sequence

<220>
<223>  fragment from laminin

<400>  187

Lys Ala Thr Pro Met Leu Lys Met Arg Thr Ser Phe His Gly Cys Ile
1               5                   10                  15

Lys

<210>  188
<211>  17
<212>  PRT
<213>  Artificial Sequence

<220>
<223>  fragment from laminin

<400>  188

Asp Gly Lys Trp His Thr Val Lys Thr Glu Tyr Ile Lys Arg Lys Ala
1               5                   10                  15

Phe

<210>  189
<211>  21
<212>  PRT
<213>  Artificial Sequence

<220>
<223>  fragment from laminin

<400>  189

Lys Glu Gly Tyr Lys Val Arg Leu Asp Leu Asn Ile Thr Leu Glu Phe
1               5                   10                  15

Arg Thr Thr Ser Lys
            20

<210>  190
<211>  22
<212>  PRT
<213>  Artificial Sequence

<220>
<223>  fragment from laminin

<400>  190

Lys Gln Asn Cys Leu Ser Ser Arg Ala Ser Phe Arg Gly Cys Val Arg
1               5                   10                  15

Asn Leu Arg Leu Ser Arg
            20

<210>  191
<211>  5
<212>  PRT
<213>  Artificial Sequence

<220>
<223>  N-terminal target epitope sequence

<400>  191

Gly Gly Ser Gly Ser
1               5